# EUROPÄISCHE PATENTSCHRIFT

(11) **EP 1 001 939 B1**
(45) Veröffentlichungstag und Bekanntmachung des Hinweises auf die Patenterteilung: **07.01.2009**
(21) Anmeldenummer: 98942612.7
(22) Anmeldetag: 23.07.1998
(51) Int. Cl.: C07D 231/20, C07D 401/12, A01N 43/56

(54) **HETEROCYCLISCH SUBSTITUIERTE 4-BENZOYL-PYRAZOLE ALS HERBIZIDE**
HETEROCYCLIC SUBSTITUTED 4-BENZOYL-PYRAZOLE AS HERBICIDES
4-BENZOYL-PYRAZOLE HETEROCYCLIQUEMENT SUBSTITUE COMME HERBICIDES

(30) Priorität: 07.08.1997 DE 19734148
(43) Veröffentlichungstag der Anmeldung: 24.05.2000
(73) Patentinhaber: BASF SE, 67056 Ludwigshafen (DE)
(72) Erfinder: ENGEL, Stefan, D-55286 Wörrstadt (DE); RHEINHEIMER, Joachim, D-67063 Ludwigshafen (DE); BAUMANN, Ernst, D-67373 Dudenhofen (DE); VON DEYN, Wolfgang, D-67435 Neustadt (DE); HILL, Regina, Luise, D-67346 Speyer (DE); MAYER, Guido, D-67433 Neustadt (DE); MISSLITZ, Ulf, D-67433 Neustadt (DE); WAGNER, Oliver, D-67061 Ludwigshafen (DE); WITSCHEL, Matthias, D-67061 Ludwigshafen (DE); OTTEN, Martina, D-67069 Ludwigshafen (DE); WALTER, Helmut, D-67283 Obrigheim (DE); WESTPHALEN, Karl-Otto, D-67346 Speyer (DE)
(86) Internationale Anmeldenummer: PCT/EP1998/004635
(87) Internationale Veröffentlichungsnummer: WO 1999/010328

(56) Entgegenhaltungen:
- EP-A- 0 282 944
- WO-A-96/26206
- WO-A-97/03045
- WO-A-97/41105

## Beschreibung

Die vorliegende Erfindung betrifft substituierte 4-Benzoyl-pyrazole der Formel I 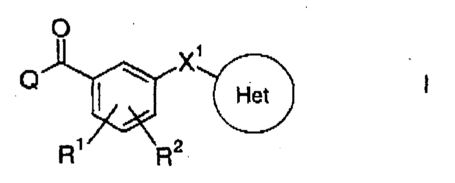 in der die Substituenten folgende Bedeutung haben:
- R¹: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR³ oder -S(O)ₙR³;
- R²: Wasserstoff oder ein wie voranstehend unter R¹ genannter Rest;
- R³: Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl; wobei die genannten Alkylreste partiell oder vollständig halogeniert sein können und/oder eine bis drei der folgenden Gruppen tragen können:
Hydroxy, Mercapto, Amino, Cyano, R³, -OR³, -SR³, -N(R³)₂, =NOR³, -OCOR³, -SCOR³, -NR³COR³, -CO₂R³, -COSR³, -CON(R³)₂, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy,
wobei
Heterocyclyl drei- bis siebengliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, und
Hetaryl, aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten, bedeutet und
wobei die acht letztgenannten Reste ihrerseits substituiert sein können durch ein bis drei Halogenatome und/oder einen oder zwei Reste aus folgender Gruppe: Nitro, Cyano, Methyl, Trifluormethyl, Methoxy, Trifluormethoxy oder Methoxycarbonyl;
- n: 0, 1 oder 2;
- Q: ein in 4-Stellung verknüpftes Pyrazol der Formel II, 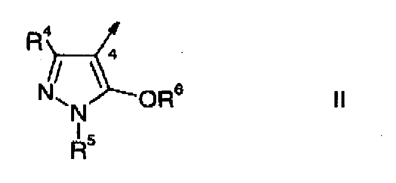 wobei
R⁴ für Wasserstoff, C₁-C₆-Alkyl oder C₁-C₆-Halogenalkyl;
R⁵ für C₁-C₆-Alkyl, C₁-C₆-Halogenalkyl, Phenyl oder Phenyl, das partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy, C₁-C₄-Halogenalkoxy;
R⁶ für Wasserstoff, C₁-C₆-Alkyl, C₁-C₆-Halogenalkyl, C₁-C₆-Alkylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, Phenylcarbonyl, Phenylcarbonylmethyl, Phenoxycarbonyl oder Phenylsulfonyl,
wobei die vier letztgenannten Substituenten unsubstituiert sind oder der Phenylring jeweils partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy, C₁-C₄-Halogenalkoxy; stehen;
- X¹: eine geradkettige oder verzweigte C₁-C₆-Alkylen-, eine C₂-C₆-Alkenylen- oder eine C₂-C₆-Alkinylenkette, die durch ein Heteroatom ausgewählt aus der Gruppe:
Sauerstoff oder Schwefel
unterbrochen ist und wobei die genannten Alkylen-, Alkenylen- oder Alkinylenreste partiell halogeniert sein können und/oder eine bis drei der folgenden Gruppen tragen können:
-OR⁷, -OCOR⁷, -OCONHR⁷ oder -OSO₂R⁷;
- R⁷: Wasserstoff, C₁-C₆-Alkyl, C₂-C₆-Alkenyl, C₂-C₆-Alkinyl, Phenyl, Phenyl-C₁-C₆-alkyl, wobei die genannten Alkyl-, Alkenyl- oder Alkinylreste partiell oder vollständig halogeniert sein können und/oder durch einen oder mehrere der folgenden Reste substituiert sein können:
Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy;
- Het: eine drei- bis sechsgliedrige, mono- oder polycyclische, teilweise oder vollständig gesättigte, heterocyclische Gruppe, die ein bis drei Heteroatome, ausgewählt aus einer Gruppe bestehend aus Sauerstoff, Stickstoff und Schwefel, enthält, oder eine drei- bis sechsgliedrige heteroaromatische Gruppe mit bis zu drei Heteroatomen ausgewählt aus folgenden drei Gruppen:
Stickstoff in Kombination mit mindestens einem Stickstoff oder
Schwefel in Kombination mit mindestens einem Stickstoff;
wobei die genannte heterocyclische oder heteroaromatische Gruppe partiell oder vollständig halogeniert sein kann und/oder durch R⁸ substituiert sein kann;
- R⁸: Wasserstoff, Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl. C₁-C₄-Halogenalkyl, C₁-C₄-Alkylthio, C₁-C₄-Halogenalkylthio, C₁-C₄-Alkoxy, C₁-C₄-Halogenalkoxy, wobei die Alkylreste in allen Fällen jeweils durch einen oder mehrere der folgenden Reste substituiert sein können:
Cyano, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy;
sowie deren landwirtschaftlich brauchbaren Salze.

Außerdem betrifft die Erfindung Verfahren und Zwischenprodukte zur Herstellung von Verbindungen der Formel I, Mittel welche diese enthalten, sowie die Verwendung der Verbindungen der Formel I und diese enthaltende Mittel zur Schadpflanzenbekämpfung.

Aus der Literatur, beispielsweise aus EP-A 282 944, WO97/03045, WO96/26206 sind 4-Benzoyl-pyrazole bekannt.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die erfindungsgemäßen 4-Benzoyl-pyrazole der Formel I sowie deren herbizide Wirkung gefunden.

Ferner wurden herbizide Mittel gefunden, die die Verbindungen I enthalten und eine sehr gute herbizide Wirkung besitzen. Darüber hinaus wurden Verfahren zur Herstellung dieser Mittel und Verfahren zur Bekämpfung von unerwünschtem Pflanzenwuchs mit den Verbindungen I gefunden.

Gegenstand der vorliegenden Erfindung sind auch Stereoisomere der Verbindungen der Formel I. Es werden sowohl reine Stereoisomere als auch Gemische hiervon erfaßt.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl oder Hydroxy-C₁-C₄-alkyl und/oder ein Phenyl oder Benzyl ersetzt sein können, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)-sulfonium und Sulfoxoniumionen, vorzugsweise Tri(C₁-C₄-alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

### Verfahren A:

Umsetzungen von Pyrazolen der Formel II (mit R⁶ = H) mit einer aktivierten Carbonsäure IIIa oder einer Carbonsäure IIIb, die vorzugsweise in situ aktiviert wird, zu dem Acylierungsprodukt V und anschließende Umlagerung zu den erfindungsgemäßen Verbindungen der Formel Ia.

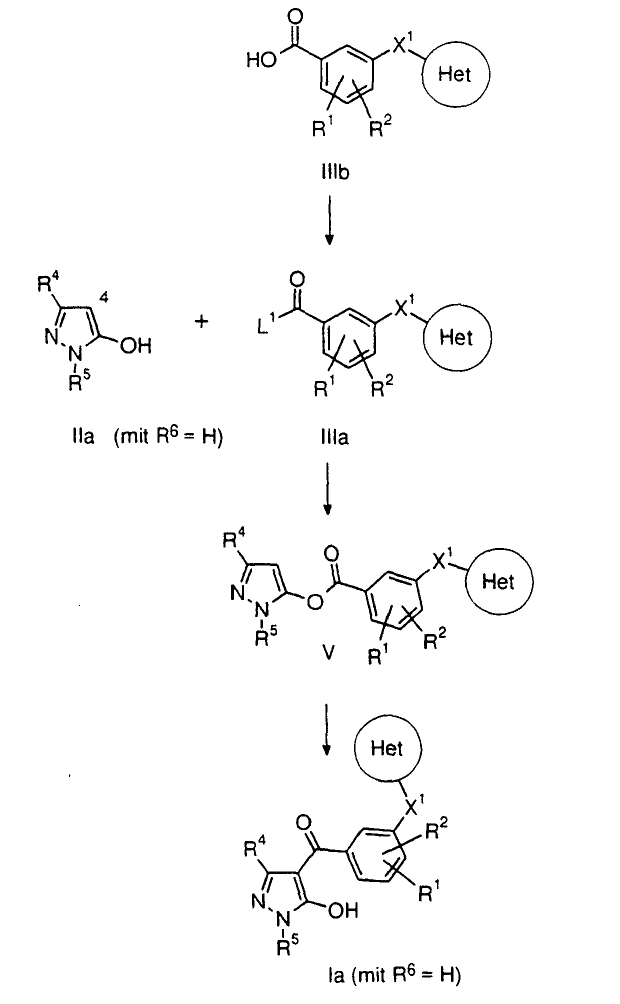

L¹ steht für eine nucleophil austauschbare Abgangsgruppe, wie Halogen z.B. Brom, Chlor, Hetaryl, z.B. Imidazolyl, Pyridyl, Carboxylat, z.B. Acetat, Trifluoracetat etc.

Die aktivierte Carbonsäure kann direkt eingesetzt werden, wie im Fall der Carbonsäurehalogenide oder in situ erzeugt werden, z.B. mit Dicyclohexylcarbodiimid, Triphenylphosphin/Azodicarbonsäureester, 2-Pyridindisulfit/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Carbonsäurehalogenide als aktivierte Carbonsäurekomponente eingesetzt, so kann es zweckmäßig sein, bei Zugabe dieses Reaktionspartners die Reaktionsmischung auf 0 - 10 °C abzukühlen. Anschließend rührt man bei 20 - 100 °C, vorzugsweise bei 25 - 50 °C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels wird der rohe Enolester der Formel VII vorzugsweise durch Chromatographie gereinigt. Es ist aber auch möglich, den rohen Enolester der Formel VII ohne weitere Reinigung zur Umlagerung einzusetzen.

Die Umlagerung der Enolester der Formel VII zu den Verbindungen der Formel I erfolgt zweckmäßigerweise bei Temperaturen von 20 bis 40 °C in einem Lösungsmittel und in Gegenwart einer Base sowie gegebenenfalls in Gegenwart einer Cyanoverbindung.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonate verwendet.

Als Cyanoverbindungen kommen anorganische Cyanide, wie Natriumcyanid, Kaliumcyanid und organische Cyanoverbindungen, wie Acetoncyanhydrin, Trimethylsilycyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Besonders bevorzugt werden Alkalicarbonate, wie Kaliumcarbonat, in Acetonitril oder Dioxan eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid, Essigsäureethylester extrahiert. Der organische Extrakt kann mit 5 - 10 %iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt.

(Beispiele für die Darstellung von Estern von Hydroxypyrazolen und für die Umlagerung der Ester sind z.B. in EP-A 282 944 oder US 4 643 757 genannt).

Die als Ausgangsmaterialien verwendeten Pyrazole der Formel II (mit R⁶ = H) sind bekannt oder können nach an sich bekannten Verfahren hergestellt werden (z.B. EP-A 240 001, J. Prakt. Chem. 315, 383 (1973)).

### Verfahren B:

### Umsetzungen von 4-Benzoyl-pyrazolen der Formel Ia mit einer Verbindung der Formel IV (mit R⁶ # H) :

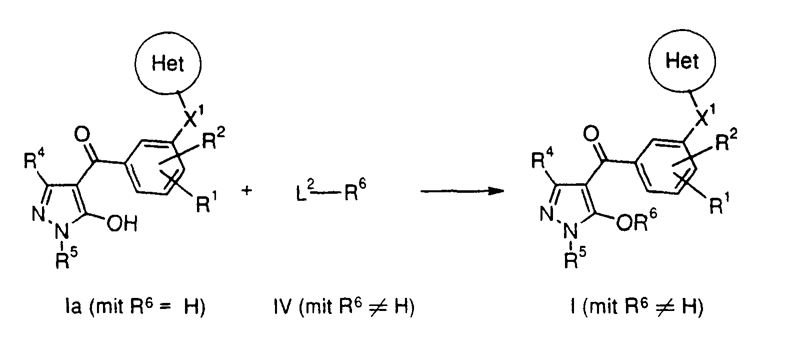

L² steht für eine nucleophil austauschbare Abgangsgruppe, wie Halogen, z.B. Brom, Chlor, Hetaryl, z.B. Imidazolyl, Pyridyl, Carboxylat, z.B. Acetat, Trifluoracetat, Sulfonat, z.B. Mesylat, Triflat etc.

Die Verbindungen der Formel IV können direkt eingesetzt werden, wie z.B. im Fall der Alkylhalogenide, Carbonsäurehalogenide, Sulfonsäurehalogenide, Carbonsäureanhydride und Sulfonsäureanhydride oder in situ erzeugt werden, z.B. aktivierte Carbonsäuren (mittels Carbonsäure und Dicyclohexylcarbodiimid, Carbonyldiimidazol etc.).

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuß der Hilfsbase z.B. 1,5 bis 3 Moläquivalente, bezogen auf Ia, kann unter Umständen vorteilhaft sein.

Als Hilfsbasen eignen sich tertiäre Alkylamine, wie Triethylamin, Pyridin, Alkalimetallcarbonate, z.B. Natriumcarbonat, Kaliumcarbonat und Alkalimetallhydride, z.B. Natriumhydrid. Bevorzugt verwendet werden Triethylamin, Pyridin und Kaliumcarbonat.

Als Lösungsmittel kommen z.B. chlorierte Kohlenwasserstoffe, wie Methylenchlorid, 1,2-Dichlorethan, aromatische Kohlenwasserstoffe, z.B. Toluol, Xylol, Chlorbenzol, Ether, wie Diethylether, Methyl-*tert*-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0 °C bis zur Höhe des Siedepunktes des Reaktionsgemisches.

Die Aufarbeitung kann in an sich bekannter Weise erfolgen.

Bevorzugt sind Benzoesäurehalogenide IIIa mit L¹ = Halogen. 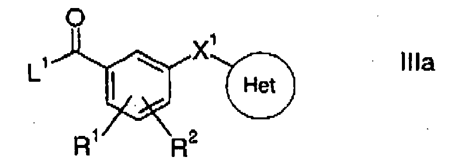 wobei die Variablen R¹, R², X¹ und Het die unter Formel III genannte Bedeutung haben und
- L¹: Halogen, insbesondere Chlor oder Brom, bedeuten.

Ebenso bevorzugt sind Benzoesäuren der Formel IIIb R⁹ = Hydroxy), 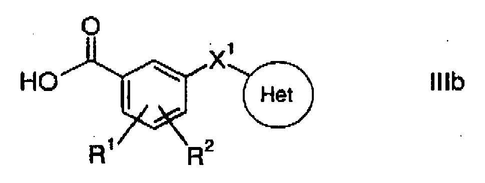 wobei wobei die variablen R¹, R², X¹ und Het die unter Formel III genannte Bedeutung haben.

Ebenso bevorzugt sind Benzoesäureester der Formel IIIc, 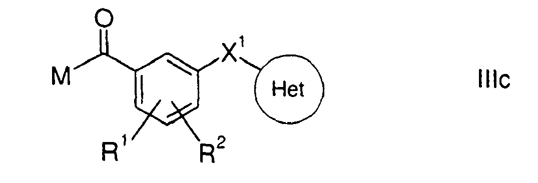 wobei die Variablen R¹, R², X¹ und Het die unter Formel III genannte Bedeutung haben und
- M: C₁-C₆-Alkoxy
bedeutet.

Die Verbindungen der Formel IIIa (mit L¹ = Halogen) können in Analogie zu literaturbekannten Methoden (vgl. L.G. Fieser, M. Fieser "Reagents for Organic Synthesis", Bd. I, S. 767-769 (1967)) durch Umsetzung von Benzoesäuren der Formel IIIb mit Halogenierungsreagentien wie Thionylchlorid, Thionylbromid, Phosgen, Diphosgen, Triphosgen, Oxalylchlorid, Oxalylbromid dargestellt werden.

Die Benzoesäuren der Formel IIIb können u. a. durch Verseifung der Benzoesäureester der Formel IIIc (mit M = C₁-C₆-Alkoxy) er halten werden.

Die erfindungsgemäßen Benzoesäureester der Formel IIIc sind nach verschiedenen literaturbekannten Methoden (z.B. a. G. Dittus in Houben-Weyl, Methoden der Organischen Chemie, Band VI/3, Sauerstoff-Verbindungen I, 4. Aufl., S. 493 ff., Georg Thieme Verlag, 1965; b. T. L. Gilchrist, Heterocyclenchemie, 2. Aufl., Verlag Chemie, 1995) darstellbar, wie in den nachfolgenden Beispielen illustriert.

### Verfahren C:

Die Substitution der Benzoesäureester VIa mit geeigneten Nucleophilen VII liefert die erfindungsgemäßen Benzoesäureester IIIc, 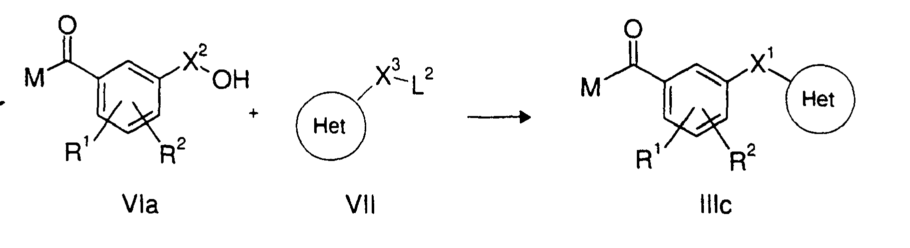 wobei M, R¹ und R² die oben genannte Bedeutung haben, L² eine geeignete, nucleophil austauschbare Abgangsgruppe, wie Halogen, z.B. Brom, Chlor, Hetaryl, z.B. Imidazolyl, Pyridyl, Carboxylat, z.B. Acetat, Trifluoracetat, Sulfonat, z.B. Mesylat, Triflat etc. darstellt,
- X²: eine geradkettige oder verzweigte Alkylen-, eine Alkenylen- oder eine Alkinylenkette mit mindestens einem und höchstens fünf Kohlenstoffatomen darstellt, wobei die genannten Alkylen-, Alkenylen- oder Alkinylenreste partiell halogeniert sein können und/oder eine bis drei der folgenden Gruppen tragen können:
-OR⁷, -OCOR⁷, -OCONHR⁷ oder -OSO₂R₇ und
- X³: eine geradkettige oder verzweigte Alkylen-, eine Alkenylen- oder eine Alkinylenkette mit höchstens fünf Kohlenstoffatomen darstellt, wobei die genannten Alkylen-, Alkenylen- oder Alkinylenreste partiell halogeniert sein können und/oder eine bis drei der folgenden Gruppen tragen können:
-OR⁷, -OCOR⁷, -OCONHR⁷ oder -OSO₂R₇.

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuß der Hilfsbase z.B. 1,5 bis 3 Moläquivalente, bezogen auf VIa, kann unter Umständen vorteilhaft sein.

Als Hilfsbasen eignen sich tertiäre Alkylamine, wie Triethylamin, Pyridin, Alkalimetallcarbonate, z.B. Natriumcarbonat, Kaliumcarbonat und Alkalimetallhydride, z.B. Natriumhydrid. Bevorzugt verwendet werden Triethylamin, Pyridin und Kaliumcarbonat.

Als Lösungsmittel kommen z.B. chlorierte Kohlenwasserstoffe, wie Methylenchlorid, 1,2-Dichlorethan, aromatische Kohlenwasserstoffe, z.B. Toluol, Xylol, Chlorbenzol, Ether, wie Diethylether, Methyl-*tert*-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0 °C bis zur Höhe des Siedepunktes des Reaktionsgemisches.

Die Aufarbeitung kann in an sich bekannter Weise erfolgen.

### Verfahren D:

Die Substitution der geeignet substituierten Heterocyclen VIII mit den Benzoesäureestern Vb liefert die erfindungsgemäßen Benzoesäureester IIIc, 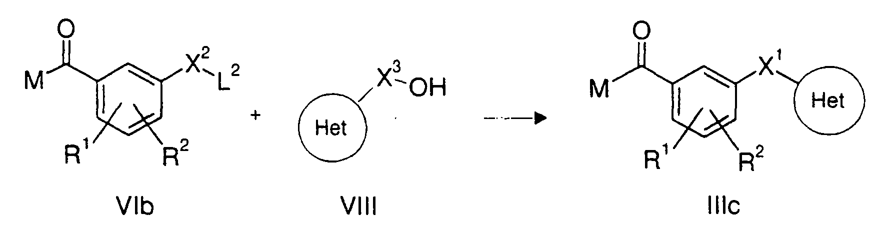 wobei M, R¹ und R² die oben genannte Bedeutung haben, L² eine geeignete, nucleophil austauschbare Abgangsgruppe, wie Halogen, z.B. Brom, Chlor, Hetaryl, z.B. Imidazolyl, Pyridyl, Carboxylat, z.B. Acetat, Trifluoracetat, Sulfonat, z.B. Mesylat, Triflat etc. darstellt,
- X²: eine geradkettige oder verzweigte Alkylen-, eine Alkenylen- oder eine Alkinylenkette mit mindestens einem und höchstens fünf Kohlenstoffatomen darstellt, wobei die genannten Alkylen-, Alkenylen- oder Alkinylenreste partiell halogeniert sein können und/oder eine bis drei der folgenden Gruppen tragen können:
-OR⁷, -OCOR⁷, -OCONHR⁷ oder -OSO₂R₇ und
- X³: eine geradkettige oder verzweigte Alkylen-, eine Alkenylen- oder eine Alkinylenkette mit höchstens fünf Kohlenstoffatomen darstellt, wobei die genannten Alkylen-, Alkenylen- oder Alkinylenreste partiell halogeniert sein können und/oder eine bis drei der folgenden Gruppen tragen können:
-OR⁷, -OCOR⁷, -OCONHR⁷ oder -OSO₂R₇.

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuß der Hilfsbase z.B. 1,5 bis 3 Moläquivalente, bezogen auf VIII, kann unter Umständen vorteilhaft sein.

Als Hilfsbasen eignen sich tertiäre Alkylamine, wie Triethylamin, Pyridin, Alkalimetallcarbonate, z.B. Natriumcarbonat, Kaliumcarbonat und Alkalimetallhydride, z.B. Natriumhydrid. Bevorzugt verwendet werden Triethylamin, Pyridin und Kaliumcarbonat.

Als Lösungsmittel kommen z.B. chlorierte Kohlenwasserstoffe, wie Methylenchlorid, 1,2-Dichlorethan, aromatische Kohlenwasserstoffe, z.B. Toluol, Xylol, Chlorbenzol, Ether, wie Diethylether, Methyl-*tert*-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0 °C bis zur Höhe des Siedepunktes des Reaktionsgemisches.

Die Aufarbeitung kann in an sich bekannter Weise erfolgen.

Hervorzuheben sind die erfindungsgemäßen Verbindungen der Formel I, wobei die Gruppe X¹ für eine durch ein sauerstoffatom unterbrochene C₁-C₃-Alkylen-, eine C₂-C₃-Alkenylen- oder C₂-C₃-Alkinylenkette steht und
- Het: eine wie oben definierte drei- bis sechsgliedrige, teilweise oder vollständig gesättigte, heterocyclische Gruppe oder eine drei- bis sechsgliedrige heteroaromatische Gruppe mit bis zu drei Heteroatomen ausgewählt aus folgenden drei Gruppen:
Stickstoff,
Sauerstoff in Kombination mit mindestens einem Stickstoff oder
Schwefel in Kombination mit mindestens einem Stickstoff,
wobei die gennante heterocyclische oder heteroaromatische Gruppe partiell oder vollständig halogeniert sein kann und/ oder durch R⁸ substituiert sein kann;
darstellt.

Darüber hinaus sind die erfindungsgemäßen Verbindungen der Formel I hervorzuheben, wobei die Gruppe Het für eine fünf- oder sechsgliedrige, teilweise oder vollständig gesättigte heterocyclische oder eine fünf- oder sechsgliedrige heteroaromatische Gruppe mit bis zu drei Heteroatomen ausgewählt aus folgenden drei Gruppen:

### Stickstoff,

Sauerstoff in Kombination mit mindestens einem Stickstoff oder Schwefel in Kombination mit mindestens einem Stickstoff;
steht, wobei die gennante heterocyclische oder heteroaromatische Gruppe partiell oder vollständig halogeniert und/oder durch R⁸ substituiert sein kann;
- R⁸: Wasserstoff, Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy, wobei die Alkylreste in allen Fällen jeweils durch einen oder mehrere der folgenden Reste substituiert sein können:
Cyano, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy.

Die für die Substituenten R¹ - R⁹ oder als Reste an Phenyl-, Hetaryl- und Heterocyclylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cycloalkyl-, Alkoxyalkyl-, Alkoxy-, Halogenalkoxy-, Alkyliminooxy-, Alkoxyamino-, Alkylsulfonyl-, Halogenalkylsulfonyl, Alkylcarbonyl-, Halogenalkylcarbonyl, Alkoxycarbonyl-, Alkoxyalkoxycarbonyl-, Alkenyl-, Cycloalkenyl-, Alkinyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

### Ferner bedeuten beispielsweise:

- C₁-C₄-Alkyl, sowie die Alkylteile von C₁-C₄-Alkylcarbonyl: Methyl, Ethyl, n-Propyl, 1-Methylethyl, Butyl, 1-Methylpropyl, 2-Methylpropyl und 1,1-Dimethylethyl;
- C₁-C₆-Alkyl, sowie die Alkylteile von C₁-C₆-Alkoxy-C₁-C₆-alkyl und C₁-C₆-Alkylcarbonyl: C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Halogenalkyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl, sowie die Halogenalkylteile von C₁-C₆-Halogenalkylcarbonyl: C₁-C₄-Halogenalkyl wie voranstehend genannt, sowie 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
- C₁-C₄-Alkoxy, sowie die Alkoxyteile von C₁-C₄-Alkoxyamino, C₁-C₄-Alkoxy-C₂-C₆-alkoxycarbonyl und C₁-C₄-Alkoxycarbonyl: Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy, sowie die Alkoxyteile von C₁-C₆-Alkoxy-C₁-C₆-alkyl, C₁-C₆-Alkoxy-C₂-C₆-alkyl, C₁-C₄-Alkoxy-C₂-C₆-alkoxycarbonyl und C₁-C₆-Alkoxycarbonyl: C₁-C₄-Alkoxy wie voranstehend genannt, sowie Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₄-Alkylsulfonyl (C₁-C₄-Alkyl-S(=O)2-): Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
- C₁-C₆-Alkylsulfonyl: C₁-C₄-Alkylsulfonyl wie voranstehend genannt, sowie Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, 1,1-Dimethylpropylsulfonyl, 1,2-Dimethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
- C₁-C₆-Halogenalkylsulfonyl: einen C₁-C₆-Alkylsulfonylrest wie voranstehend genannt, der partiell oder vollständig durch Fluor, Chlor, Brom und/ oder Iod substituiert ist, also Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2,2,2-Trichlorethylsulfonyl, 2-Chlor-2-fluorethylsulfonyl, 2-Chlor-2,2-difluorethylsulfonyl, 2,2-Dichlor-2-fluorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl und Dodecafluorhexylsulfonyl;
- C₁-C₄-Alkyliminooxy: Methyliminooxy, Ethyliminooxy, 1-Propyliminooxy, 2-Propyliminooxy, 1-Butyliminooxy und 2-Butyliminooxy;
- C₃-C₆-Alkenyl: Prop-1-en-1-yl, Prop-2-en-1-yl, 1-Methylethenyl, Buten-1-yl, Buten-2-yl, Buten-3-yl, 1-Methyl-prop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl;
- C₂-C₆-Alkenyl: C₃-C₆-Alkenyl wie voranstehend genannt, sowie Ethenyl;
- C₃-C₆-Alkinyl: Prop-1-in-1-yl, Prop-2-in-1-yl, But-1-in-1-yl, But-1-in-3-yl, But-1-in-4-yl, But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-1-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl;
- C₂-C₆-Alkinyl: C₃-C₆-Alkinyl, wie voranstehend genannt, sowie Ethinyl:
- C₃-C₆-Cycloalkyl: Cyclopropyl, Cyclobutyl, Cyclopentyl und Cyclohexyl;
- C₄-C₆-Cycloalkenyl: Cyclobuten-1-yl, Cyclobuten-3-yl, Cyclopenten-1-yl, Cyclopenten-3-yl, Cyclopenten-4-yl, Cyclohexen-1-yl, Cyclohexen-3-yl und Cyclohexen-4-yl;
- Heterocyclyl, sowie die Heteroxyclylreste in Heterocyclyloxy: drei- bis siebengliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, wie Oxiranyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofuran-2-yl, 2,3-Dihydrofuran-3-yl, 2,3-Dihydrofuran-4-yl, 2,3-Dihydrofuran-5-yl, 2,5-Dihydrofuran-2-yl, 2,5-Dihydrofuran-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroxazol-5-yl, 2,3-Dihydroisothiazol-3-yl, 2,3-Dihydroisothiazol-4-yl, 2,3-Dihydroisothiazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydropyrazol-3-yl, 2,3-Dihydropyrazol-4-yl, 2,3-Dihydropyrazol-5-yl, 4,5-Dihydropyrazol-3-yl, 4,5-Dihydropyrazol-4-yl, 4,5-Dihydropyrazol-5-yl, 2,5-Dihydropyrazol-3-yl, 2,5-Dihydropyrazol-4-yl, 2,5-Dihydropyrazol-5-yl, 2,3-Dihydrooxazol-2-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 3-Tetrahydropyranyl, 4-Tetrahydropyranyl, 2-Tetrahydrothiopyranyl, 3-Tetrahydrothiopyranyl, 4-Tetrahydrothiopyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl,
- Hetaryl, sowie die Hetarylreste in Hetaryloxy: aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol- 2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4,5-Tetrazin-3-yl, sowie die entsprechenden benzokondensierten Derivate.

Alle Phenyl-, Hetaryl- und Heterocyclylringe sind vorzugsweise unsubstituiert oder tragen ein bis drei Halogenatome und/oder einen oder zwei Reste aus folgender Gruppe: Nitro, Cyano, Methyl, Trifluormethyl, Methoxy, Trifluormethoxy oder Methoxycarbonyl.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹: Nitro, Halogen, Cyano, Rhodano, C₁-C₆-Alkyl, C₁-C₆-Halogen-alkyl, C₁-C₆-Alkoxy-C₁-C₆-alkyl, C₂-C₆-Alkenyl, C₂-C₆-Alkinyl, -OR³ oder -S(O)ₙR³;
besonders bevorzugt Nitro, Halogen wie z.B. Fluor, Chlor oder Brom, C₁-C₆-Halogenalkyl, -OR³ oder -SO₂R³;
- R²: Wasserstoff, Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR³ oder -S(O)ₙR³;
besonders bevorzugt Wasserstoff, Nitro, Halogen wie z.B. Fluor, Chlor oder Brom, C₁-C₆-Alkyl, C₁-C₆-Halogenalkyl, -OR³ oder -SO₂R³;
n 0, 1 oder 2, besonders bevorzugt 0 oder 2;
- R³: Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl; besonders bevorzugt Wasserstoff, C₁-C₄-Alkyl, C₁-C₄-Halogen-alkyl, C₂-C₃-Alkenyl, C₂-C₃-Alkinyl oder Phenyl; wobei die genannten Alkylreste partiell oder vollständig halogeniert sein können und/oder eine bis drei der folgenden Gruppen tragen können:
Hydroxy, Mercapto Amino, Cyano, R³, -OR³, -SR³, -N(R³)₂, =NOR³, -OCOR³, -SCOR³, -NR³COR³, -CO₂R³, -COSR³, -CON(R³)₂, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits wie angegeben substituiert sein können;
- R⁴: Wasserstoff, C₁-C₆-Alkyl oder C₁-C₆-Halogenalkyl; besonders bevorzugt Wasserstoff, Methyl, Ethyl oder Trifluormethyl;
- R⁵: C₁-C₆-Alkyl oder C₁-C₆-Halogenalkyl; besonders bevorzugt Methyl, Ethyl, Propyl, Isopropyl, Butyl oder Isobutyl;
- R⁶: Wasserstoff, C₁-C₆-Alkyl, C₁-C₆-Alkylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, Phenylcarbonylmethyl, oder Phenylsulfonyl, wobei der Phenylring der zwei letztgenannten Substituenten partiell oder vollständig halogeniert sein kann und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy;
- X¹: eine geradkettige oder verzweigte C₁-C₄-Alkylen-, eine C₂-C₄-Alkenylen- oder eine C₂-C₄-Alkinylenkette, besonders bevorzugt eine Ethylen-, Propylen-, Propenylen- oder Propinylenkette, die durch ein Heteroatom ausgewählt aus der Gruppe Sauerstoff oder Schwefel, bevorzugt Sauerstoff
unterbrochen ist, wobei die genannten Alkylen-, Alkenylen- oder Alkinylenreste partiell halogeniert sein können und/oder eine bis drei der folgenden Gruppen tragen können:
-OR⁷, -OCOR⁷, -OCONHR⁷ oder -OSO₂R⁷;
- R⁷: Wasserstoff, C₁-C₄-Alkyl, C₂-C₄-Alkenyl, C₂-C₄-Alkinyl, Phenyl, Phenyl-C₁-C₄-alkyl, wobei die genannten Alkyl-, Alkenyl oder Alkinylreste partiell oder vollständig halogeniert sein können und/oder durch einen oder mehrere der folgenden Reste substituiert sein können:
Hydroxy, Mercapto, Amino, Cyano, Nitro, Formyl, G₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy,
C₁-C₄-Halogenalkoxy;
- Het: eine wie oben definierte drei- bis sechsgliedrige, bevorzugt eine fünf- oder sechsgliedrige, teilweise oder vollständig gesättigte, heterocyclische Gruppe oder eine drei- bis sechsgliedrige, bevorzugt fünf- oder sechsgliedrige heteroaromatische Gruppe mit bis zu drei Heteroatomen, besonders bevorzugt mit einem oder zwei Heteroatomen ausgewählt aus folgenden drei Gruppen:
Stickstoff,
Sauerstoff in Kombination mit mindestens einem Stickstoff oder
Schwefel in Kombination mit mindestens einem Stickstoff,
besonders bevorzugt aus folgenden beiden Gruppen:
Stickstoff oder
Sauerstoff in Kombination mit mindestens einem Stickstoff,
wobei die gennante heterocyclische oder heteroaromatische Gruppe partiell oder vollständig halogeniert sein kann und/ oder durch R⁸ substituiert sein kann;
- R⁸: Wasserstoff, Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy, wobei die Alkylreste in allen Fällen jeweils durch einen oder mehrere der folgenden Reste substituiert sein können:
Cyano, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁₋C₄-Halogenalkoxy.

Insbesondere bevorzugt sind die Verbindungen der Formel Ia, wobei R¹ in Position 2 und R² in Position 4 des Phenylringes gebunden sind.

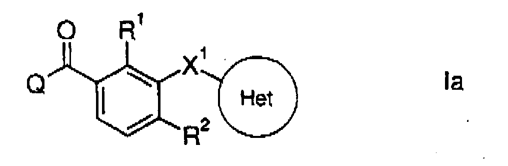

Außerordentlich bevorzugt sind die Verbindungen der Formel Ia, in der die Substituenten R¹ und R² und Q die oben genannte Bedeutung haben, X¹ für eine durch ein Sauerstoffatom unterbrochene C₁-C₃-Alkylen-, eine C₂-C₃-Alkenylen- oder C₂-C₃-Alkinylenkette steht und
- Het: eine wie oben definierte drei- bis sechsgliedrige, bevorzugt eine fünf- oder sechsgliedrige, teilweise oder vollständig gesättigte, heterocyclische Gruppe oder eine drei- bis sechsgliedrige, bevorzugt fünf- oder sechsgliedrige heteroaromatische Gruppe mit bis zu drei Heteroatomen, besonders bevorzugt mit einem oder zwei Heteroatomen ausgewählt aus folgenden drei Gruppen:
Stickstoff,
Sauerstoff in Kombination mit mindestens einem Stickstoff oder
Schwefel in Kombination mit mindestens einem Stickstoff,
besonders bevorzugt aus folgenden beiden Gruppen:
Stickstoff oder
Sauerstoff in Kombination mit mindestens einem Stickstoff,
wobei die gennante heterocyclische oder heteroaromatische Gruppe partiell oder vollständig halogeniert sein kann und/ oder durch R⁸ substituiert sein kann;
bedeutet.

Darüber hinaus sind die erfindungsgemäßen Verbindungen der Formel Ia außerordentlich bevorzugt, in der die Substituenten R¹ R² und X¹ die oben genannte Bedeutung haben und Het für eine wie oben definierte fünf- oder sechsgliedrige, teilweise oder vollständig gesättigte, heterocyclische Gruppe oder eine fünf- oder sechsgliedrige heteroaromatische Gruppe mit bis zu drei Heteroatomen, besonders bevorzugt mit einem oder zwei Heteroatomen ausgewählt aus folgenden drei Gruppen:
Stickstoff ,
Sauerstoff in Kombination mit mindestens einem Stickstoff oder
Schwefel in Kombination mit mindestens einem Stickstoff,
besonders bevorzugt aus folgenden beiden Gruppen:
   Stickstoff oder
   Sauerstoff in Kombination mit mindestens einem Stickstoff,
   wobei die gennante heterocyclische oder heteroaromatische Gruppe partiell oder vollständig halogeniert sein kann und/oder durch R⁸ substituiert sein kann;
   steht.

Insbesondere bevorzugt sind die Verbindungen Ib der Tabellen 1 bis 144.

| Nr. | X¹ | Het |
|---|---|---|
| 620 | CH₂OCH₂CH=CH | 5-Methyl-4-isoxazolyl |
| 621 | CH₂OCH₂CH=CH | 5-Cyclopropyl-4-isoxazolyl |
| 622 | CH₂OCH₂CH=CH | 5-Phenyl-4-isoxazolyl |
| 623 | CH₂OCH₂CH=CH | 3,5-Dimethyl-4-isoxazolyl |
| 624 | CH₂OCH₂CH=CH | 4,5-Dihydro-4-isoxazolyl |
| 625 | CH₂OCH₂CH=CH | 3-Methyl-4,5-dihydro-4-isoxazolyl |
| 626 | CH₂OCH₂CH=CH | 5-Methyl-4,5-dihydro-4-isoxazolyl |
| 627 | CH₂OCH₂CH=CH | 3,5-Dimethyl-4,5-dihydro-4-isoxazolyl |
| 628 | CH₂OCH₂CH=CH | 5-Isoxazolyl |
| 629 | CH₂OCH₂CH=CH | 3-Methyl-5-isoxazolyl |
| 630 | CH₂OCH₂CH=CH | 4-Methyl-5-isoxazolyl |
| 631 | CH₂OCH₂CH=CH | 3,4-Dimethyl-5-isoxazolyl |
| 632 | CH₂OCH₂CH=CH | 4,5-Dihydro-5-isoxazolyl |
| 633 | CH₂OCH₂CH=CH | 3-Methyl-4,5-dihydro-5-isoxazolyl |
| 634 | CH₂OCH₂CH=CH | 4-Methyl-4,5-dihydro-5-isoxazolyl |
| 635 | CH₂OCH₂CH=CH | 3,4-Dimethyl-4,5-dihydro-5-isoxazolyl |
| 636 | CH₂OCH₂CH=CH | 3-Isothiazolyl |
| 637 | CH₂OCH₂CH=CH | 4-Methyl-3-isothiazolyl |
| 638 | CH₂OCH₂CH=CH | 5-Methyl-3-isothiazolyl |
| 639 | CH₂OCH₂CH=CH | 4-Isothiazolyl |
| 640 | CH₂OCH₂CH=CH | 3-Methyl-4-isothiazolyl |
| 641 | CH₂OCH₂CH=CH | 5-Methyl-4-isothiazolyl |
| 642 | CH₂OCH₂CH=CH | 5-Isothiazolyl |
| 643 | CH₂OCH₂CH=CH | 3-Methyl-5-isothiazolyl |
| 644 | CH₂OCH₂CH=CH | 4-Methyl-5-isothiazolyl |
| 645 | CH₂OCH₂CH=CH | 2-Oxazolyl |
| 646 | CH₂OCH₂CH=CH | 4-Oxazolyl |
| 647 | CH₂OCH₂CH=CH | 5-Oxazolyl |
| 648 | CH₂OCH₂CH=CH | 2-Thiazolyl |
| 649 | CH₂OCH₂CH=CH | 4-Thiazolyl |
| 650 | CH₂OCH₂CH=CH | 5-Thiazolyl |
| 651 | CH₂OCH₂CH=CH | 3-Pyrazolyl |
| 652 | CH₂OCH₂CH=CH | 4-Pyrazolyl |
| 653 | CH₂OCH₂CH=CH | 1-Methyl-3-pyrazolyl |
| 654 | CH₂OCH₂CH=CH | 1-Methyl-4-pyrazolyl |
| 655 | CH₂OCH₂CH=CH | 1-Methyl-5-pyrazolyl |
| 656 | CH₂OCH₂CH=CH | 2-Imidazolyl |
| 657 | CH₂OCH₂CH=CH | 1-Methyl-2-imidazolyl |
| 658 | CH₂OCH₂CH=CH | 5-Methyl-[1,3,4]-2-oxadiazolyl |
| 659 | CH₂OCH₂CH=CH | 5-Methyl-[1,2,4]-3-oxadiazolyl |
| 660 | CH₂OCH₂CH=CH | 5-Methyl-[1,3,4]-2-thiadiazolyl |
| 661 | CH₂OCH₂CH=CH | 5-Methyl-[1,2,4]-3-thiadiazolyl |
| 662 | CH₂OCH₂CH=CH | [1,2,4]-3-triazolyl |
| 663 | CH₂OCH₂CH=CH | [1,2,3]-4-triazolyl |
| 664 | CH₂OCH₂CH=CH | 2-Pyridyl |
| 665 | CH₂OCH₂CH=CH | 6-Chlor-2-pyridyl |
| 666 | CH₂OCH₂CH=CH | 6-Methoxy-2-pyridyl |
| 667 | CH₂OCH₂CH=CH | 6-Trifluormethyl-2-pyridyl |
| 668 | CH₂OCH₂CH=CH | 3-Pyridyl |
| 669 | CH₂OCH₂CH=CH | 2-Chlor-3-pyridyl |
| 670 | CH₂OCH₂CH=CH | 2-Methoxy-3-pyridyl |
| 671 | CH₂OCH₂CH=CH | 4-Pyridyl |
| 672 | CH₂OCH₂CH=CH | 2-Chlor-4-pyridyl |
| 673 | CH₂OCH₂CH=CH | 2-Methoxy-4-pyridyl |
| 674 | CH₂OCH₂CH=CH | 2-Ethoxy-4-pyridyl |
| 675 | CH₂OCH₂CH=CH | 2-Methylthio-4-pyridyl |
| 676 | CH₂OCH₂CH=CH | 2-Trifluormethyl-5-pyridyl |
| 677 | CH₂OCH₂CH=CH | 2-Pyrimidinyl |
| 678 | CH₂OCH₂CH=CH | 3-Pyrimidinyl |
| 679 | CH₂OCH₂CH=CH | 4-Pyrimidinyl |
| 680 | CH₂OCH₂CH=CH | 2-Pyrazinyl |
| 681 | CH₂OCH₂CH=CH | 3-Pyridazinyl |
| 682 | CH₂OCH₂CH=CH | 4-Pyridazinyl |
| 683 | CH₂OCH₂CH=CH | 2-(2*H*-1,3-oxazinyl) |
| 684 | CH₂OCH₂CH=CH | 2-(6*H*-1,3-oxazinyl) |
| 685 | CH₂OCH₂CH=CH | 4-(6*H*-1,3-oxazinyl) |
| 686 | CH₂OCH₂CH=CH | 6-(6*H*-1,3-oxazinyl) |
| 687 | CH₂OCH₂CH=CH | [1,3,5]-2-Triazinyl |
| 688 | CH₂OCH₂CH=CH | [1,2,4]-3-Triazinyl |
| 689 | CH₂OCH₂CH=CH | [1,2,4]-5-Triazinyl |
| 690 | CH₂OCH₂CH=CH | [1,2,4]-6-Triazinyl |
| 691 | CH=CHCH₂O | Oxiranyl |
| 692 | CH=CHCH₂O | 3-Methyl-2-oxiranyl |
| 693 | CH=CHCH₂O | 2-Oxetanyl |
| 694 | CH=CHCH₂O | 3-Hydroxy-3-methyl-2-oxetanyl |
| 695 | CH=CHCH₂O | 3-Hydroxy-3-ethyl-2-oxetanyl |
| 696 | CH=CHCH₂O | 3-Hydroxy-3-propyl-2-oxetanyl |
| 697 | CH=CHCH₂O | 3-Hydroxy-3-butyl-2-oxetanyl |
| 698 | CH=CHCH₂O | 3-Methoxy-3-methyl-2-oxetanyl |
| 699 | CH=CHCH₂O | 3-Methoxy-3-ethyl-2-oxetanyl |
| 700 | CH=CHCH₂O | 3-Methoxy-3-propyl-2-oxetanyl |
| 701 | CH=CHCH₂O | 3-Methoxy-3-butyl-2-oxetanyl |
| 702 | CH=CHCH₂O | 3-Trimethylsilyloxy-3-methyl-2-oxetanyl |
| 703 | CH=CHCH₂O | 3-Trimethylsilyloxy-3-ethyl-2-oxetanyl |
| 704 | CH=CHCH₂O | 3-Trimethylsilyloxy-3-propyl-2-oxetanyl |
| 705 | CH=CHCH₂O | 3-Trimethylsilyloxy-3-butyl-2-oxetanyl |
| 706 | CH=CHCH₂O | 3-Oxetanyl |
| 707 | CH=CHCH₂O | 2-Furyl |
| 708 | CH=CHCH₂O | 4,5-Dihydro-2-furyl |
| 709 | CH=CHCH₂O | 2,3-Dihydro-2-furyl |
| 710 | CH=CHCH₂O | 3-Furyl |
| 711 | CH=CHCH₂O | 4,5-Dihydro-3-furyl |
| 712 | CH=CHCH₂O | 2,3-Dihydro-3-furyl |
| 713 | CH=CHCH₂O | 2-Thienyl |
| 714 | CH=CHCH₂O | 4,5-Dihydro-2-thienyl |
| 715 | CH=CHCH₂O | 2,3-Dihydro-2-thienyl |
| 716 | CH=CHCH₂O | 5-Chlor-2-thienyl |
| 717 | CH=CHCH₂O | 5-Methyl-2-thienyl |
| 718 | CH=CHCH₂O | 3-Thienyl |
| 719 | CH=CHCH₂O | 4,5-Dihydro-3-thienyl |
| 720 | CH=CHCH₂O | 2,3-Dihydro-3-thienyl |
| 721 | CH=CHCH₂O | 2-Pyrrolyl |
| 722 | CH=CHCH₂O | 2,5-Dihydro-2-pyrrolyl |
| 723 | CH=CHCH₂O | 3-Pyrrol |
| 724 | CH=CHCH₂O | 2,5-Dihydro-3-pyrrolyl |
| 725 | CH=CHCH₂O | 3-Isoxazolyl |
| 726 | CH=CHCH₂O | 4-Methyl-3-isoxazolyl |
| 727 | CH=CHCH₂O | 5-Methyl-3-isoxazolyl |
| 728 | CH=CHCH₂O | 4,5-Dimethyl-3-isoxazolyl |
| 729 | CH=CHCH₂O | 4,5-Dihydro-3-isoxazolyl |
| 730 | CH=CHCH₂O | 4-Methyl-4,5-dihydro-3-isoxazolyl |
| 731 | CH=CHCH₂O | 5-Methyl-4,5-dihydro-3-isoxazolyl |
| 732 | CH=CHCH₂O | 4,5-Dimethyl-4,5-dihydro-3-isoxazolyl |
| 733 | CH=CHCH₂O | 4-Isoxazolyl |
| 734 | CH=CHCH₂O | 3-Methyl-4-isoxazolyl |
| 735 | CH=CHCH₂O | 5-Methyl-4-isoxazolyl |
| 736 | CH=CHCH₂O | 5-Cyclopropyl-4-isoxazolyl |
| 737 | CH=CHCH₂O | 5-Phenyl-4-isoxazolyl |
| 738 | CH=CHCH₂O | 3,5-Dimethyl-4-isoxazolyl |
| 739 | CH=CHCH₂O | 4,5-Dihydro-4-isoxazolyl |
| 740 | CH=CHCH₂O | 3-Methyl-4,5-dihydro-4-isoxazolyl |
| 741 | CH=CHCH₂O | 5-Methyl-4,5-dihydro-4-isoxazolyl |
| 742 | CH=CHCH₂O | 3,5-Dimethyl-4,5-dihydro-4-isoxazolyl |
| 743 | CH=CHCH₂O | 5-Isoxazolyl |
| 744 | CH=CHCH₂O | 3-Methyl-5-isoxazolyl |
| 745 | CH=CHCH₂O | 4-Methyl-5-isoxazolyl |
| 746 | CH=CHCH₂O | 3,4-Dimethyl-5-isoxazolyl |
| 747 | CH=CHCH₂O | 4,5-Dihydro-5-isoxazolyl |
| 748 | CH=CHCH₂O | 3-Methyl-4,5-dihydro-5-isoxazolyl |
| 749 | CH=CHCH₂O | 4-Methyl-4,5-dihydro-5-isoxazolyl |
| 750 | CH=CHCH₂O | 3,4-Dimethyl-4,5-dihydro-5-isoxazolyl |
| 751 | CH=CHCH₂O | 3-Isothiazolyl |
| 752 | CH=CHCH₂O | 4-Methyl-3-isothiazolyl |
| 753 | CH=CHCH₂O | 5-Methyl-3-isothiazolyl |
| 754 | CH=CHCH₂O | 4-Isothiazolyl |
| 755 | CH=CHCH₂O | 3-Methyl-4-isothiazolyl |
| 756 | CH=CHCH₂O | 5-Methyl-4-isothiazolyl |
| 757 | CH=CHCH₂O | 5-Isothiazolyl |
| 758 | CH=CHCH₂O | 3-Methyl-5-isothiazolyl |
| 759 | CH=CHCH₂O | 4-Methyl-5-isothiazolyl |
| 760 | CH=CHCH₂O | 2-Oxazolyl |
| 761 | CH=CHCH₂O | 4-Oxazolyl |
| 762 | CH=CHCH₂O | 5-Oxazolyl |
| 763 | CH=CHCH₂O | 2-Thiazolyl |
| 764 | CH=CHCH₂O | 4-Thiazolyl |
| 765 | CH=CHCH₂O | 5-Thiazolyl |
| 766 | CH=CHCH₂O | 3-Pyrazolyl |
| 767 | CH=CHCH₂O | 4-Pyrazolyl |
| 768 | CH=CHCH₂O | 1-Methyl-3-pyrazolyl |
| 769 | CH=CHCH₂O | 1-Methyl-4-pyrazolyl |
| 770 | CH=CHCH₂O | 1-Methyl-5-pyrazolyl |
| 771 | CH=CHCH₂O | 2-Imidazolyl |
| 772 | CH=CHCH₂O | 1-Methyl-2-imidazolyl |
| 773 | CH=CHCH₂O | 5-Methyl-[1,3,4]-2-oxadiazolyl |
| 774 | CH=CHCH₂O | 5-Methyl-[1,2,4]-3-oxadiazolyl |
| 775 | CH=CHCH₂O | 5-Methyl-[1,3,4]-2-thiadiazolyl |
| 776 | CH=CHCH₂O | 5-Methyl-[1,2,4]-3-thiadiazolyl |
| 777 | CH=CHCH₂O | [1,2,4]-3-triazolyl |
| 778 | CH=CHCH₂O | [1,2,3]-4-triazolyl |
| 779 | CH=CHCH₂O | 2-Pyridyl |
| 780 | CH=CHCH₂O | 6-Chlor-2-pyridyl |
| 781 | CH=CHCH₂O | 6-Methoxy-2-pyridyl |
| 782 | CH=CHCH₂O | 6-Trifluormethyl-2-pyridyl |
| 783 | CH=CHCH₂O | 3-Pyridyl |
| 784 | CH=CHCH₂O | 2-Chlor-3-pyridyl |
| 785 | CH=CHCH₂O | 2-Methoxy-3-pyridyl |
| 786 | CH=CHCH₂O | 4-Pyridyl |
| 787 | CH=CHCH₂O | 2-Chlor-4-pyridyl |
| 788 | CH=CHCH₂O | 2-Methoxy-4-pyridyl |
| 789 | CH=CHCH₂O | 2-Ethoxy-4-pyridyl |
| 790 | CH=CHCH₂O | 2-Methylthio-4-pyridyl |
| 791 | CH=CHCH₂O | 2-Trifluormethyl-5-pyridyl |
| 792 | CH=CHCH₂O | 2-Pyrimidinyl |
| 793 | CH=CHCH₂O | 3-Pyrimidinyl |
| 794 | CH=CHCH₂O | 4-Pyrimidinyl |
| 795 | CH=CHCH₂O | 2-Pyrazinyl |
| 796 | CH=CHCH₂O | 3-Pyridazinyl |
| 797 | CH=CHCH₂O | 4-Pyridazinyl |
| 798 | CH=CHCH₂O | 2-(2*H*-1,3-oxazinyl) |
| 799 | CH=CHCH₂O | 2-(6*H*-1,3-oxazinyl) |
| 800 | CH=CHCH₂O | 4-(6*H*-1,3-oxazinyl) |
| 801 | CH=CHCH₂O | 6-(6*H*-1,3-oxazinyl) |
| 802 | CH=CHCH₂O | [1,3,5]-2-Triazinyl |
| 803 | CH=CHCH₂O | [1,2,4]-3-Triazinyl |
| 804 | CH=CHCH₂O | [1,2,4]-5-Triazinyl |
| 805 | CH=CHCH₂O | [1,2,4]-6-Triazinyl |
| 806 | -C≡C-CH₂O | Oxiranyl |
| 807 | -C≡C-CH₂O | 3-Methyl-2-oxiranyl |
| 808 | -C≡C-CH₂O | 2-Oxetanyl |
| 809 | -C≡C-CH₂O | 3-Hydroxy-3-methyl-2-oxetanyl |
| 810 | -C≡C-CH₂O | 3-Hydroxy-3-ethyl-2-oxetanyl |
| 811 | -C≡C-CH₂O | 3-Hydroxy-3-propyl-2-oxetanyl |
| 812 | -C≡C-CH₂O | 3-Hydroxy-3-butyl-2-oxetanyl |
| 813 | -C≡C-CH₂O | 3-Methoxy-3-methyl-2-oxetanyl |
| 814 | -C≡C-CH₂O | 3-Methoxy-3-ethyl-2-oxetanyl |
| 815 | -C≡C-CH₂O | 3-Methoxy-3-propyl-2-oxetanyl |
| 816 | -C≡C-CH₂O | 3-Methoxy-3-butyl-2-oxetanyl |
| 817 | -C≡C-CH₂O | 3-Trimethylsilyloxy-3-methyl-2-oxetanyl |
| 818 | -C≡C-CH₂O | 3-Trimethylsilyloxy-3-ethyl-2-oxetanyl |
| 819 | -C≡C-CH₂O | 3-Trimethylsilyloxy-3-propyl-2-oxetanyl |
| 820 | -C≡C-CH₂O | 3-Trimethylsilyloxy-3-butyl-2-oxetanyl |
| 821 | -C≡C-CH₂O | 3-Oxetanyl |
| 822 | -C≡C-CH₂O | 2-Furyl |
| 823 | -C≡C-CH₂O | 4,5-Dihydro-2-furyl |
| 824 | -C≡C-CH₂O | 2,3-Dihydro-2-furyl |
| 825 | -C≡C-CH₂O | 3-Furyl |
| 826 | -C≡C-CH₂O | 4,5-Dihydro-3-furyl |
| 827 | -C≡C-CH₂O | 2,3-Dihydro-3-furyl |
| 828 | -C≡C-CH₂O | 2-Thienyl |
| 829 | -C≡C-CH₂O | 4,5-Dihydro-2-thienyl |
| 830 | -C≡C-CH₂O | 2,3-Dihydro-2-thienyl |
| 831 | -C≡C-CH₂O | 5-Chlor-2-thienyl |
| 832 | -C≡C-CH₂O | 5-Methyl-2-thienyl |
| 833 | -C≡C-CH₂O | 3-Thienyl |
| 834 | -C≡C-CH₂O | 4,5-Dihydro-3-thienyl |
| 835 | -C≡C-CH₂O | 2,3-Dihydro-3-thienyl |
| 836 | -C≡C-CH₂O | 2-Pyrrolyl |
| 837 | -C≡C-CH₂O | 2,5-Dihydro-2-pyrrolyl |
| 838 | -C≡C-CH₂O | 3-Pyrrol |
| 839 | -C≡C-CH₂O | 2,5-Dihydro-3-pyrrolyl |
| 840 | -C≡C-CH₂O | 3-Isoxazolyl |
| 841 | -C≡C-CH₂O | 4-Methyl-3-isoxazolyl |
| 842 | -C≡C-CH₂O | 5-Methyl-3-isoxazolyl |
| 843 | -C≡C-CH₂O | 4,5-Dimethyl-3-isoxazolyl |
| 844 | -C≡C-CH₂O | 4,5-Dihydro-3-isoxazolyl |
| 845 | -C≡C-CH₂O | 4-Methyl-4,5-dihydro-3-isoxazolyl |
| 846 | -C≡C-CH₂O | 5-Methyl-4,5-dihydro-3-isoxazolyl |
| 847 | -C≡C-CH₂O | 4,5-Dimethyl-4,5-dihydro-3-isoxazolyl |
| 848 | -C≡C-CH₂O | 4-Isoxazolyl |
| 849 | -C≡C-CH₂O | 3-Methyl-4-isoxazolyl |
| 850 | -C≡C-CH₂O | 5-Methyl-4-isoxazolyl |
| 851 | -C≡C-CH₂O | 5-Cyclopropyl-4-isoxazolyl |
| 852 | -C≡C-CH₂O | 5-Phenyl-4-isoxazolyl |
| 853 | -C≡C-CH₂O | 3,5-Dimethyl-4-isoxazolyl |
| 854 | -C≡C-CH₂O | 4,5-Dihydro-4-isoxazolyl |
| 855 | -C≡C-CH₂O | 3-Methyl-4,5-dihydro-4-isoxazolyl |
| 856 | -C≡C-CH₂O | 5-Methyl-4,5-dihydro-4-isoxazolyl |
| 857 | -C≡C-CH₂O | 3,5-Dimethyl-4,5-dihydro-4-isoxazolyl |
| 858 | -C≡C-CH₂O | 5-Isoxazolyl |
| 859 | -C≡C-CH₂O | 3-Methyl-5-isoxazolyl |
| 860 | -C≡C-CH₂O | 4-Methyl-5-isoxazolyl |
| 861 | -C≡C-CH₂O | 3,4-Dimethyl-5-isoxazolyl |
| 862 | -C≡C-CH₂O | 4,5-Dihydro-5-isoxazolyl |
| 863 | -C≡C-CH₂O | 3-Methyl-4,5-dihydro-5-isoxazolyl |
| 864 | -C≡C-CH₂O | 4-Methyl-4,5-dihydro-5-isoxazolyl |
| 865 | -C≡C-CH₂O | 3,4-Dimethyl-4,5-dihydro-5-isoxazolyl |
| 866 | -C≡C-CH₂O | 3-Isothiazolyl |
| 867 | -C≡C-CH₂O | 4-Methyl-3-isothiazolyl |
| 868 | -C≡C-CH₂O | 5-Methyl-3-isothiazolyl |
| 869 | -C≡C-CH₂O | 4-Isothiazolyl |
| 870 | -C≡C-CH₂O | 3-Methyl-4-isothiazolyl |
| 871 | -C≡C-CH₂O | 5-Methyl-4-isothiazolyl |
| 872 | -C≡C-CH₂O | 5-Isothiazolyl |
| 873 | -C≡C-CH₂O | 3-Methyl-5-isothiazolyl |
| 874 | -C≡C-CH₂O | 4-Methyl-5-isothiazolyl |
| 875 | -C≡C-CH₂O | 2-Oxazolyl |
| 876 | -C≡C-CH₂O | 4-Oxazolyl |
| 877 | -C≡C-CH₂O | 5-Oxazolyl |
| 878 | -C≡C-CH₂O | 2-Thiazolyl |
| 879 | -C≡C-CH₂O | 4-Thiazolyl |
| 880 | -C≡C-CH₂O | 5-Thiazolyl |
| 881 | -C≡C-CH₂O | 3-Pyrazolyl |
| 882 | -C≡C-CH₂O | 4-Pyrazolyl |
| 883 | -C≡C-CH₂O | 1-Methyl-3-pyrazolyl |
| 884 | -C≡C-CH₂O | 1-Methyl-4-pyrazolyl |
| 885 | -C≡C-CH₂O | 1-Methyl-5-pyrazolyl |
| 886 | -C≡C-CH₂O | 2-Imidazolyl |
| 887 | -C≡C-CH₂O | 1-Methyl-2-imidazolyl |
| 888 | -C≡C-CH₂O | 5-Methyl-[1,3,4]-2-oxadiazolyl |
| 889 | -C≡C-CH₂O | 5-Methyl-[1,2,4]-3-oxadiazolyl |
| 890 | -C≡C-CH₂O | 5-Methyl-[1,3,4]-2-thiadiazolyl |
| 891 | -C≡C-CH₂O | 5-Methyl-[1,2,4]-3-thiadiazolyl |
| 892 | -C≡C-CH₂O | [1,2,4]-3-triazolyl |
| 893 | -C≡C-CH₂O | [1,2,3]-4-triazolyl |
| 894 | -C≡C-CH₂O | 2-Pyridyl |
| 895 | -C≡C-CH₂O | 6-Chlor-2-pyridyl |
| 896 | -C≡C-CH₂O | 6-Methoxy-2-pyridyl |
| 897 | -C≡C-CH₂O | 6-Trifluormethyl-2-pyridyl |
| 898 | -C≡C-CH₂O | 3-Pyridyl |
| 899 | -C≡C-CH₂O | 2-Chlor-3-pyridyl |
| 900 | -C≡C-CH₂O | 2-Methoxy-3-pyridyl |
| 901 | -C≡C-CH₂O | 4-Pyridyl |
| 902 | -C≡C-CH₂O | 2-Chlor-4-pyridyl |
| 903 | -C≡C-CH₂O | 2-Methoxy-4-pyridyl |
| 904 | -C≡C-CH₂O | 2-Ethoxy-4-pyridyl |
| 905 | -C≡C-CH₂O | 2-Methylthio-4-pyridyl |
| 906 | -C≡C-CH₂O | 2-Trifluormethyl-5-pyridyl |
| 907 | -C≡C-CH₂O | 2-Pyrimidinyl |
| 908 | -C≡C-CH₂O | 3-Pyrimidinyl |
| 909 | -C≡C-CH₂O | 4-Pyrimidinyl |
| 910 | -C≡C-CH₂O | 2-Pyrazinyl |
| 911 | -C≡C-CH₂O | 3-Pyridazinyl |
| 912 | -C≡C-CH₂O | 4-Pyridazinyl |
| 913 | -C≡C-CH₂O | 2-(2*H*-1,3-oxazinyl) |
| 914 | -C≡C-CH₂O | 2-(6*H*-1,3-oxazinyl) |
| 915 | -C≡C-CH₂O | 4-(6*H*-1,3-oxazinyl) |
| 916 | -C≡C-CH₂O | 6-(6*H*-1,3-oxazinyl) |
| 917 | -C≡C-CH₂O | [1,3,5]-2-Triazinyl |
| 918 | -C≡C-CH₂O | [1,2,4]-3-Triazinyl |
| 919 | -C≡C-CH₂O | [1,2,4]-5-Triazinyl |
| 920 | -C≡C-CH₂O | [1,2,4]-6-Triazinyl |

Die folgenden Tabellen 1 - 144 basieren auf den 4-Benzoyl-pyrazolen der Formel Ib.

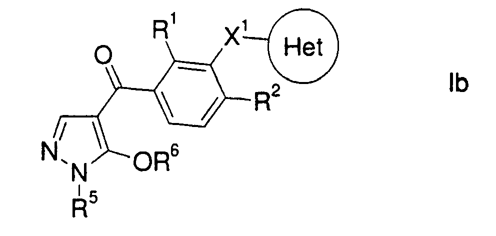

### Tabelle 1: Verbindungen 1.1 - 1.920

Verbindungen der allgemeinen Formel Ib, in der R¹ Chlor und R² Chlor, R⁵ Methyl und R⁶ Wasserstoff bedeutet und die Substituenten X¹ und Het für jede einzelne Verbindung jeweils einer Zeile der Tabelle A entsprechen.

### Tabelle 2: Verbindungen 2.1 - 2.920

Verbindungen der allgemeinen Formel Ib, in der R¹ Chlor und R² Chlor, R⁵ Ethyl und R⁶ Wasserstoff bedeutet und die Substituenten X¹ und Het für jede einzelne Verbindung jeweils einer Zeile der Tabelle A entsprechen.

### Tabelle 3: Verbindungen 3.1 - 3.920

Verbindungen der allgemeinen Formel Ib, in der R¹ Chlor und R² Chlor, R⁵ n-Propyl und R⁶ Wasserstoff bedeutet und die Substituenten X¹ und Het für jede einzelne Verbindung jeweils einer Zeile der Tabelle A entsprechen.

### Tabelle 4: Verbindungen 4.1 - 4.920

Verbindungen der allgemeinen Formel Ib, in der R¹ Chlor und R² Chlor, R⁵ Methyl und R⁶ Methyl bedeutet und die Substituenten X¹ und Het für jede einzelne Verbindung jeweils einer Zeile der Tabelle A entsprechen.

### Tabelle 5: Verbindungen 5.1 - 5.920

Verbindungen der allgemeinen Formel Ib, in der R¹ Chlor und R² Chlor, R⁵ Ethyl und R⁶ Methyl bedeutet und die Substituenten X¹ und Het für jede einzelne Verbindung jeweils einer Zeile der Tabelle A entsprechen.

### Tabelle 6: Verbindungen 6.1 - 6.920

Verbindungen der allgemeinen Formel Ib, in der R¹ Chlor und R² Chlor, R⁵ n-Propyl und R⁶ Methyl bedeutet und die Substituenten X¹ und Het für jede einzelne Verbindung jeweils einer Zeile der Tabelle A entsprechen.

### Tabelle 7: Verbindungen 7.1 - 7.920

Verbindungen der allgemeinen Formel Ib, in der R¹ Chlor und R² Chlor, R⁵ Methyl und R⁶ Ethyl bedeutet und die Substituenten X¹ und Het für jede einzelne Verbindung jeweils einer Zeile der Tabelle A entsprechen.

### Tabelle 8: Verbindungen 8.1 - 8.920

Verbindungen der allgemeinen Formel Ib, in der R¹ Chlor und R² Chlor, R⁵ Ethyl und R⁶ Ethyl bedeutet und die Substituenten X¹ und Het für jede einzelne Verbindung jeweils einer Zeile der Tabelle A entsprechen.

### Tabelle 9: Verbindungen 9.1 - 9.920

Verbindungen der allgemeinen Formel Ib, in der R¹ Chlor und R² Chlor, R⁵ n-Propyl und R⁶ Ethyl bedeutet und die Substituenten X¹ und Het für jede einzelne Verbindung jeweils einer Zeile der Tabelle A entsprechen.

### Tabelle 10: Verbindungen 10.1 - 10.920

Verbindungen der allgemeinen Formel Ib, in der R¹ Chlor und R² Chlor, R⁵ Methyl und R⁶ Methylcarbonyl bedeutet und die Substituenten X¹ und Het für jede einzelne Verbindung jeweils einer Zeile der Tabelle A entsprechen.

### Tabelle 11: Verbindungen 11.1 - 11.920

Verbindungen der allgemeinen Formel Ib, in der R¹ Chlor und R² Chlor, R⁵ Ethyl und R⁶ Methylcarbonyl bedeutet und die Substituenten X¹ und Het für jede einzelne Verbindung jeweils einer Zeile der Tabelle A entsprechen.

### Tabelle 12: Verbindungen 12.1 - 12.920

Verbindungen der allgemeinen Formel Ib, in der R¹ Chlor und R² Chlor, R⁵ n-Propyl und R⁶ Methylcarbonyl bedeutet und die Substituenten X¹ und Het für jede einzelne Verbindung jeweils einer Zeile der Tabelle A entsprechen.

### Tabelle 13: Verbindungen 13.1 - 13.920

Verbindungen der allgemeinen Formel Ib, in der R¹ Chlor und R² Chlor, R⁵ Methyl und R⁶ Ethylcarbonyl bedeutet und die Substituenten X¹ und Het für jede einzelne Verbindung jeweils einer Zeile der Tabelle A entsprechen.

### Tabelle 14: Verbindungen 14.1 - 14.920

Verbindungen der allgemeinen Formel Ib, in der R¹ Chlor und R² Chlor, R⁵ Ethyl und R⁶ Ethylcarbonyl bedeutet und die Substituenten X¹ und Het für jede einzelne Verbindung jeweils einer Zeile der Tabelle A entsprechen.

### Tabelle 15: Verbindungen 15.1 - 15.920

Verbindungen der allgemeinen Formel Ib, in der R¹ Chlor und R² Chlor, R⁵ n-Propyl und R⁶ Ethylcarbonyl bedeutet und die Substituenten X¹ und Het für jede einzelne Verbindung jeweils einer Zeile der Tabelle A entsprechen.

### Tabelle 16: Verbindungen 16.1 - 16.920

Verbindungen der allgemeinen Formel Ib, in der R¹ Chlor und R² Chlor, R⁵ Methyl und R⁶ Methylsulfonyl bedeutet und die Substituenten X¹ und Het für jede einzelne Verbindung jeweils einer Zeile der Tabelle A entsprechen.

### Tabelle 17: Verbindungen 17.1 - 17.920

Verbindungen der allgemeinen Formel Ib, in der R¹ Chlor und R² Chlor, R⁵ Ethyl und R⁶ Methylsulfonyl bedeutet und die Substituenten X¹ und Het für jede einzelne Verbindung jeweils einer Zeile der Tabelle A entsprechen.

### Tabelle 18: Verbindungen 18.1 - 18.920

Verbindungen der allgemeinen Formel Ib, in der R¹ Chlor und R² Chlor, R⁵ n-Propyl und R⁶ Methylsulfonyl bedeutet und die Substituenten X¹ und Het für jede einzelne Verbindung jeweils einer Zeile der Tabelle A entsprechen.

### Tabelle 19: Verbindungen 19.1 - 19.920

Verbindungen der allgemeinen Formel Ib, in der R¹ Chlor und R² Chlor, R⁵ Methyl und R⁶ Ethylsulfonyl bedeutet und die Substituenten X¹ und Het für jede einzelne Verbindung jeweils einer Zeile der Tabelle A entsprechen.

### Tabelle 20: Verbindungen 20.1 - 20.920

Verbindungen der allgemeinen Formel Ib, in der R¹ Chlor und R² Chlor, R⁵ Ethyl und R⁶ Ethylsulfonyl bedeutet und die Substituenten X¹ und Het für jede einzelne Verbindung jeweils einer Zeile der Tabelle A entsprechen.

### Tabelle 21: Verbindungen 21.1 - 21.920

Verbindungen der allgemeinen Formel Ib, in der R¹ Chlor und R² Chlor, R⁵ *n*-Propyl und R⁶ Ethylsulfonyl bedeutet und die Substituenten X¹ und Het für jede einzelne Verbindung jeweils einer Zeile der Tabelle A entsprechen.

### Tabelle 22: Verbindungen 22.1 - 22.920

Verbindungen der allgemeinen Formel Ib, in der R¹ Chlor und R² Chlor, R⁵ Methyl und R⁶ 4-Methylphenylsulfonyl bedeutet und die Substituenten X¹ und Het für jede einzelne Verbindung jeweils einer Zeile der Tabelle A entsprechen.

### Tabelle 23: Verbindungen 23.1 - 23.920

Verbindungen der allgemeinen Formel Ib, in der R¹ Chlor und R² Chlor, R⁵ Ethyl und R⁶ 4-Methylphenylsulfonyl bedeutet und die Substituenten X¹ und Het für jede einzelne Verbindung jeweils einer Zeile der Tabelle A entsprechen.

### Tabelle 24: Verbindungen 24.1 - 24.920

Verbindungen der allgemeinen Formel Ib, in der R¹ Chlor und R² Chlor, R⁵ n-Propyl und R⁶ 4-Methylphenylsulfonyl bedeutet und die Substituenten X¹ und Het für jede einzelne Verbindung jeweils einer Zeile der Tabelle A entsprechen.

### Tabelle 25: Verbindungen 25.1 - 25.920

Verbindungen der allgemeinen Formel Ib, in der R¹ Chlor und R² Methylsulfonyl, R⁵ Methyl und R⁶ Wasserstoff bedeutet und die Substituenten X¹ und Het für jede einzelne Verbindung jeweils einer Zeile der Tabelle A entsprechen.

### Tabelle 26: Verbindungen 26.1 - 26.920

Verbindungen der allgemeinen Formel Ib, in der R¹ Chlor und R² Methylsulfonyl, R⁵ Ethyl und R⁶ Wasserstoff bedeutet und die Substituenten X¹ und Het für jede einzelne Verbindung jeweils einer Zeile der Tabelle A entsprechen.

### Tabelle 27: Verbindungen 27.1 - 27.920

Verbindungen der allgemeinen Formel Ib, in der R¹ Chlor und R² Methylsulfonyl, R⁵ n-Propyl und R⁶ Wasserstoff bedeutet und die Substituenten X¹ und Het für jede einzelne Verbindung jeweils einer Zeile der Tabelle A entsprechen.

### Tabelle 28: Verbindungen 28.1 - 28.920

Verbindungen der allgemeinen Formel Ib, in der R¹ Chlor und R² Methylsulfonyl, R⁵ Methyl und R⁶ Methyl bedeutet und die Substituenten X¹ und Het für jede einzelne Verbindung jeweils einer Zeile der Tabelle A entsprechen.

### Tabelle 29: Verbindungen 29.1 - 29.920

Verbindungen der allgemeinen Formel Ib, in der R¹ Chlor und R² Methylsulfonyl, R⁵ Ethyl und R⁶ Methyl bedeutet und die Substituenten X¹ und Het für jede einzelne Verbindung jeweils einer Zeile der Tabelle A entsprechen.

### Tabelle 30: Verbindungen 30.1 - 30.920

Verbindungen der allgemeinen Formel Ib, in der R¹ Chlor und R² Methylsulfonyl, R⁵ n-Propyl und R⁶ Methyl bedeutet und die Substituenten X¹ und Het für jede einzelne Verbindung jeweils einer Zeile der Tabelle A entsprechen.

### Tabelle 31: Verbindungen 31.1 - 31.920

Verbindungen der allgemeinen Formel Ib, in der R¹ Chlor und R² Methylsulfonyl, R⁵ Methyl und R⁶ Ethyl bedeutet und die Substituenten X¹ und Het für jede einzelne Verbindung jeweils einer Zeile der Tabelle A entsprechen.

### Tabelle 32: Verbindungen 32.1 - 32.920

Verbindungen der allgemeinen Formel Ib, in der R¹ Chlor und R² Methylsulfonyl, R⁵ Ethyl und R⁶ Ethyl bedeutet und die Substituenten X¹ und Het für jede einzelne Verbindung jeweils einer Zeile der Tabelle A entsprechen.

### Tabelle 33: Verbindungen 33.1 - 33.920

Verbindungen der allgemeinen Formel Ib, in der R¹ Chlor und R² Methylsulfonyl, R⁵ n-Propyl und R⁶ Ethyl bedeutet und die Substituenten X¹ und Het für jede einzelne Verbindung jeweils einer Zeile der Tabelle A entsprechen.

### Tabelle 34: Verbindungen 34.1 - 34.920

Verbindungen der allgemeinen Formel Ib, in der R¹ Chlor und R² Methylsulfonyl, R⁵ Methyl und R⁶ Methylcarbonyl bedeutet und die Substituenten X¹ und Het für jede einzelne Verbindung jeweils einer Zeile der Tabelle A entsprechen.

### Tabelle 35: Verbindungen 35.1 - 35.920

Verbindungen der allgemeinen Formel Ib, in der R¹ Chlor und R² Methylsulfonyl, R⁵ Ethyl und R⁶ Methylcarbonyl bedeutet und die Substituenten X¹ und Het für jede einzelne Verbindung jeweils einer Zeile der Tabelle A entsprechen.

### Tabelle 36: Verbindungen 36.1 - 36.920

Verbindungen der allgemeinen Formel Ib, in der R¹ Chlor und R² Methylsulfonyl, R⁵ n-Propyl und R⁶ Methylcarbonyl bedeutet und die Substituenten X¹ und Het für jede einzelne Verbindung jeweils einer Zeile der Tabelle A entsprechen.

### Tabelle 37: Verbindungen 37.1 - 37.920

Verbindungen der allgemeinen Formel Ib, in der R¹ Chlor und R² Methylsulfonyl, R⁵ Methyl und R⁶ Ethylcarbonyl bedeutet und die Substituenten X¹ und Het für jede einzelne Verbindung jeweils einer Zeile der Tabelle A entsprechen.

### Tabelle 38: Verbindungen 38.1 - 38.920

Verbindungen der allgemeinen Formel Ib, in der R¹ Chlor und R² Methylsulfonyl, R⁵ Ethyl und R⁶ Ethylcarbonyl bedeutet und die Substituenten X¹ und Het für jede einzelne Verbindung jeweils einer Zeile der Tabelle A entsprechen.

### Tabelle 39: Verbindungen 39.1 - 39.920

Verbindungen der allgemeinen Formel Ib, in der R¹ Chlor und R² Methylsulfonyl, R⁵ n-Propyl und R⁶ Ethylcarbonyl bedeutet und die Substituenten X¹ und Het für jede einzelne Verbindung jeweils einer Zeile der Tabelle A entsprechen.

### Tabelle 40: Verbindungen 40.1 - 40.920

Verbindungen der allgemeinen Formel Ib, in der R¹ Chlor und R² Methylsulfonyl, R⁵ Methyl und R⁶ Methylsulfonyl bedeutet und die Substituenten X¹ und Het für jede einzelne Verbindung jeweils einer Zeile der Tabelle A entsprechen.

### Tabelle 41: Verbindungen 41.1 - 41.920

Verbindungen der allgemeinen Formel Ib, in der R¹ Chlor und R² Methylsulfonyl, R⁵ Ethyl und R⁶ Methylsulfonyl bedeutet und die Substituenten X¹ und Het für jede einzelne Verbindung jeweils einer Zeile der Tabelle A entsprechen.

### Tabelle 42: Verbindungen 42.1 - 42.920

Verbindungen der allgemeinen Formel Ib, in der R¹ Chlor und R² Methylsulfonyl, R⁵ n-Propyl und R⁶ Methylsulfonyl bedeutet und die Substituenten X¹ und Het für jede einzelne Verbindung jeweils einer Zeile der Tabelle A entsprechen.

### Tabelle 43: Verbindungen 43.1 - 43.920

Verbindungen der allgemeinen Formel Ib, in der R¹ Chlor und R² Methylsulfonyl, R⁵ Methyl und R⁶ Ethylsulfonyl bedeutet und die Substituenten X¹ und Het für jede einzelne Verbindung jeweils einer Zeile der Tabelle A entsprechen.

### Tabelle 44: Verbindungen 44.1 - 44.920

Verbindungen der allgemeinen Formel Ib, in der R¹ Chlor und R² Methylsulfonyl, R⁵ Ethyl und R⁶ Ethylsulfonyl bedeutet und die Substituenten X¹ und Het für jede einzelne Verbindung jeweils einer Zeile der Tabelle A entsprechen.

### Tabelle 45: Verbindungen 45.1 - 45.920

Verbindungen der allgemeinen Formel Ib, in der R¹ Chlor und R² Methylsulfonyl, R⁵ n-Propyl und R⁶ Ethylsulfonyl bedeutet und die Substituenten X¹ und Het für jede einzelne Verbindung jeweils einer Zeile der Tabelle A entsprechen.

### Tabelle 46: Verbindungen 46.1 - 46.920

Verbindungen der allgemeinen Formel Ib, in der R¹ Chlor und R² Methylsulfonyl, R⁵ Methyl und R⁶ 4-Methylphenylsulfonyl bedeutet und die Substituenten X¹ und Het für jede einzelne Verbindung jeweils einer Zeile der Tabelle A entsprechen.

### Tabelle 47: Verbindungen 47.1 - 47.920

Verbindungen der allgemeinen Formel Ib, in der R¹ Chlor und R² Methylsulfonyl, R⁵ Ethyl und R⁶ 4-Methylphenylsulfonyl bedeutet und die Substituenten X¹ und Het für jede einzelne Verbindung jeweils einer Zeile der Tabelle A entsprechen.

### Tabelle 48: Verbindungen 48.1 - 48.920

Verbindungen der allgemeinen Formel Ib, in der R¹ Chlor und R² Methylsulfonyl, R⁵ n-Propyl und R⁶ 4-Methylphenylsulfonyl bedeutet und die Substituenten X¹ und Het für jede einzelne Verbindung jeweils einer Zeile der Tabelle A entsprechen.

### Tabelle 49: Verbindungen 49.1 - 49.920

Verbindungen der allgemeinen Formel Ib, in der R¹ Chlor und R² Trifluormethyl, R⁵ Methyl und R⁶ Wasserstoff bedeutet und die Substituenten X¹ und Het für jede einzelne Verbindung jeweils einer Zeile der Tabelle A entsprechen.

### Tabelle 50: Verbindungen 50.1 - 50.920

Verbindungen der allgemeinen Formel Ib, in der R¹ Chlor und R² Trifluormethyl, R⁵ Ethyl und R⁶ Wasserstoff bedeutet und die Substituenten X¹ und Het für jede einzelne Verbindung jeweils einer Zeile der Tabelle A entsprechen.

### Tabelle 51: Verbindungen 51.1 - 51.920

Verbindungen der allgemeinen Formel Ib, in der R¹ Chlor und R² Trifluormethyl, R⁵ n-Propyl und R⁶ Wasserstoff bedeutet und die Substituenten X¹ und Het für jede einzelne Verbindung jeweils einer Zeile der Tabelle A entsprechen.

### Tabelle 52: Verbindungen 52.1 - 52.920

Verbindungen der allgemeinen Formel Ib, in der R¹ Chlor und R² Trifluormethyl, R⁵ Methyl und R⁶ Methyl bedeutet und die Substituenten X¹ und Het für jede einzelne Verbindung jeweils einer Zeile der Tabelle A entsprechen.

### Tabelle 53: Verbindungen 53.1 - 53.920

Verbindungen der allgemeinen Formel Ib, in der R¹ Chlor und R² Trifluormethyl, R⁵ Ethyl und R⁶ Methyl bedeutet und die Substituenten X¹ und Het für jede einzelne Verbindung jeweils einer Zeile der Tabelle A entsprechen.

### Tabelle 54: Verbindungen 54.1 - 54.920

Verbindungen der allgemeinen Formel Ib, in der R¹ Chlor und R² Trifluormethyl, R⁵ n-Propyl und R⁶ Methyl bedeutet und die Substituenten X¹ und Het für jede einzelne Verbindung jeweils einer Zeile der Tabelle A entsprechen.

### Tabelle 55: Verbindungen 55.1 - 55.920

Verbindungen der allgemeinen Formel Ib, in der R¹ Chlor und R² Trifluormethyl, R⁵ Methyl und R⁶ Ethyl bedeutet und die Substituenten X¹ und Het für jede einzelne Verbindung jeweils einer Zeile der Tabelle A entsprechen.

### Tabelle 56: Verbindungen 56.1 - 56.920

Verbindungen der allgemeinen Formel Ib, in der R¹ Chlor und R² Trifluormethyl, R⁵ Ethyl und R⁶ Ethyl bedeutet und die Substituenten X¹ und Het für jede einzelne Verbindung jeweils einer Zeile der Tabelle A entsprechen.

### Tabelle 57: Verbindungen 57.1 - 57.920

Verbindungen der allgemeinen Formel Ib, in der R¹ Chlor und R² Trifluormethyl, R⁵ n-Propyl und R⁶ Ethyl bedeutet und die Substituenten X¹ und Het für jede einzelne Verbindung jeweils einer Zeile der Tabelle A entsprechen.

### Tabelle 58: Verbindungen 58.1 - 58.920

Verbindungen der allgemeinen Formel Ib, in der R¹ Chlor und R² Trifluormethyl, R⁵ Methyl und R⁶ Methylcarbonyl bedeutet und die Substituenten X¹ und Het für jede einzelne Verbindung jeweils einer Zeile der Tabelle A entsprechen.

### Tabelle 59: Verbindungen 59.1 - 59.920

Verbindungen der allgemeinen Formel Ib, in der R¹ Chlor und R² Trifluormethyl, R⁵ Ethyl und R⁶ Methylcarbonyl bedeutet und die Substituenten X¹ und Het für jede einzelne Verbindung jeweils einer Zeile der Tabelle A entsprechen.

### Tabelle 60: Verbindungen 60.1 - 60.920

Verbindungen der allgemeinen Formel Ib, in der R¹ Chlor und R² Chlor, R⁵ n-Propyl und R⁶ Methylcarbonyl bedeutet und die Substituenten X¹ und Het für jede einzelne Verbindung jeweils einer Zeile der Tabelle A entsprechen.

### Tabelle 61: Verbindungen 61.1 - 61.920

Verbindungen der allgemeinen Formel Ib, in der R¹ Chlor und R² Trifluormethyl, R⁵ Methyl und R⁶ Ethylcarbonyl bedeutet und die Substituenten X¹ und Het für jede einzelne Verbindung jeweils einer Zeile der Tabelle A entsprechen.

### Tabelle 62: Verbindungen 62.1 - 62.920

Verbindungen der allgemeinen Formel Ib, in der R¹ Chlor und R² Trifluormethyl, R⁵ Ethyl und R⁶ Ethylcarbonyl bedeutet und die Substituenten X¹ und Het für jede einzelne Verbindung jeweils einer Zeile der Tabelle A entsprechen.

### Tabelle 63: Verbindungen 63.1 - 63.920

Verbindungen der allgemeinen Formel Ib, in der R¹ Chlor und R² Trifluormethyl, R⁵ n-Propyl und R⁶ Ethylcarbonyl bedeutet und die Substituenten X¹ und Het für jede einzelne Verbindung jeweils einer Zeile der Tabelle A entsprechen.

### Tabelle 64: Verbindungen 64.1 - 64.920

Verbindungen der allgemeinen Formel Ib, in der R¹ Chlor und R² Trifluormethyl, R⁵ Methyl und R⁶ Methylsulfonyl bedeutet und die Substituenten X¹ und Het für jede einzelne Verbindung jeweils einer Zeile der Tabelle A entsprechen.

### Tabelle 65: Verbindungen 65.1 - 65.920

Verbindungen der allgemeinen Formel Ib, in der R¹ Chlor und R² Trifluormethyl, R⁵ Ethyl und R⁶ Methylsulfonyl bedeutet und die Substituenten X¹ und Het für jede einzelne Verbindung jeweils einer Zeile der Tabelle A entsprechen.

### Tabelle 66: Verbindungen 66.1 - 66.920

Verbindungen der allgemeinen Formel Ib, in der R¹ Chlor und R² Trifluormethyl, R⁵ n-Propyl und R⁶ Methylsulfonyl bedeutet und die Substituenten X¹ und Het für jede einzelne Verbindung jeweils einer Zeile der Tabelle A entsprechen.

### Tabelle 67: Verbindungen 67.1 - 67.920

Verbindungen der allgemeinen Formel Ib, in der R¹ Chlor und R² Trifluormethyl, R⁵ Methyl und R⁶ Ethylsulfonyl bedeutet und die Substituenten X¹ und Het für jede einzelne Verbindung jeweils einer Zeile der Tabelle A entsprechen.

### Tabelle 68: Verbindungen 68.1 - 68.920

Verbindungen der allgemeinen Formel Ib, in der R¹ Chlor und R² Trifluormethyl, R⁵ Ethyl und R⁶ Ethylsulfonyl bedeutet und die Substituenten X¹ und Het für jede einzelne Verbindung jeweils einer Zeile der Tabelle A entsprechen.

### Tabelle 69: Verbindungen 69.1 - 69.920

Verbindungen der allgemeinen Formel Ib, in der R¹ Chlor und R² Trifluormethyl, R⁵ n-Propyl und R⁶ Ethylsulfonyl bedeutet und die Substituenten X¹ und Het für jede einzelne Verbindung jeweils einer Zeile der Tabelle A entsprechen.

### Tabelle 70: Verbindungen 70.1 - 70.920

Verbindungen der allgemeinen Formel Ib, in der R¹ Chlor und R² Trifluormethyl, R⁵ Methyl und R⁶ 4-Methylphenylsulfonyl bedeutet und die Substituenten X¹ und Het für jede einzelne Verbindung jeweils einer Zeile der Tabelle A entsprechen.

### Tabelle 71: Verbindungen 71.1 - 71.920

Verbindungen der allgemeinen Formel Ib, in der R¹ Chlor und R² Trifluormethyl, R⁵ Ethyl und R⁶ 4-Methylphenylsulfonyl bedeutet und die Substituenten X¹ und Het für jede einzelne Verbindung jeweils einer Zeile der Tabelle A entsprechen.

### Tabelle 72: Verbindungen 72.1 - 72.920

Verbindungen der allgemeinen Formel Ib, in der R¹ Chlor und R² Trifluormethyl, R⁵ n-Propyl und R⁶ 4-Methylphenylsulfonyl bedeutet und die Substituenten X¹ und Het für jede einzelne Verbindung jeweils einer Zeile der Tabelle A entsprechen.

### Tabelle 73: Verbindungen 73.1 - 73.920

Verbindungen der allgemeinen Formel Ib, in der R¹ Methyl und R² Chlor, R⁵ Methyl und R⁶ Wasserstoff bedeutet und die Substituenten X¹ und Het für jede einzelne Verbindung jeweils einer Zeile der Tabelle A entsprechen.

### Tabelle 74: Verbindungen 74.1 - 74.920

Verbindungen der allgemeinen Formel Ib, in der R¹ Methyl und R² Chlor, R⁵ Ethyl und R⁶ Wasserstoff bedeutet und die Substituenten X¹ und Het für jede einzelne Verbindung jeweils einer Zeile der Tabelle A entsprechen.

### Tabelle 75: Verbindungen 75.1 - 75.920

Verbindungen der allgemeinen Formel Ib, in der R¹ Methyl und R² Chlor, R⁵ n-Propyl und R⁶ Wasserstoff bedeutet und die Substituenten X¹ und Het für jede einzelne Verbindung jeweils einer Zeile der Tabelle A entsprechen.

### Tabelle 76: Verbindungen 76.1 - 76.920

Verbindungen der allgemeinen Formel Ib, in der R¹ Methyl und R² Chlor, R⁵ Methyl und R⁶ Methyl bedeutet und die Substituenten X¹ und Het für jede einzelne Verbindung jeweils einer Zeile der Tabelle A entsprechen.

### Tabelle 77: Verbindungen 77.1 - 77.920

Verbindungen der allgemeinen Formel Ib, in der R¹ Methyl und R² Chlor, R⁵ Ethyl und R⁶ Methyl bedeutet und die Substituenten X¹ und Het für jede einzelne Verbindung jeweils einer Zeile der Tabelle A entsprechen.

### Tabelle 78: Verbindungen 78.1 - 78.920

Verbindungen der allgemeinen Formel Ib, in der R¹ Methyl und R² Chlor, R⁵ n-Propyl und R⁶ Methyl bedeutet und die Substituenten X¹ und Het für jede einzelne Verbindung jeweils einer Zeile der Tabelle A entsprechen.

### Tabelle 79: Verbindungen 79.1 - 79.920

Verbindungen der allgemeinen Formel Ib, in der R¹ Methyl und R² Chlor, R⁵ Methyl und R⁶ Ethyl bedeutet und die Substituenten X¹ und Het für jede einzelne Verbindung jeweils einer Zeile der Tabelle A entsprechen.

### Tabelle 80: Verbindungen 80.1 - 80.920

Verbindungen der allgemeinen Formel Ib, in der R¹ Methyl und R² Chlor, R⁵ Ethyl und R⁶ Ethyl bedeutet und die Substituenten X¹ und Het für jede einzelne Verbindung jeweils einer Zeile der Tabelle A entsprechen.

### Tabelle 81: Verbindungen 81.1 - 81.920

Verbindungen der allgemeinen Formel Ib, in der R¹ Methyl und R² Chlor, R⁵ n-Propyl und R⁶ Ethyl bedeutet und die Substituenten X¹ und Het für jede einzelne Verbindung jeweils einer Zeile der Tabelle A entsprechen.

### Tabelle 82: Verbindungen 82.1 - 82.920

Verbindungen der allgemeinen Formel Ib, in der R¹ Methyl und R² Chlor, R⁵ Methyl und R⁶ Methylcarbonyl bedeutet und die Substituenten X¹ und Het für jede einzelne Verbindung jeweils einer Zeile der Tabelle A entsprechen.

### Tabelle 83: Verbindungen 83.1 - 83.920

Verbindungen der allgemeinen Formel Ib, in der R¹ Methyl und R² Chlor, R⁵ Ethyl und R⁶ Methylcarbonyl bedeutet und die Substituenten X¹ und Het für jede einzelne Verbindung jeweils einer Zeile der Tabelle A entsprechen.

### Tabelle 84: Verbindungen 84.1 - 84.920

Verbindungen der allgemeinen Formel Ib, in der R¹ Methyl und R² Chlor, R⁵ n-Propyl und R⁶ Methylcarbonyl bedeutet und die Substituenten X¹ und Het für jede einzelne Verbindung jeweils einer Zeile der Tabelle A entsprechen.

### Tabelle 85: Verbindungen 85.1 - 85.920

Verbindungen der allgemeinen Formel Ib, in der R¹ Methyl und R² Chlor, R⁵ Methyl und R⁶ Ethylcarbonyl bedeutet und die Substituenten X¹ und Het für jede einzelne Verbindung jeweils einer Zeile der Tabelle A entsprechen.

### Tabelle 86: Verbindungen 86.1 - 86.920

Verbindungen der allgemeinen Formel Ib, in der R¹ Methyl und R² Chlor, R⁵ Ethyl und R⁶ Ethylcarbonyl bedeutet und die Substituenten X¹ und Het für jede einzelne Verbindung jeweils einer Zeile der Tabelle A entsprechen.

### Tabelle 87: Verbindungen 87.1 - 87.920

Verbindungen der allgemeinen Formel Ib, in der R¹ Methyl und R² Chlor, R⁵ n-Propyl und R⁶ Ethylcarbonyl bedeutet und die Substituenten X¹ und Het für jede einzelne Verbindung jeweils einer Zeile der Tabelle A entsprechen.

### Tabelle 88: Verbindungen 88.1 - 88.920

Verbindungen der allgemeinen Formel Ib, in der R¹ Methyl und R² Chlor, R⁵ Methyl und R⁶ Methylsulfonyl bedeutet und die Substituenten X¹ und Het für jede einzelne Verbindung jeweils einer Zeile der Tabelle A entsprechen.

### Tabelle 89: Verbindungen 89.1 - 89.920

Verbindungen der allgemeinen Formel Ib, in der R¹ Methyl und R² Chlor, R⁵ Ethyl und R⁶ Methylsulfonyl bedeutet und die Substituenten X¹ und Het für jede einzelne Verbindung jeweils einer Zeile der Tabelle A entsprechen.

### Tabelle 90: Verbindungen 90.1 - 90.920

Verbindungen der allgemeinen Formel Ib, in der R¹ Methyl und R² Chlor, R⁵ *n*-Propyl und R⁶ Methylsulfonyl bedeutet und die Substituenten X¹ und Het für jede einzelne Verbindung jeweils einer Zeile der Tabelle A entsprechen.

### Tabelle 91: Verbindungen 91.1 - 91.920

Verbindungen der allgemeinen Formel Ib, in der R¹ Methyl und R² Chlor, R⁵ Methyl und R⁶ Ethylsulfonyl bedeutet und die Substituenten X¹ und Het für jede einzelne Verbindung jeweils einer Zeile der Tabelle A entsprechen.

### Tabelle 92: Verbindungen 92.1 - 92.920

Verbindungen der allgemeinen Formel Ib, in der R¹ Methyl und R² Chlor, R⁵ Ethyl und R⁶ Ethylsulfonyl bedeutet und die Substituenten X¹ und Het für jede einzelne Verbindung jeweils einer Zeile der Tabelle A entsprechen.

### Tabelle 93: Verbindungen 93.1 - 93.920

Verbindungen der allgemeinen Formel Ib, in der R¹ Methyl und R² Chlor, R⁵ n-Propyl und R⁶ Ethylsulfonyl bedeutet und die Substituenten X¹ und Het für jede einzelne Verbindung jeweils einer Zeile der Tabelle A entsprechen.

### Tabelle 94: Verbindungen 94.1 - 94.920

Verbindungen der allgemeinen Formel Ib, in der R¹ Methyl und R² Chlor, R⁵ Methyl und R⁶ 4-Methylphenylsulfonyl bedeutet und die Substituenten X¹ und Het für jede einzelne Verbindung jeweils einer Zeile der Tabelle A entsprechen.

### Tabelle 95: Verbindungen 95.1 - 95.920

Verbindungen der allgemeinen Formel Ib, in der R¹ Methyl und R² Chlor, R⁵ Ethyl und R⁶ 4-Methylphenylsulfonyl bedeutet und die Substituenten X¹ und Het für jede einzelne Verbindung jeweils einer Zeile der Tabelle A entsprechen.

### Tabelle 96: Verbindungen 96.1 - 96.920

Verbindungen der allgemeinen Formel Ib, in der R¹ Methyl und R² Chlor, R⁵ n-Propyl und R⁶ 4-Methylphenylsulfonyl bedeutet und die Substituenten X¹ und Het für jede einzelne Verbindung jeweils einer Zeile der Tabelle A entsprechen.

### Tabelle 97: Verbindungen 97.1 - 97.920

Verbindungen der allgemeinen Formel Ib, in der R¹ Methyl und R² Methylsulfonyl, R⁵ Methyl und R⁶ Wasserstoff bedeutet und die Substituenten X¹ und Het für jede einzelne Verbindung jeweils einer Zeile der Tabelle A entsprechen.

### Tabelle 98: Verbindungen 98.1 - 98.920

Verbindungen der allgemeinen Formel Ib, in der R¹ Methyl und R² Methylsulfonyl, R⁵ Ethyl und R⁶ Wasserstoff bedeutet und die Substituenten X¹ und Het für jede einzelne Verbindung jeweils einer Zeile der Tabelle A entsprechen.

### Tabelle 99: Verbindungen 99.1 - 99.920

Verbindungen der allgemeinen Formel Ib, in der R¹ Methyl und R² Methylsulfonyl, R⁵ n-Propyl und R⁶ Wasserstoff bedeutet und die Substituenten X¹ und Het für jede einzelne Verbindung jeweils einer Zeile der Tabelle A entsprechen.

### Tabelle 100: Verbindungen 100.1 - 100.920

Verbindungen der allgemeinen Formel Ib, in der R¹ Methyl und R² Methylsulfonyl, R⁵ Methyl und R⁶ Methyl bedeutet und die Substituenten X¹ und Het für jede einzelne Verbindung jeweils einer Zeile der Tabelle A entsprechen.

### Tabelle 101: Verbindungen 101.1 - 101.920

Verbindungen der allgemeinen Formel Ib, in der R¹ Methyl und R² Methylsulfonyl, R⁵ Ethyl und R⁶ Methyl bedeutet und die Substituenten X¹ und Het für jede einzelne Verbindung jeweils einer Zeile der Tabelle A entsprechen.

### Tabelle 102: Verbindungen 102.1 - 102.920

Verbindungen der allgemeinen Formel Ib, in der R¹ Methyl und R² Methylsulfonyl, R⁵ n-Propyl und R⁶ Methyl bedeutet und die Substituenten X¹ und Het für jede einzelne Verbindung jeweils einer Zeile der Tabelle A entsprechen.

### Tabelle 103: Verbindungen 103.1 - 103.920

Verbindungen der allgemeinen Formel Ib, in der R¹ Methyl und R² Methylsulfonyl, R⁵ Methyl und R⁶ Ethyl bedeutet und die Substituenten X¹ und Het für jede einzelne Verbindung jeweils einer Zeile der Tabelle A entsprechen.

### Tabelle 104: Verbindungen 104.1 - 104.920

Verbindungen der allgemeinen Formel Ib, in der R¹ Methyl und R² Methylsulfonyl, R⁵ Ethyl und R⁶ Ethyl bedeutet und die Substituenten X¹ und Het für jede einzelne Verbindung jeweils einer Zeile der Tabelle A entsprechen.

### Tabelle 105: Verbindungen 105.1 - 105.920

Verbindungen der allgemeinen Formel Ib, in der R¹ Methyl und R² Methylsulfonyl, R⁵ n-Propyl und R⁶ Ethyl bedeutet und die Substituenten X¹ und Het für jede einzelne Verbindung jeweils einer Zeile der Tabelle A entsprechen.

### Tabelle 106: Verbindungen 106.1 - 106. 920

Verbindungen der allgemeinen Formel Ib, in der R¹ Methyl und R² Methylsulfonyl, R⁵ Methyl und R⁶ Methylcarbonyl bedeutet und die Substituenten X¹ und Het für jede einzelne Verbindung jeweils einer Zeile der Tabelle A entsprechen.

### Tabelle 107: Verbindungen 107.1 - 107.920

Verbindungen der allgemeinen Formel Ib, in der R¹ Methyl und R² Methylsulfonyl, R⁵ Ethyl und R⁶ Methylcarbonyl bedeutet und die Substituenten X¹ und Het für jede einzelne Verbindung jeweils einer Zeile der Tabelle A entsprechen.

### Tabelle 108: Verbindungen 108.1 - 108.920

Verbindungen der allgemeinen Formel Ib, in der R¹ Methyl und R² Methylsulfonyl, R⁵ n-Propyl und R⁶ Methylcarbonyl bedeutet und die Substituenten X¹ und Het für jede einzelne Verbindung jeweils einer Zeile der Tabelle A entsprechen.

### Tabelle 109: Verbindungen 109.1 - 109.920

Verbindungen der allgemeinen Formel Ib, in der R¹ Methyl und R² Methylsulfonyl, R⁵ Methyl und R⁶ Ethylcarbonyl bedeutet und die Substituenten X¹ und Het für jede einzelne Verbindung jeweils einer Zeile der Tabelle A entsprechen.

### Tabelle 110: Verbindungen 110.1 - 110.920

Verbindungen der allgemeinen Formel Ib, in der R¹ Methyl und R² Methylsulfonyl, R⁵ Ethyl und R⁶ Ethylcarbonyl bedeutet und die Substituenten X¹ und Het für jede einzelne Verbindung jeweils einer Zeile der Tabelle A entsprechen.

### Tabelle 111: Verbindungen 111.1 - 111.920

Verbindungen der allgemeinen Formel Ib, in der R¹ Methyl und R² Methylsulfonyl, R⁵ n-Propyl und R⁶ Ethylcarbonyl bedeutet und die Substituenten X¹ und Het für jede einzelne Verbindung jeweils einer Zeile der Tabelle A entsprechen.

### Tabelle 112: Verbindungen 112.1 - 112.920

Verbindungen der allgemeinen Formel Ib, in der R¹ Methyl und R² Methylsulfonyl, R⁵ Methyl und R⁶ Methylsulfonyl bedeutet und die Substituenten X¹ und Het für jede einzelne Verbindung jeweils einer Zeile der Tabelle A entsprechen.

### Tabelle 113: Verbindungen 113.1 - 113.920

Verbindungen der allgemeinen Formel Ib, in der R¹ Methyl und R² Methylsulfonyl, R⁵ Ethyl und R⁶ Methylsulfonyl bedeutet und die Substituenten X¹ und Het für jede einzelne Verbindung jeweils einer Zeile der Tabelle A entsprechen.

### Tabelle 114: Verbindungen 114.1 - 114.920

Verbindungen der allgemeinen Formel Ib, in der R¹ Methyl und R² Methylsulfonyl, R⁵ *n*-Propyl und R⁶ Methylsulfonyl bedeutet und die Substituenten X¹ und Het für jede einzelne Verbindung jeweils einer Zeile der Tabelle A entsprechen.

### Tabelle 115: Verbindungen 115.1 - 115.920

Verbindungen der allgemeinen Formel Ib, in der R¹ Methyl und R² Methylsulfonyl, R⁵ Methyl und R⁶ Ethylsulfonyl bedeutet und die Substituenten X¹ und Het für jede einzelne Verbindung jeweils einer Zeile der Tabelle A entsprechen.

### Tabelle 116: Verbindungen 116.1 - 116.920

Verbindungen der allgemeinen Formel Ib, in der R¹ Methyl und R² Methylsulfonyl, R⁵ Ethyl und R⁶ Ethylsulfonyl bedeutet und die Substituenten X¹ und Het für jede einzelne Verbindung jeweils einer Zeile der Tabelle A entsprechen.

### Tabelle 117: Verbindungen 117.1 - 117.920

Verbindungen der allgemeinen Formel Ib, in der R¹ Methyl und R² Methylsulfonyl, R⁵ *n*-Propyl und R⁶ Ethylsulfonyl bedeutet und die Substituenten X¹ und Het für jede einzelne Verbindung jeweils einer Zeile der Tabelle A entsprechen.

### Tabelle 118: Verbindungen 118.1 - 118.920

Verbindungen der allgemeinen Formel Ib, in der R¹ Methyl und R² Methylsulfonyl, R⁵ Methyl und R⁶ 4-Methylphenylsulfonyl bedeutet und die Substituenten X¹ und Het für jede einzelne Verbindung jeweils einer Zeile der Tabelle A entsprechen.

### Tabelle 119: Verbindungen 119.1 - 119.920

Verbindungen der allgemeinen Formel Ib, in der R¹ Methyl und R² Methylsulfonyl, R⁵ Ethyl und R⁶ 4-Methylphenylsulfonyl bedeutet und die Substituenten X¹ und Het für jede einzelne Verbindung jeweils einer Zeile der Tabelle A entsprechen.

### Tabelle 120: Verbindungen 120.1 - 120.920

Verbindungen der allgemeinen Formel Ib, in der R¹ Methyl und R² Methylsulfonyl, R⁵ n-Propyl und R⁶ 4-Methylphenylsulfonyl bedeutet und die Substituenten X¹ und Het für jede einzelne Verbindung jeweils einer Zeile der Tabelle A entsprechen.

### Tabelle 121: Verbindungen 121.1 - 121.920

Verbindungen der allgemeinen Formel Ib, in der R¹ Methyl und R² Trifluormethyl, R⁵ Methyl und R⁶ Wasserstoff bedeutet und die Substituenten X¹ und Het für jede einzelne Verbindung jeweils einer Zeile der Tabelle A entsprechen.

### Tabelle 122: Verbindungen 122.1 - 122.920

Verbindungen der allgemeinen Formel Ib, in der R¹ Methyl und R² Trifluormethyl, R⁵ Ethyl und R⁶ Wasserstoff bedeutet und die Substituenten X¹ und Het für jede einzelne Verbindung jeweils einer Zeile der Tabelle A entsprechen.

### Tabelle 123: Verbindungen 123.1 - 123.920

Verbindungen der allgemeinen Formel Ib, in der R¹ Methyl und R² Trifluormethyl, R⁵ n-Propyl und R⁶ Wasserstoff bedeutet und die Substituenten X¹ und Het für jede einzelne Verbindung jeweils einer Zeile der Tabelle A entsprechen.

### Tabelle 124: Verbindungen 124.1 - 124.920

Verbindungen der allgemeinen Formel Ib, in der R¹ Methyl und R² Trifluormethyl, R⁵ Methyl und R⁶ Methyl bedeutet und die Substituenten X¹ und Het für jede einzelne Verbindung jeweils einer Zeile der Tabelle A entsprechen.

### Tabelle 125: Verbindungen 12.1 - 125.920

Verbindungen der allgemeinen Formel Ib, in der R¹ Methyl und R² Trifluormethyl, R⁵ Ethyl und R⁶ Methyl bedeutet und die Substituenten X¹ und Het für jede einzelne Verbindung jeweils einer Zeile der Tabelle A entsprechen.

### Tabelle 126: Verbindungen 126.1 - 126.920

Verbindungen der allgemeinen Formel Ib, in der R¹ Methyl und R² Trifluormethyl, R⁵ n-Propyl und R⁶ Methyl bedeutet und die Substituenten X¹ und Het für jede einzelne Verbindung jeweils einer Zeile der Tabelle A entsprechen.

### Tabelle 127: Verbindungen 127.1 - 127.920

Verbindungen der allgemeinen Formel Ib, in der R¹ Methyl und R² Trifluormethyl, R⁵ Methyl und R⁶ Ethyl bedeutet und die Substituenten X¹ und Het für jede einzelne Verbindung jeweils einer Zeile der Tabelle A entsprechen.

### Tabelle 128: Verbindungen 128.1 - 128.920

Verbindungen der allgemeinen Formel Ib, in der R¹ Methyl und R² Trifluormethyl, R⁵ Ethyl und R⁶ Ethyl bedeutet und die Substituenten X¹ und Het für jede einzelne Verbindung jeweils einer Zeile der Tabelle A entsprechen.

### Tabelle 129: Verbindungen 129.1 - 129.920

Verbindungen der allgemeinen Formel Ib, in der R¹ Methyl und R² Trifluormethyl, R⁵ *n*-Propyl und R⁶ Ethyl bedeutet und die Substituenten X¹ und Het für jede einzelne Verbindung jeweils einer Zeile der Tabelle A entsprechen.

### Tabelle 130: Verbindungen 130.1 - 130.920

Verbindungen der allgemeinen Formel Ib, in der R¹ Methyl und R² Trifluormethyl, R⁵ Methyl und R⁶ Methylcarbonyl bedeutet und die Substituenten X¹ und Het für jede einzelne Verbindung jeweils einer Zeile der Tabelle A entsprechen.

### Tabelle 131: Verbindungen 131.1 - 131.920

Verbindungen der allgemeinen Formel Ib, in der R¹ Methyl und R² Trifluormethyl, R⁵ Ethyl und R⁶ Methylcarbonyl bedeutet und die Substituenten X¹ und Het für jede einzelne Verbindung jeweils einer Zeile der Tabelle A entsprechen.

### Tabelle 132: Verbindungen 132.1 - 132. 920

Verbindungen der allgemeinen Formel Ib, in der R¹ Methyl und R² Trifluormethyl, R⁵ n-Propyl und R⁶ Methylcarbonyl bedeutet und die Substituenten X¹ und Het für jede einzelne Verbindung jeweils einer Zeile der Tabelle A entsprechen.

### Tabelle 133: Verbindungen 133.1 - 133.920

Verbindungen der allgemeinen Formel Ib, in der R¹ Methyl und R² Trifluormethyl, R⁵ Methyl und R⁶ Ethylcarbonyl bedeutet und die Substituenten X¹ und Het für jede einzelne Verbindung jeweils einer Zeile der Tabelle A entsprechen.

### Tabelle 134: Verbindungen 134.1 - 134.920

Verbindungen der allgemeinen Formel Ib, in der R¹ Methyl und R² Trifluormethyl, R⁵ Ethyl und R⁶ Ethylcarbonyl bedeutet und die Substituenten X¹ und Het für jede einzelne Verbindung jeweils einer Zeile der Tabelle A entsprechen.

### Tabelle 135: Verbindungen 135.1 - 135.920

Verbindungen der allgemeinen Formel Ib, in der R¹ Methyl und R² Trifluormethyl, R⁵ n-Propyl und R⁶ Ethylcarbonyl bedeutet und die Substituenten X¹ und Het für jede einzelne Verbindung jeweils einer Zeile der Tabelle A entsprechen.

### Tabelle 136: Verbindungen 136.1 - 136.920

Verbindungen der allgemeinen Formel Ib, in der R¹ Methyl und R² Trifluormethyl, R⁵ Methyl und R⁶ Methylsulfonyl bedeutet und die Substituenten X¹ und Het für jede einzelne Verbindung jeweils einer Zeile der Tabelle A entsprechen.

### Tabelle 137: Verbindungen 137.1 - 137.920

Verbindungen der allgemeinen Formel Ib, in der R¹ Methyl und R² Trifluormethyl, R⁵ Ethyl und R⁶ Methylsulfonyl bedeutet und die Substituenten X¹ und Het für jede einzelne Verbindung jeweils einer Zeile der Tabelle A entsprechen.

### Tabelle 138: Verbindungen 138.1 - 138.920

Verbindungen der allgemeinen Formel Ib, in der R¹ Methyl und R² Trifluormethyl, R⁵ n-Propyl und R⁶ Methylsulfonyl bedeutet und die Substituenten X¹ und Het für jede einzelne Verbindung jeweils einer Zeile der Tabelle A entsprechen.

### Tabelle 139: Verbindungen 139.1 - 139.920

Verbindungen der allgemeinen Formel Ib, in der R¹ Methyl und R² Trifluormethyl, R⁵ Methyl und R⁶ Ethylsulfonyl bedeutet und die Substituenten X¹ und Het für jede einzelne Verbindung jeweils einer Zeile der Tabelle A entsprechen.

### Tabelle 140: Verbindungen 140.1 - 140.920

Verbindungen der allgemeinen Formel Ib, in der R¹ Methyl und R² Trifluormethyl, R⁵ Ethyl und R⁶ Ethylsulfonyl bedeutet und die Substituenten X¹ und Het für jede einzelne Verbindung jeweils einer Zeile der Tabelle A entsprechen.

### Tabelle 141: Verbindungen 141.1 - 141.920

Verbindungen der allgemeinen Formel Ib, in der R¹ Methyl und R² Trifluormethyl, R⁵ *n*-Propyl und R⁶ Ethylsulfonyl bedeutet und die Substituenten X¹ und Het für jede einzelne Verbindung jeweils einer Zeile der Tabelle A entsprechen.

### Tabelle 142: Verbindungen 142.1 - 142.920

Verbindungen der allgemeinen Formel Ib, in der R¹ Methyl und R² Trifluormethyl, R⁵ Methyl und R⁶ 4-Methylphenylsulfonyl bedeutet und die Substituenten X¹ und Het für jede einzelne Verbindung jeweils einer Zeile der Tabelle A entsprechen.

### Tabelle 143: Verbindungen 143.1 - 143.920

Verbindungen der allgemeinen Formel Ib, in der R¹ Methyl und R² Trifluormethyl, R⁵ Ethyl und R⁶ 4-Methylphenylsulfonyl bedeutet und die Substituenten X¹ und Het für jede einzelne Verbindung jeweils einer Zeile der Tabelle A entsprechen.

### Tabelle 144: Verbindungen 1444.1 - 144.920

Verbindungen der allgemeinen Formel Ib, in der R¹ Methyl und R² Trifluormethyl, R⁵ *n*-Propyl und R⁶ 4-Methylphenylsulfonyl bedeutet und die Substituenten X¹ und Het für jede einzelne Verbindung jeweils einer Zeile der Tabelle A entsprechen.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden. Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die substituierten 4-Benzoyl-pyrazole als solche oder in einem Ö1 oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Ö1 bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensati onsprodukte von sulfoniertem Naphthalin und seiner Derivate mit Formaldehyd, Kondensationsprodukte des Naphthalins bzw, der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Sektrum) eingesetzt.

Die erfindungsgemäßen Verbindungen I können beispielsweise wie folgt formuliert werden:
- I: 20 Gewichtsteile der Verbindung I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
- II: 20 Gewichtsteile der Verbindung I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des wirkstoffs enthält.
- III: 20 Gewichtsteile des Wirkstoffs I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
- IV: 20 Gewichtsteile des Wirkstoffs I werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20 000 Gewichtsteilen Wasser enthält man eine Spritzbrühe, die 0,1 Gew. % des Wirkstoffs enthält.
- V: 3 Gewichtsteile des Wirkstoffs I werden mit 97 Gewichtsteilen feinteiligem Kaolin vermischt. Man erhält auf diese Weise ein Stäubemittel, das 3 Gew. % des Wirkstoffs enthält.
- VI: 20 Gewichtsteile des Wirkstoffs I werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
- VII: 1 Gewichtsteil der Verbindung I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
- VIII: 1 Gewichtsteil der Verbindung I wird in einer Mischung gelöst, die aus 80 Gewichtsteilen Cyclohexanon und 20 Gewichtsteilen Wettol ® EM 31 (nicht ionischer Emulgator auf der Basis von ethoxyliertem Ricinusöl). Man erhält ein stabiles Emulsionskonzentrat.

Zur Verbreiterung des Wirkungsspektrums und zur Erzielung synergistischer Effekte können die substituierten 4-Benzoyl-pyrazole mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF3-phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.)

Nachfolgend werden die Synthesen einiger Edukte und Produkte beschrieben. 2-Chlor-3-(N-3,5-dimethylpyrazolyloxymethyl)-phenyl)-4-sulfonylmethyl-(N-methyl-2-hydroxypyrazol)-methanon.

### Stufe a: 2-Chlor-3-brommethyl-4-sulfonylmethyl-benzoesäuremethylester

80 g (0,3 mol) 2-Chlor-3-methyl-4-sulfonylmethyl-benzoesäuremethylester werden in 1 1 Tetrachlormethan mit 54 g (0,31 mol) N-Bromsuccinimid und 1,5 g Azoisobutyronitril 6 h auf 76°C erwärmt. Das Reaktionsgemisch wird filtriert und i. Vak. vom Lösungsmittel befreit. Ausbeute: 104 g; Fp. 83 - 85°C.

### Stufe b: 2-Chlor-3-(N-3,5-dimethylpyrazolyloxymethyl)-4-sulfonylmethyl-benzoesäuremethylester

3,3 g 3,5-Dimethylpyrazol und 4,2 g Kaliumcarbonat werden in 30 ml N,N-Dimethylformamid 1 h auf 70°C erwärmt und mit einer Lösung aus 10 g (29,3 mmol) 2-chlor-3-brommethyl-4-sulfonylmethyl-benzoesäuremethylester in 100 ml N,N-Dimethylformamid versetzt und 4 h auf 40°C erwärmt. Das Reaktionsgemisch wird i. Vak. vom Lösungsmittel befreit, in Essigsäureethylester aufgenommen und mit 5 %iger, wäßriger Natriumhydroxid-Lösung gewaschen. Die organische Phase wird mit Natriumsulfat getrocknet, filtriert und im Vak. vom Lösungsmittel befreit. Ausbeute: 10,2 g; Fp. 90 - 95°C.

### Stufe c: 2-Chlor-3-(N-3,5-dimethylpyrazolyloxymethyl)-4-sulfonylmethyl-benzoesäure

9,9 g (26,6 mmol) 2-Chlor-3-(N-3,5-dimethylpyrazolyloxymethyl)-4-sulfonylmethylbenzoesäuremethylester werden in einem Gemisch aus 50 ml Tetrahydrofuran und 50 ml dest. Wasser bei Raumtemp. 12 h mit 1,3 g Lithiumhydroxid behandelt. Das Reaktionsgemisch wird in 300 ml dest. Wasser eingetragen, mit 10 %iger, wäßriger Salzsäure auf pH 1 eingestellt und mit Essigsäureethylester extrahiert. Die vereinigten organischen Phasen werden mit Natriumsulfat getrocknet, filtriert und i. Vak. vom Lösungsmittel befreit. Ausbeute: 6,2 g; Fp. 201 - 207°C.

### Stufe d: 2-Chlor-3-(N-3,5-dimethylpyrazolyloxymethyl) -phenyl)-4-sulfonylmethyl-(N-methyl-2-hydroxypyrazol)-methanon

2,0 g (5,6 mmol) 2-Chlor-3-(N-3,5-dimethylpyrazolyloxymethyl)-4-sulfonylmethylbenzoesäure 0,6 g (5,6 mmol) N-Methyl-5-hydroxypyrazol und 1,4 g N,N-Dicyclohexylcarbodiimid werden in 50 ml Tetrahydrofuran 6 h auf 40°C erwärmt. Das Reaktionsgemisch wird filtriert und in Essigsäureethylester aufgenommen. Die organische Phase wird mit gesättigter, wäßriger Natriumhydrogencarbonat-Lösung gewaschen, mit Natriumsulfat getrocknet, filtriert und i. Vak. vom Lösungsmittel befreit.

Das Rohprodukt wird mit 1,2 g Kaliumcarbonat in 20 ml 1,4-Dioxan 6 h auf 100°C erwärmt. Das Reaktionsgemisch wird in 500 ml dest. Wasser eingetragen und mit Diethylether extrahiert. Die organische Phase wird mit 10 %iger, wäßriger Salzsäure auf pH 3 eingestellt und mit Dichlormethan extrahiert. Die organische Phase wird mit dest. Wasser gewaschen, mit Natriumsulfat getrocknet, filtriert und i. Vak. vom Lösungsmittel befreit. Ausbeute: 1,5 g, Fp. 74°C.

**Tabelle 145**

| | | | | | | |
|---|---|---|---|---|---|---|
| 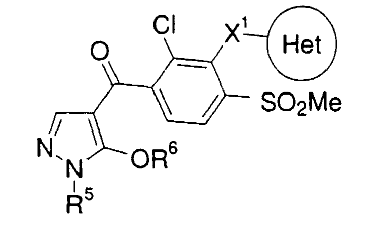 | | | | | | |

| Nr. | R⁵ | R⁶ | X¹ | Het | Fp. [°C] | ¹H-NMR [ppm] |
|---|---|---|---|---|---|---|
| 145.1 | CH₃ | H | CH₂O | 1-Pyrazolyl | 160 | |
| 145.2 | CH₃ | i-Propyl | CH₂O | 1-Pyrazolyl | | 1,4 (d, 6 H) ; |
| | | | | | | 3,4 (s, 3 H) ; |
| | | | | | | 3,7 (s, 3 H); |
| | | | | | | 5,3 (m, 1 H); |
| | | | | | | 6,1 (s, 2 H) ; |
| | | | | | | 6,2 (d, 1 H) ; |
| | | | | | | 7,2 (s, 1 H) ; |
| | | | | | | 7,3 (s, 1 H); |
| | | | | | | 7,4 (d, 1 H); |
| | | | | | | 7,6 (d, 1 H) ; |
| | | | | | | 8,2 (d, 1 H) |
| 145.3 | CH₃ | H | CH₂O | 3,5-Dimethyl-1-pyrazolyl | 74 | |
| 145.4 | CH₃ | i-Propyl | CH₂O | 3,5-Dimethyl-1-pyrazolyl | 149 | |
| 145.5 | CH₃ | SO₂CH₃ | CH₂O | 3,5-Dimethyl-1-pyrazolyl | 157 | |
| 145.6 | CH₃ | H | CH₂O | 4-Chlor-1-pyrazolyl | 164 | |
| 145.7 | CH₃ | i-Propyl | CH₂O | 4-Chlor- | | 1,4 (d, 6 H) ; |
| | | | | 1-pyrazolyl | | 3,4 (s, 3 H); |
| | | | | | | 3,7 (s, 3 H) ; |
| | | | | | | 5,3 (m, 1 H) ; |
| | | | | | | 6,1 (s, 2 H) ; |
| | | | | | | 7,2 (dd, 2 H); |
| | | | | | | 7,5 (s, 1 H) ; |
| | | | | | | 7,6 (s, 1 H) ; |
| | | | | | | 8,2 (d, 1 H) |
| 145.8 | CH₃ | SO₂CH₃ | CH₂O | 4-Chlor- | | 3,4 (s, 3H); |
| | | | | 1-pyrazolyl | | 3,6 (s, 3H); |
| | | | | | | 3,9 (s, 3H) ; |
| | | | | | | 6,1 (s, 2 H); |
| | | | | | | 7,2 (s, 1 H); |
| | | | | | | 7,4 (s, 1 H); |
| | | | | | | 7,5 (s, 1 H) ; |
| | | | | | | 7,6 (d, 1 H); |
| | | | | | | 8,2 (d, 1 H) |
| 145.9 | CH₃ | H | CH₂O | 2-Pyridyl | 94 | |
| 145.10 | CH₃ | i-Propyl | CH₂O | 2-Pyridyl | 66 | |
| 145.11 | C₂H₅ | H | CH₂O | 1-Pyrazolyl | 63 | |
| 145.12 | C₂H₅ | H | CH₂O | 2-Pyridyl | 90 | |

### Anwendungsbeispiele

Die herbizide Wirkung der substituierten 4-Benzoyl-pyrazole der Formel I ließ sich durch Gewächshausversuche zeigen:

Als Kulturgefäße dienten Plastiktöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

**Tabelle A**

| Nr. | X¹ | Het |
|---|---|---|
| 1 | OCH₂ | Oxiranyl |
| 2 | OCH₂ | 3-Methyl-2-oxiranyl |
| 3 | OCH₂ | 2-Oxetanyl |
| 4 | OCH₂ | 3-Hydroxy-3-methyl-2-oxetanyl |
| 5 | OCH₂ | 3-Hydroxy-3-ethyl-2-oxetanyl |
| 6 | OCH₂ | 3-Hydroxy-3-propyl-2-oxetanyl |
| 7 | OCH₂ | 3-Hydroxy-3-butyl-2-oxetanyl |
| 8 | OCH₂ | 3-Methoxy-3-methyl-2-oxetanyl |
| 9 | OCH₂ | 3-Methoxy-3-ethyl-2-oxetanyl |
| 10 | OCH₂ | 3-Methoxy-3-propyl-2-oxetanyl |
| 11 | OCH₂ | 3-Methoxy-3-butyl-2-oxetanyl |
| 12 | OCH₂ | 3-Trimethylsilyloxy-3-methyl-2-oxetanyl |
| 13 | OCH₂ | 3-Trimethylsilyloxy-3-ethyl-2-oxetanyl |
| 14 | OCH₂ | 3-Trimethylsilyloxy-3-propyl-2-oxetanyl |
| 15 | OCH₂ | 3-Trimethylsilyloxy-3-butyl-2-oxetanyl |
| 16 | OCH₂ | 3-Oxetanyl |
| 17 | OCH₂ | 2-Furyl |
| 18 | OCH₂ | 4,5-Dihydro-2-furyl |
| 19 | OCH₂ | 2,3-Dihydro-2-furyl |
| 20 | OCH₂ | 3-Furyl |
| 21 | OCH₂ | 4,5-Dihydro-3-furyl |
| 22 | OCH₂ | 2,3-Dihydro-3-furyl |
| 23 | OCH₂ | 2-Thienyl |
| 24 | OCH₂ | 4,5-Dihydro-2-thienyl |
| 25 | OCH₂ | 2,3-Dihydro-2-thienyl |
| 26 | OCH₂ | 5-Chlor-2-thienyl |
| 27 | OCH₂ | 5-Methyl-2-thienyl |
| 28 | OCH₂ | 3-Thienyl |
| 29 | OCH₂ | 4,5-Dihydro-3-thienyl |
| 30 | OCH₂ | 2,3-Dihydro-3-thienyl |
| 31 | OCH₂ | 2-Pyrrolyl |
| 32 | OCH₂ | 2,5-Dihydro-2-pyrrolyl |
| 33 | OCH₂ | 3-Pyrrol |
| 34 | OCH₂ | 2,5-Dihydro-3-pyrrolyl |
| 35 | OCH₂ | 3-Isoxazolyl |
| 36 | OCH₂ | 4-Methyl-3-isoxazolyl |
| 37 | OCH₂ | 5-Methyl-3-isoxazolyl |
| 38 | OCH₂ | 4,5-Dimethyl-3-isoxazolyl |
| 39 | OCH₂ | 4,5-Dihydro-3-isoxazolyl |
| 40 | OCH₂ | 4-Methyl-4,5-dihydro-3-isoxazolyl |
| 41 | OCH₂ | 5-Methyl-4,5-dihydro-3-isoxazolyl |
| 42 | OCH₂ | 4,5-Dimethyl-4,5-dihydro-3-isoxazolyl |
| 43 | OCH₂ | 4-Isoxazolyl |
| 44 | OCH₂ | 3-Methyl-4-isoxazolyl |
| 45 | OCH₂ | 5-Methyl-4-isoxazolyl |
| 46 | OCH₂ | 5-Cyclopropyl-4-isoxazolyl |
| 47 | OCH₂ | 5-Phenyl-4-isoxazolyl |
| 48 | OCH₂ | 3,5-Dimethyl-4-isoxazolyl |
| 49 | OCH₂ | 4,5-Dihydro-4-isoxazolyl |
| 50 | OCH₂ | 3-Methyl-4,5-dihydro-4-isoxazolyl |
| 51 | OCH₂ | 5-Methyl-4,5-dihydro-4-isoxazolyl |
| 52 | OCH₂ | 3,5-Dimethyl-4,5-dihydro-4-isoxazolyl |
| 53 | OCH₂ | 5-Isoxazolyl |
| 54 | OCH₂ | 3-Methyl-5-isoxazolyl |
| 55 | OCH₂ | 4-Methyl-5-isoxazolyl |
| 56 | OCH₂ | 3,4-Dimethyl-5-isoxazolyl |
| 57 | OCH₂ | 4,5-Dihydro-5-isoxazolyl |
| 58 | OCH₂ | 3-Methyl-4,5-dihydro-5-isoxazolyl |
| 59 | OCH₂ | 4-Methyl-4,5-dihydro-5-isoxazolyl |
| 60 | OCH₂ | 3,4-Dimethyl-4,5-dihydro-5-isoxazolyl |
| 61 | OCH₂ | 3-Isothiazolyl |
| 62 | OCH₂ | 4-Methyl-3-isothiazolyl |
| 63 | OCH₂ | 5-Methyl-3-isothiazolyl |
| 64 | OCH₂ | 4-Isothiazolyl |
| 65 | OCH₂ | 3-Methyl-4-isothiazolyl |
| 66 | OCH₂ | 5-Methyl-4-isothiazolyl |
| 67 | OCH₂ | 5-Isothiazolyl |
| 68 | OCH₂ | 3-Methyl-5-isothiazolyl |
| 69 | OCH₂ | 4-Methyl-5-isothiazolyl |
| 70 | OCH₂ | 2-Oxazolyl |
| 71 | OCH₂ | 4-Oxazolyl |
| 72 | OCH₂ | 5-Oxazolyl |
| 73 | OCH₂ | 2-Thiazolyl |
| 74 | OCH₂ | 4-Thiazolyl |
| 75 | OCH₂ | 5-Thiazolyl |
| 76 | OCH₂ | 3-Pyrazolyl |
| 77 | OCH₂ | 4-Pyrazolyl |
| 78 | OCH₂ | 1-Methyl-3-pyrazolyl |
| 79 | OCH₂ | 1-Methyl-4-pyrazolyl |
| 80 | OCH₂ | 1-Methyl-5-pyrazolyl |
| 81 | OCH₂ | 2-Imidazolyl |
| 82 | OCH₂ | 1-Methyl-2-imidazolyl |
| 83 | OCH₂ | 5-Methyl-[1,3,4]-2-oxadiazolyl |
| 84 | OCH₂ | 5-Methyl-[1,2,4]-3-oxadiazolyl |
| 85 | OCH₂ | 5-Methyl-[1,3,4]-2-thiadiazolyl |
| 86 | OCH₂ | 5-Methyl-[1,2,4]-3-thiadiazolyl |
| 87 | OCH₂ | [1,2,4]-3-triazolyl |
| 88 | OCH₂ | [1,2,3]-4-triazolyl |
| 89 | OCH₂ | 2-Pyridyl |
| 90 | OCH₂ | 6-Chlor-2-pyridyl |
| 91 | OCH₂ | 6-Methoxy-2-pyridyl |
| 92 | OCH₂ | 6-Trifluormethyl-2-pyridyl |
| 93 | OCH₂ | 3-Pyridyl |
| 94 | OCH₂ | 2-Chlor-3-pyridyl |
| 95 | OCH₂ | 2-Methoxy-3-pyridyl |
| 96 | OCH₂ | 4-Pyridyl |
| 97 | OCH₂ | 2-Chlor-4-pyridyl |
| 98 | OCH₂ | 2-Methoxy-4-pyridyl |
| 99 | OCH₂ | 2-Ethoxy-4-pyridyl |
| 100 | OCH₂ | 2-Methylthio-4-pyridyl |
| 101 | OCH₂ | 2-Trifluormethyl-5-pyridyl |
| 102 | OCH₂ | 2-Pyrimidinyl |
| 103 | OCH₂ | 3-Pyrimidinyl |
| 104 | OCH₂ | 4-Pyrimidinyl |
| 105 | OCH₂ | 2-Pyrazinyl |
| 106 | OCH₂ | 3-Pyridazinyl |
| 107 | OCH₂ | 4-Pyridazinyl |
| 108 | OCH₂ | 2-(2H-1,3-oxazinyl) |
| 109 | OCH₂ | 2-(6H-1,3-oxazinyl) |
| 110 | OCH₂ | 4-(6H-1,3-oxazinyl) |
| 111 | OCH₂ | 6-(6H-1,3-oxazinyl) |
| 112 | OCH₂ | [1,3,5]-2-Triazinyl |
| 113 | OCH₂ | [1,2,4]-3-Triazinyl |
| 114 | OCH₂ | [1,2,4]-5-Triazinyl |
| 115 | OCH₂ | [1,2,4]-6-Triazinyl |
| 116 | | Oxiranyl |
| 117 | | 3-Methyl-2-oxiranyl |
| 118 | | 2-Oxetanyl |
| 119 | | 3-Hydroxy-3-methyl-2-oxetanyl |
| 120 | | 3-Hydroxy-3-ethyl-2-oxetanyl |
| 121 | | 3-Hydroxy-3-propyl-2-oxetanyl |
| 122 | | 3-Hydroxy-3-butyl-2-oxetanyl |
| 123 | | 3-Methoxy-3-methyl-2-oxetanyl |
| 124 | | 3-Methoxy-3-ethyl-2-oxetanyl |
| 125 | | 3-Methoxy-3-propyl-2-oxetanyl |
| 126 | | 3-Methoxy-3-butyl-2-oxetanyl |
| 127 | | 3-Trimethylsilyloxy-3-methyl-2-oxetanyl |
| 128 | | 3-Trimethylsilyloxy-3-ethyl-2-oxetanyl |
| 129 | | 3-Trimethylsilyloxy-3-propyl-2-oxetanyl |
| 130 | | 3-Trimethylsilyloxy-3-butyl-2-oxetanyl |
| 131 | | 3-Oxetanyl |
| 132 | | 2-Furyl |
| 133 | | 4,5-Dihydro-2-furyl |
| 134 | | 2,3-Dihydro-2-furyl |
| 135 | | 3-Furyl |
| 136 | | 4,5-Dihydro-3-furyl |
| 137 | | 2,3-Dihydro-3-furyl |
| 138 | | 2-Thienyl |
| 139 | CH₂O | 4,5-Dihydro-2-thienyl |
| 140 | CH₂O | 2,3-Dihydro-2-thienyl |
| 141 | CH₂O | 5-Chlor-2-thienyl |
| 142 | CH₂O | 5-Methyl-2-thienyl |
| 143 | CH₂O | 3-Thienyl |
| 144 | CH₂O | 4,5-Dihydro-3-thienyl |
| 145 | CH₂O | 2,3-Dihydro-3-thienyl |
| 146 | CH₂O | 2-Pyrrolyl |
| 147 | CH₂O | 2,5-Dihydro-2-pyrrolyl |
| 148 | CH₂O | 3-Pyrrol |
| 149 | CH₂O | 2,5-Dihydro-3-pyrrolyl |
| 150 | CH₂O | 3-Isoxazolyl |
| 151 | CH₂O | 4-Methyl-3-isoxazolyl |
| 152 | CH₂O | 5-Methyl-3-isoxazolyl |
| 153 | CH₂O | 4,5-Dimethyl-3-isoxazolyl |
| 154 | CH₂O | 4,5-Dihydro-3-isoxazolyl |
| 155 | CH₂O | 4-Methyl-4,5-dihydro-3-isoxazolyl |
| 156 | CH₂O | 5-Methyl-4,5-dihydro-3-isoxazolyl |
| 157 | CH₂O | 4,5-Dimethyl-4,5-dihydro-3-isoxazolyl |
| 158 | CH₂O | 4-Isoxazolyl |
| 159 | CH₂O | 3-Methyl-4-isoxazolyl |
| 160 | CH₂O | 5-Methyl-4-isoxazolyl |
| 161 | CH₂O | 5-Cyclopropyl-4-isoxazolyl |
| 162 | CH₂O | 5-Phenyl-4-isoxazolyl |
| 163 | CH₂O | 3,5-Dimethyl-4-isoxazolyl |
| 164 | CH₂O | 4,5-Dihydro-4-isoxazolyl |
| 165 | CH₂O | 3-Methyl-4,5-dihydro-4-isoxazolyl |
| 166 | CH₂O | 5-Methyl-4,5-dihydro-4-isoxazolyl |
| 167 | CH₂O | 3,5-Dimethyl-4,5-dihydro-4-isoxazolyl |
| 168 | CH₂O | 5-Isoxazolyl |
| 169 | CH₂O | 3-Methyl-5-isoxazolyl |
| 170 | CH₂O | 4-Methyl-5-isoxazolyl |
| 171 | CH₂O | 3,4-Dimethyl-5-isoxazolyl |
| 172 | CH₂O | 4,5-Dihydro-5-isoxazolyl |
| 173 | CH₂O | 3-Methyl-4,5-dihydro-5-isoxazolyl |
| 174 | CH₂O | 4-Methyl-4,5-dihydro-5-isoxazolyl |
| 175 | CH₂O | 3,4-Dimethyl-4,5-dihydro-5-isoxazolyl |
| 176 | CH₂O | 3-Isothiazolyl |
| 177 | CH₂O | 4-Methyl-3-isothiazolyl |
| 178 | CH₂O | 5-Methyl-3-isothiazolyl |
| 179 | CH₂O | 4-Isothiazolyl |
| 180 | CH₂O | 3-Methyl-4-isothiazolyl |
| 181 | CH₂O | 5-Methyl-4-isothiazolyl |
| 182 | CH₂O | 5-Isothiazolyl |
| 183 | CH₂O | 3-Methyl-5-isothiazolyl |
| 184 | CH₂O | 4-Methyl-5-isothiazolyl |
| 185 | CH₂O | 2-Oxazolyl |
| 186 | CH₂O | 4-Oxazolyl |
| 187 | CH₂O | 5-Oxazolyl |
| 188 | CH₂O | 2-Thiazolyl |
| 189 | CH₂O | 4-Thiazolyl |
| 190 | CH₂O | 5-Thiazolyl |
| 191 | CH₂O | 3-Pyrazolyl |
| 192 | CH₂O | 4-Pyrazolyl |
| 193 | CH₂O | 1-Methyl-3-pyrazolyl |
| 194 | CH₂O | 1-Methyl-4-pyrazolyl |
| 195 | CH₂O | 1-Methyl-5-pyrazolyl |
| 196 | CH₂O | 2-Imidazolyl |
| 197 | CH₂O | 1-Methyl-2-imidazolyl |
| 198 | CH₂O | 5-Methyl-[1,3,4]-2-oxadiazolyl |
| 199 | CH₂O | 5-Methyl-[1,2,4]-3-oxadiazolyl |
| 200 | CH₂O | 5-Methyl-[1,3,4]-2-thiadiazolyl |
| 201 | CH₂O | 5-Methyl-[1,2,4]-3-thiadiazolyl |
| 202 | CH₂O | [1,2,4]-3-triazolyl |
| 203 | CH₂O | [1,2,3]-4-triazolyl |
| 204 | CH₂O | 2-Pyridyl |
| 205 | CH₂O | 6-Chlor-2-pyridyl |
| 206 | CH₂O | 6-Methoxy-2-pyridyl |
| 207 | CH₂O | 6-Trifluormethyl-2-pyridyl |
| 208 | CH₂O | 3-Pyridyl |
| 209 | CH₂O | 2-Chlor-3-pyridyl |
| 210 | CH₂O | 2-Methoxy-3-pyridyl |
| 211 | CH₂O | 4-Pyridyl |
| 212 | CH₂O | 2-Chlor-4-pyridyl |
| 213 | CH₂O | 2-Methoxy-4-pyridyl |
| 214 | CH₂O | 2-Ethoxy-4-pyridyl |
| 215 | CH₂O | 2-Methylthio-4-pyridyl |
| 216 | CH₂O | 2-Trifluormethyl-5-pyridyl |
| 217 | CH₂O | 2-Pyrimidinyl |
| 218 | CH₂O | 3-Pyrimidinyl |
| 219 | CH₂O | 4-Pyrimidinyl |
| 220 | CH₂O | 2-Pyrazinyl |
| 221 | CH₂O | 3-Pyridazinyl |
| 222 | CH₂O | 4-Pyridazinyl |
| 223 | CH₂O | 2-(2H-1,3-oxazinyl) |
| 224 | CH₂O | 2-(6*H*-1,3-oxazinyl) |
| 225 | CH₂O | 4-(6H-1,3-oxazinyl) |
| 226 | CH₂O | 6-(6*H*-1,3-oxazinyl) |
| 227 | CH₂O | [1,3,5]-2-Triazinyl |
| 228 | CH₂O | [1,2,4]-3-Triazinyl |
| 229 | CH₂O | [1,2,4]-5-Triazinyl |
| 230 | CH₂O | [1,2,4]-6-Triazinyl |
| 231 | OCH₂CH₂ | Oxiranyl |
| 232 | OCH₂CH₂ | 3-Methyl-2-oxiranyl |
| 233 | OCH₂CH₂ | 2-Oxetanyl |
| 234 | OCH₂CH₂ | 3-Hydroxy-3-methyl-2-oxetanyl |
| 235 | OCH₂CH₂ | 3-Hydroxy-3-ethyl-2-oxetanyl |
| 236 | OCH₂CH₂ | 3-Hydroxy-3-propyl-2-oxetanyl |
| 237 | OCH₂CH₂ | 3-Hydroxy-3-butyl-2-oxetanyl |
| 238 | OCH₂CH₂ | 3-Methoxy-3-methyl-2-oxetanyl |
| 239 | OCH₂CH₂ | 3-Methoxy-3-ethyl-2-oxetanyl |
| 240 | OCH₂CH₂ | 3-Methoxy-3-propyl-2-oxetanyl |
| 241 | OCH₂CH₂ | 3-Methoxy-3-butyl-2-oxetanyl |
| 242 | OCH₂CH₂ | 3-Trimethylsilyloxy-3-methyl-2-oxetanyl |
| 243 | OCH₂CH₂ | 3-Trimethylsilyloxy-3-ethyl-2-oxetanyl |
| 244 | OCH₂CH₂ | 3-Trimethylsilyloxy-3-propyl-2-oxetanyl |
| 245 | OCH₂CH₂ | 3-Trimethylsilyloxy-3-butyl-2-oxetanyl |
| 246 | OCH₂CH₂ | 3-Oxetanyl |
| 247 | OCH₂CH₂ | 2-Furyl |
| 248 | OCH₂CH₂ | 4,5-Dihydro-2-furyl |
| 249 | OCH₂CH₂ | 2,3-Dihydro-2-furyl |
| 250 | OCH₂CH₂ | 3-Furyl |
| 251 | OCH₂CH₂ | 4,5-Dihydro-3-furyl |
| 252 | OCH₂CH₂ | 2,3-Dihydro-3-furyl |
| 253 | OCH₂CH₂ | 2-Thienyl |
| 254 | OCH₂CH₂ | 4,5-Dihydro-2-thienyl |
| 255 | OCH₂CH₂ | 2,3-Dihydro-2-thienyl |
| 256 | OCH₂CH₂ | 5-Chlor-2-thienyl |
| 257 | OCH₂CH₂ | 5-Methyl-2-thienyl |
| 258 | OCH₂CH₂ | 3-Thienyl |
| 259 | OCH₂CH₂ | 4,5-Dihydro-3-thienyl |
| 260 | OCH₂CH₂ | 2,3-Dihydro-3-thienyl |
| 261 | OCH₂CH₂ | 2-Pyrrolyl |
| 262 | OCH₂CH₂ | 2,5-Dihydro-2-pyrrolyl |
| 263 | OCH₂CH₂ | 3-Pyrrol |
| 264 | OCH₂CH₂ | 2,5-Dihydro-3-pyrrolyl |
| 265 | OCH₂CH₂ | 3-Isoxazolyl |
| 266 | OCH₂CH₂ | 4-Methyl-3-isoxazolyl |
| 267 | OCH₂CH₂ | 5-Methyl-3-isoxazolyl |
| 268 | OCH₂CH₂ | 4,5-Dimethyl-3-isoxazolyl |
| 269 | OCH₂CH₂ | 4,5-Dihydro-3-isoxazolyl |
| 270 | OCH₂CH₂ | 4-Methyl-4,5-dihydro-3-isoxazolyl |
| 271 | OCH₂CH₂ | 5-Methyl-4,5-dihydro-3-isoxazolyl |
| 272 | OCH₂CH₂ | 4,5-Dimethyl-4,5-dihydro-3-isoxazolyl |
| 273 | OCH₂CH₂ | 4-Isoxazolyl |
| 274 | OCH₂CH₂ | 3-Methyl-4-isoxazolyl |
| 275 | OCH₂CH₂ | 5-Methyl-4-isoxazolyl |
| 276 | OCH₂CH₂ | 5-Cyclopropyl-4-isoxazolyl |
| 277 | OCH₂CH₂ | 5-Phenyl-4-isoxazolyl |
| 278 | OCH₂CH₂ | 3,5-Dimethyl-4-isoxazolyl |
| 279 | OCH₂CH₂ | 4,5-Dihydro-4-isoxazolyl |
| 280 | OCH₂CH₂ | 3-Methyl-4,5-dihydro-4-isoxazolyl |
| 281 | OCH₂CH₂ | 5-Methyl-4,5-dihydro-4-isoxazolyl |
| 282 | OCH₂CH₂ | 3,5-Dimethyl-4,5-dihydro-4-isoxazolyl |
| 283 | OCH₂CH₂ | 5-Isoxazolyl |
| 284 | OCH₂CH₂ | 3-Methyl-5-isoxazolyl |
| 285 | OCH₂CH₂ | 4-Methyl-5-isoxazolyl |
| 286 | OCH₂CH₂ | 3,4-Dimethyl-5-isoxazolyl |
| 287 | OCH₂CH₂ | 4,5-Dihydro-5-isoxazolyl |
| 288 | OCH₂CH₂ | 3-Methyl-4,5-dihydro-5-isoxazolyl |
| 289 | OCH₂CH₂ | 4-Methyl-4,5-dihydro-5-isoxazolyl |
| 290 | OCH₂CH₂ | 3,4-Dimethyl-4,5-dihydro-5-isoxazolyl |
| 291 | OCH₂CH₂ | 3-Isothiazolyl |
| 292 | OCH₂CH₂ | 4-Methyl-3-isothiazolyl |
| 293 | OCH₂CH₂ | 5-Methyl-3-isothiazolyl |
| 294 | OCH₂CH₂ | 4-Isothiazolyl |
| 295 | OCH₂CH₂ | 3-Methyl-4-isothiazolyl |
| 296 | OCH₂CH₂ | 5-Methyl-4-isothiazolyl |
| 297 | OCH₂CH₂ | 5-Isothiazolyl |
| 298 | OCH₂CH₂ | 3-Methyl-5-isothiazolyl |
| 299 | OCH₂CH₂ | 4-Methyl-5-isothiazolyl |
| 300 | OCH₂CH₂ | 2-Oxazolyl |
| 301 | OCH₂CH₂ | 4-Oxazolyl |
| 302 | OCH₂CH₂ | 5-Oxazolyl |
| 303 | OCH₂CH₂ | 2-Thiazolyl |
| 304 | OCH₂CH₂ | 4-Thiazolyl |
| 305 | OCH₂CH₂ | 5-Thiazolyl |
| 306 | OCH₂CH₂ | 3-Pyrazolyl |
| 307 | OCH₂CH₂ | 4-Pyrazolyl |
| 308 | OCH₂CH₂ | 1-Methyl-3-pyrazolyl |
| 309 | OCH₂CH₂ | 1-Methyl-4-pyrazolyl |
| 310 | OCH₂CH₂ | 1-Methyl-5-pyrazolyl |
| 311 | OCH₂CH₂ | 2-Imidazolyl |
| 312 | OCH₂CH₂ | 1-Methyl-2-imidazolyl |
| 313 | OCH₂CH₂ | 5-Methyl-[1,3,4]-2-oxadiazolyl |
| 314 | OCH₂CH₂ | 5-Methyl-[1,2,4]-3-oxadiazolyl |
| 315 | OCH₂CH₂ | 5-Methyl-[1,3,4]-2-thiadiazolyl |
| 316 | OCH₂CH₂ | 5-Methyl-[1,2,4]-3-thiadiazolyl |
| 317 | OCH₂CH₂ | [1,2,4]-3-triazolyl |
| 318 | OCH₂CH₂ | [1,2,3]-4-triazolyl |
| 319 | OCH₂CH₂ | 2-Pyridyl |
| 320 | OCH₂CH₂ | 6-Chlor-2-pyridyl |
| 321 | OCH₂CH₂ | 6-Methoxy-2-pyridyl |
| 322 | OCH₂CH₂ | 6-Trifluormethyl-2-pyridyl |
| 323 | OCH₂CH₂ | 3-Pyridyl |
| 324 | OCH₂CH₂ | 2-Chlor-3-pyridyl |
| 325 | OCH₂CH₂ | 2-Methoxy-3-pyridyl |
| 326 | OCH₂CH₂ | 4-Pyridyl |
| 327 | OCH₂CH₂ | 2-Chlor-4-pyridyl |
| 328 | OCH₂CH₂ | 2-Methoxy-4-pyridyl |
| 329 | OCH₂CH₂ | 2-Ethoxy-4-pyridyl |
| 330 | OCH₂CH₂ | 2-Methylthio-4-pyridyl |
| 331 | OCH₂CH₂ | 2-Trifluormethyl-5-pyridyl |
| 332 | OCH₂CH₂ | 2-Pyrimidinyl |
| 333 | OCH₂CH₂ | 3-Pyrimidinyl |
| 334 | OCH₂CH₂ | 4-Pyrimidinyl |
| 335 | OCH₂CH₂ | 2-Pyrazinyl |
| 336 | OCH₂CH₂ | 3-Pyridazinyl |
| 337 | OCH₂CH₂ | 4-Pyridazinyl |
| 338 | OCH₂CH₂ | 2-(2H-1,3-oxazinyl) |
| 339 | OCH₂CH₂ | 2-(6H-1,3-oxazinyl) |
| 340 | OCH₂CH₂ | 4-(6H-1,3-oxazinyl) |
| 341 | OCH₂CH₂ | 6-(6H-1,3-oxazinyl) |
| 342 | OCH₂CH₂ | [1,3,5]-2-Triazinyl |
| 343 | OCH₂CH₂ | [1,2,4]-3-Triazinyl |
| 344 | OCH₂CH₂ | [1,2,4]-5-Triazinyl |
| 345 | OCH₂CH₂ | [1,2,4]-6-Triazinyl |
| 346 | CH₂CH₂O | Oxiranyl |
| 347 | CH₂CH₂O | 3-Methyl-2-oxiranyl |
| 348 | CH₂CH₂O | 2-Oxetanyl |
| 349 | CH₂CH₂O | 3-Hydroxy-3-methyl-2-oxetanyl |
| 350 | CH₂CH₂O | 3-Hydroxy-3-ethyl-2-oxetanyl |
| 351 | CH₂CH₂O | 3-Hydroxy-3-propyl-2-oxetanyl |
| 352 | CH₂CH₂O | 3-Hydroxy-3-butyl-2-oxetanyl |
| 353 | CH₂CH₂O | 3-Methoxy-3-methyl-2-oxetanyl |
| 354 | CH₂CH₂O | 3-Methoxy-3-ethyl-2-oxetanyl |
| 355 | CH₂CH₂O | 3-Methoxy-3-propyl-2-oxetanyl |
| 356 | CH₂CH₂O | 3-Methoxy-3-butyl-2-oxetanyl |
| 357 | CH₂CH₂O | 3-Trimethylsilyloxy-3-methyl-2-oxetanyl |
| 358 | CH₂CH₂O | 3-Trimethylsilyloxy-3-ethyl-2-oxetanyl |
| 359 | CH₂CH₂O | 3-Trimethylsilyloxy-3-propyl-2-oxetanyl |
| 360 | CH₂CH₂O | 3-Trimethylsilyloxy-3-butyl-2-oxetanyl |
| 361 | CH₂CH₂O | 3-Oxetanyl |
| 362 | CH₂CH₂O | 2-Furyl |
| 363 | CH₂CH₂O | 4,5-Dihydro-2-furyl |
| 364 | CH₂CH₂O | 2,3-Dihydro-2-furyl |
| 365 | CH₂CH₂O | 3-Furyl |
| 366 | CH₂CH₂O | 4,5-Dihydro-3-furyl |
| 367 | CH₂CH₂O | 2,3-Dihydro-3-furyl |
| 368 | CH₂CH₂O | 2-Thienyl |
| 369 | CH₂CH₂O | 4,5-Dihydro-2-thienyl |
| 370 | CH₂CH₂O | 2,3-Dihydro-2-thienyl |
| 371 | CH₂CH₂O | 5-Chlor-2-thienyl |
| 372 | CH₂CH₂O | 5-Methyl-2-thienyl |
| 373 | CH₂CH₂O | 3-Thienyl |
| 374 | CH₂CH₂O | 4,5-Dihydro-3-thienyl |
| 375 | CH₂CH₂O | 2,3-Dihydro-3-thienyl |
| 376 | CH₂CH₂O | 2-Pyrrolyl |
| 377 | CH₂CH₂O | 2,5-Dihydro-2-pyrrolyl |
| 378 | CH₂CH₂O | 3-Pyrrol |
| 379 | CH₂CH₂O | 2,5-Dihydro-3-pyrrolyl |
| 380 | CH₂CH₂O | 3-Isoxazolyl |
| 381 | CH₂CH₂O | 4-Methyl-3-isoxazolyl |
| 382 | CH₂CH₂O | 5-Methyl-3-isoxazolyl |
| 383 | CH₂CH₂O | 4,5-Dimethyl-3-isoxazolyl |
| 384 | CH₂CH₂O | 4,5-Dihydro-3-isoxazolyl |
| 385 | CH₂CH₂O | 4-Methyl-4,5-dihydro-3-isoxazolyl |
| 386 | CH₂CH₂O | 5-Methyl-4,5-dihydro-3-isoxazolyl |
| 387 | CH₂CH₂O | 4,5-Dimethyl-4,5-dihydro-3-isoxazolyl |
| 388 | CH₂CH₂O | 4-Isoxazolyl |
| 389 | CH₂CH₂O | 3-Methyl-4-isoxazolyl |
| 390 | CH₂CH₂O | 5-Methyl-4-isoxazolyl |
| 391 | CH₂CH₂O | 5-Cyclopropyl-4-isoxazolyl |
| 392 | CH₂CH₂O | 5-Phenyl-4-isoxazolyl |
| 393 | CH₂CH₂O | 3,5-Dimethyl-4-isoxazolyl |
| 394 | CH₂CH₂O | 4,5-Dihydro-4-isoxazolyl |
| 395 | CH₂CH₂O | 3-Methyl-4,5-dihydro-4-isoxazolyl |
| 396 | CH₂CH₂O | 5-Methyl-4,5-dihydro-4-isoxazolyl |
| 397 | CH₂CH₂O | 3,5-Dimethyl-4,5-dihydro-4-isoxazolyl |
| 398 | CH₂CH₂O | 5-Isoxazolyl |
| 399 | CH₂CH₂O | 3-Methyl-5-isoxazolyl |
| 400 | CH₂CH₂O | 4-Methyl-5-isoxazolyl |
| 401 | CH₂CH₂O | 3,4-Dimethyl-5-isoxazolyl |
| 402 | CH₂CH₂O | 4,5-Dihydro-5-isoxazolyl |
| 403 | CH₂CH₂O | 3-Methyl-4,5-dihydro-5-isoxazolyl |
| 404 | CH₂CH₂O | 4-Methyl-4,5-dihydro-5-isoxazolyl |
| 405 | CH₂CH₂O | 3,4-Dimethyl-4,5-dihydro-5-isoxazolyl |
| 406 | CH₂CH₂O | 3-Isothiazolyl |
| 407 | CH₂CH₂O | 4-Methyl-3-isothiazolyl |
| 408 | CH₂CH₂O | 5-Methyl-3-isothiazolyl |
| 409 | CH₂CH₂O | 4-Isothiazolyl |
| 410 | CH₂CH₂O | 3-Methyl-4-isothiazolyl |
| 411 | CH₂CH₂O | 5-Methyl-4-isothiazolyl |
| 412 | CH₂CH₂O | 5-Isothiazolyl |
| 413 | CH₂CH₂O | 3-Methyl-5-isothiazolyl |
| 414 | CH₂CH₂O | 4-Methyl-5-isothiazolyl |
| 415 | CH₂CH₂O | 2-Oxazolyl |
| 416 | CH₂CH₂O | 4-Oxazolyl |
| 417 | CH₂CH₂O | 5-Oxazolyl |
| 418 | CH₂CH₂O | 2-Thiazolyl |
| 419 | CH₂CH₂O | 4-Thiazolyl |
| 420 | CH₂CH₂O | 5-Thiazolyl |
| 421 | CH₂CH₂O | 3-Pyrazolyl |
| 422 | CH₂CH₂O | 4-Pyrazolyl |
| 423 | CH₂CH₂O | 1-Methyl-3-pyrazolyl |
| 424 | CH₂CH₂O | 1-Methyl-4-pyrazolyl |
| 425 | CH₂CH₂O | 1-Methyl-5-pyrazolyl |
| 426 | CH₂CH₂O | 2-Imidazolyl |
| 427 | CH₂CH₂O | 1-Methyl-2-imidazolyl |
| 428 | CH₂CH₂O | 5-Methyl-[1,3,4]-2-oxadiazolyl |
| 429 | CH₂CH₂O | 5-Methyl-[1,2,4]-3-oxadiazolyl |
| 430 | CH₂CH₂O | 5-Methyl-[1,3,4]-2-thiadiazolyl |
| 431 | CH₂CH₂O | 5-Methyl-[1,2,4]-3-thiadiazolyl |
| 432 | CH₂CH₂O | [1,2,4]-3-triazolyl |
| 433 | CH₂CH₂O | [1,2,3]-4-triazolyl |
| 434 | CH₂CH₂O | 2-Pyridyl |
| 435 | CH₂CH₂O | 6-Chlor-2-pyridyl |
| 436 | CH₂CH₂O | 6-Methoxy-2-pyridyl |
| 437 | CH₂CH₂O | 6-Trifluormethyl-2-pyridyl |
| 438 | CH₂CH₂O | 3-Pyridyl |
| 439 | CH₂CH₂O | 2-Chlor-3-pyridyl |
| 440 | CH₂CH₂O | 2-Methoxy-3-pyridyl |
| 441 | CH₂CH₂O | 4-Pyridyl |
| 442 | CH₂CH₂O | 2-Chlor-4-pyridyl |
| 443 | CH₂CH₂O | 2-Methoxy-4-pyridyl |
| 444 | CH₂CH₂O | 2-Ethoxy-4-pyridyl |
| 445 | CH₂CH₂O | 2-Methylthio-4-pyridyl |
| 446 | CH₂CH₂O | 2-Trifluormethyl-5-pyridyl |
| 447 | CH₂CH₂O | 2-Pyrimidinyl |
| 448 | CH₂CH₂O | 3-Pyrimidinyl |
| 449 | CH₂CH₂O | 4-Pyrimidinyl |
| 450 | CH₂CH₂O | 2-Pyrazinyl |
| 451 | CH₂CH₂O | 3-Pyridazinyl |
| 452 | CH₂CH₂O | 4-Pyridazinyl |
| 453 | CH₂CH₂O | 2-(2H-1,3-oxazinyl) |
| 454 | CH₂CH₂O | 2-(6H-1,3-oxazinyl) |
| 455 | CH₂CH₂O | 4-(6H-1,3-oxazinyl) |
| 456 | CH₂CH₂O | 6-(6H-1,3-oxazinyl) |
| 457 | CH₂CH₂O | [1,3,5]-2-Triazinyl |
| 458 | CH₂CH₂O | [1,2,4]-3-Triazinyl |
| 459 | CH₂CH₂O | [1,2,4]-5-Triazinyl |
| 460 | CH₂CH₂O | [1,2,4]-6-Triazinyl |
| 461 | CH₂OCH₂ | Oxiranyl |
| 462 | CH₂OCH₂ | 3-Methyl-2-oxiranyl |
| 463 | CH₂OCH₂ | 2-Oxetanyl |
| 464 | CH₂OCH₂ | 3-Hydroxy-3-methyl-2-oxetanyl |
| 465 | CH₂OCH₂ | 3-Hydroxy-3-ethyl-2-oxetanyl |
| 466 | CH₂OCH₂ | 3-Hydroxy-3-propyl-2-oxetanyl |
| 467 | CH₂OCH₂ | 3-Hydroxy-3-butyl-2-oxetanyl |
| 468 | CH₂OCH₂ | 3-Methoxy-3-methyl-2-oxetanyl |
| 469 | CH₂OCH₂ | 3-Methoxy-3-ethyl-2-oxetanyl |
| 470 | CH₂OCH₂ | 3-Methoxy-3-propyl-2-oxetanyl |
| 471 | CH₂OCH₂ | 3-Methoxy-3-butyl-2-oxetanyl |
| 472 | CH₂OCH₂ | 3-Trimethylsilyloxy-3-methyl-2-oxetanyl |
| 473 | CH₂OCH₂ | 3-Trimethylsilyloxy-3-ethyl-2-oxetanyl |
| 474 | CH₂OCH₂ | 3-Trimethylsilyloxy-3-propyl-2-oxetanyl |
| 475 | CH₂OCH₂ | 3-Trimethylsilyloxy-3-butyl-2-oxetanyl |
| 476 | CH₂OCH₂ | 3-Oxetanyl |
| 477 | CH₂OCH₂ | 2-Furyl |
| 478 | CH₂OCH₂ | 4,5-Dihydro-2-furyl |
| 479 | CH₂OCH₂ | 2,3-Dihydro-2-furyl |
| 480 | CH₂OCH₂ | 3-Furyl |
| 481 | CH₂OCH₂ | 4,5-Dihydro-3-furyl |
| 482 | CH₂OCH₂ | 2,3-Dihydro-3-furyl |
| 483 | CH₂OCH₂ | 2-Thienyl |
| 484 | CH₂OCH₂ | 4,5-Dihydro-2-thienyl |
| 485 | CH₂OCH₂ | 2,3-Dihydro-2-thienyl |
| 486 | CH₂OCH₂ | 5-Chlor-2-thienyl |
| 487 | CH₂OCH₂ | 5-Methyl-2-thienyl |
| 488 | CH₂OCH₂ | 3-Thienyl |
| 489 | CH₂OCH₂ | 4,5-Dihydro-3-thienyl |
| 490 | CH₂OCH₂ | 2,3-Dihydro-3-thienyl |
| 491 | CH₂OCH₂ | 2-Pyrrolyl |
| 492 | CH₂OCH₂ | 2,5-Dihydro-2-pyrrolyl |
| 493 | CH₂OCH₂ | 3-Pyrrol |
| 494 | CH₂OCH₂ | 2,5-Dihydro-3-pyrrolyl |
| 495 | CH₂OCH₂ | 3-Isoxazolyl |
| 496 | CH₂OCH₂ | 4-Methyl-3-isoxazolyl |
| 497 | CH₂OCH₂ | 5-Methyl-3-isoxazolyl |
| 498 | CH₂OCH₂ | 4,5-Dimethyl-3-isoxazolyl |
| 499 | CH₂OCH₂ | 4,5-Dihydro-3-isoxazolyl |
| 500 | CH₂OCH₂ | 4-Methyl-4,5-dihydro-3-isoxazolyl |
| 501 | CH₂OCH₂ | 5-Methyl-4,5-dihydro-3-isoxazolyl |
| 502 | CH₂OCH₂ | 4,5-Dimethyl-4,5-dihydro-3-isoxazolyl |
| 503 | CH₂OCH₂ | 4-Isoxazolyl |
| 504 | CH₂OCH₂ | 3-Methyl-4-isoxazolyl |
| 505 | CH₂OCH₂ | 5-Methyl-4-isoxazolyl |
| 506 | CH₂OCH₂ | 5-Cyclopropyl-4-isoxazolyl |
| 507 | CH₂OCH₂ | 5-Phenyl-4-isoxazolyl |
| 508 | CH₂OCH₂ | 3,5-Dimethyl-4-isoxazolyl |
| 509 | CH₂OCH₂ | 4,5-Dihydro-4-isoxazolyl |
| 510 | CH₂OCH₂ | 3-Methyl-4,5-dihydro-4-isoxazolyl |
| 511 | CH₂OCH₂ | 5-Methyl-4,5-dihydro-4-isoxazolyl |
| 512 | CH₂OCH₂ | 3,5-Dimethyl-4,5-dihydro-4-isoxazolyl |
| 513 | CH₂OCH₂ | 5-Isoxazolyl |
| 514 | CH₂OCH₂ | 3-Methyl-5-isoxazolyl |
| 515 | CH₂OCH₂ | 4-Methyl-5-isoxazolyl |
| 516 | CH₂OCH₂ | 3,4-Dimethyl-5-isoxazolyl |
| 517 | CH₂OCH₂ | 4,5-Dihydro-5-isoxazolyl |
| 518 | CH₂OCH₂ | 3-Methyl-4,5-dihydro-5-isoxazolyl |
| 519 | CH₂OCH₂ | 4-Methyl-4,5-dihydro-5-isoxazolyl |
| 520 | CH₂OCH₂ | 3,4-Dimethyl-4,5-dihydro-5-isoxazolyl |
| 521 | CH₂OCH₂ | 3-Isothiazolyl |
| 522 | CH₂OCH₂ | 4-Methyl-3-isothiazolyl |
| 523 | CH₂OCH₂ | 5-Methyl-3-isothiazolyl |
| 524 | CH₂OCH₂ | 4-Isothiazolyl |
| 525 | CH₂OCH₂ | 3-Methyl-4-isothiazolyl |
| 526 | CH₂OCH₂ | 5-Methyl-4-isothiazolyl |
| 527 | CH₂OCH₂ | 5-Isothiazolyl |
| 528 | CH₂OCH₂ | 3-Methyl-5-isothiazolyl |
| 529 | CH₂OCH₂ | 4-Methyl-5-isothiazolyl |
| 530 | CH₂OCH₂ | 2-Oxazolyl |
| 531 | CH₂OCH₂ | 4-Oxazolyl |
| 532 | CH₂OCH₂ | 5-Oxazolyl |
| 533 | CH₂OCH₂ | 2-Thiazolyl |
| 534 | CH₂OCH₂ | 4-Thiazolyl |
| 535 | CH₂OCH₂ | 5-Thiazolyl |
| 536 | CH₂OCH₂ | 3-Pyrazolyl |
| 537 | CH₂OCH₂ | 4-Pyrazolyl |
| 538 | CH₂OCH₂ | 1-Methyl-3-pyrazolyl |
| 539 | CH₂OCH₂ | 1-Methyl-4-pyrazolyl |
| 540 | CH₂OCH₂ | 1-Methyl-5-pyrazolyl |
| 541 | CH₂OCH₂ | 2-Imidazolyl |
| 542 | CH₂OCH₂ | 1-Methyl-2-imidazolyl |
| 543 | CH₂OCH₂ | 5-Methyl-[1,3,4]-2-oxadiazolyl |
| 544 | CH₂OCH₂ | 5-Methyl-[1,2,4]-3-oxadiazolyl |
| 545 | CH₂OCH₂ | 5-Methyl-[1,3,4]-2-thiadiazolyl |
| 546 | CH₂OCH₂ | 5-Methyl-[1,2,4]-3-thiadiazolyl |
| 547 | CH₂OCH₂ | [1,2,4]-3-triazolyl |
| 548 | CH₂OCH₂ | [1,2,3]-4-triazolyl |
| 549 | CH₂OCH₂ | 2-Pyridyl |
| 550 | CH₂OCH₂ | 6-Chlor-2-pyridyl |
| 551 | CH₂OCH₂ | 6-Methoxy-2-pyridyl |
| 552 | CH₂OCH₂ | 6-Trifluormethyl-2-pyridyl |
| 553 | CH₂OCH₂ | 3-Pyridyl |
| 554 | CH₂OCH₂ | 2-Chlor-3-pyridyl |
| 555 | CH₂OCH₂ | 2-Methoxy-3-pyridyl |
| 556 | CH₂OCH₂ | 4-Pyridyl |
| 557 | CH₂OCH₂ | 2-Chlor-4-pyridyl |
| 558 | CH₂OCH₂ | 2-Methoxy-4-pyridyl |
| 559 | CH₂OCH₂ | 2-Ethoxy-4-pyridyl |
| 560 | CH₂OCH₂ | 2-Methylthio-4-pyridyl |
| 561 | CH₂OCH₂ | 2-Trifluormethyl-5-pyridyl |
| 562 | CH₂OCH₂ | 2-Pyrimidinyl |
| 563 | CH₂OCH₂ | 3-Pyrimidinyl |
| 564 | CH₂OCH₂ | 4-Pyrimidinyl |
| 565 | CH₂OCH₂ | 2-Pyrazinyl |
| 566 | CH₂OCH₂ | 3-Pyridazinyl |
| 567 | CH₂OCH₂ | 4-Pyridazinyl |
| 568 | CH₂OCH₂ | 2-(2H-1,3-oxazinyl) |
| 569 | CH₂OCH₂ | 2-(6H-1,3-oxazinyl) |
| 570 | CH₂OCH₂ | 4-(6H-1,3-oxazinyl) |
| 571 | CH₂OCH₂ | 6-(6H-1,3-oxazinyl) |
| 572 | CH₂OCH₂ | [1,3,5]-2-Triazinyl |
| 573 | CH₂OCH₂ | [1,2,4]-3-Triazinyl |
| 574 | CH₂OCH₂ | [1,2,4]-5-Triazinyl |
| 575 | CH₂OCH₂ | [1,2,4]-6-Triazinyl |
| 576 | CH₂OCH₂CH=CH | Oxiranyl |
| 577 | CH₂OCH₂CH=CH | 3-Methyl-2-oxiranyl |
| 578 | CH₂OCH₂CH=CH | 2-Oxetanyl |
| 579 | CH₂OCH₂CH=CH | 3-Hydroxy-3-methyl-2-oxetanyl |
| 580 | CH₂OCH₂CH=CH | 3-Hydroxy-3-ethyl-2-oxetanyl |
| 581 | CH₂OCH₂CH=CH | 3-Hydroxy-3-propyl-2-oxetanyl |
| 582 | CH₂OCH₂CH=CH | 3-Hydroxy-3-butyl-2-oxetanyl |
| 583 | CH₂OCH₂CH=CH | 3-Methoxy-3-methyl-2-oxetanyl |
| 584 | CH₂OCH₂CH=CH | 3-Methoxy-3-ethyl-2-oxetanyl |
| 585 | CH₂OCH₂CH=CH | 3-Methoxy-3-propyl-2-oxetanyl |
| 586 | CH₂OCH₂CH=CH | 3-Methoxy-3-butyl-2-oxetanyl |
| 587 | CH₂OCH₂CH=CH | 3-Trimethylsilyloxy-3-methyl-2-oxetanyl |
| 588 | CH₂OCH₂CH=CH | 3-Trimethylsilyloxy-3-ethyl-2-oxetanyl |
| 589 | CH₂OCH₂CH=CH | 3-Trimethylsilyloxy-3-propyl-2-oxetanyl |
| 590 | CH₂OCH₂CH=CH | 3-Trimethylsilyloxy-3-butyl-2-oxetanyl |
| 591 | CH₂OCH₂CH=CH | 3-Oxetanyl |
| 592 | CH₂OCH₂CH=CH | 2-Furyl |
| 593 | CH₂OCH₂CH=CH | 4,5-Dihydro-2-furyl |
| 594 | CH₂OCH₂CH=CH | 2,3-Dihydro-2-furyl |
| 595 | CH₂OCH₂CH=CH | 3-Furyl |
| 596 | CH₂OCH₂CH=CH | 4,5-Dihydro-3-furyl |
| 597 | CH₂OCH₂CH=CH | 2,3-Dihydro-3-furyl |
| 598 | CH₂OCH₂CH=CH | 2-Thienyl |
| 599 | CH₂OCH₂CH=CH | 4,5-Dihydro-2-thienyl |
| 600 | CH₂OCH₂CH=CH | 2,3-Dihydro-2-thienyl |
| 601 | CH₂OCH₂CH=CH | 5-Chlor-2-thienyl |
| 602 | CH₂OCH₂CH=CH | 5-Methyl-2-thienyl |
| 603 | CH₂OCH₂CH=CH | 3-Thienyl |
| 604 | CH₂OCH₂CH=CH | 4,5-Dihydro-3-thienyl |
| 605 | CH₂OCH₂CH=CH | 2,3-Dihydro-3-thienyl |
| 606 | CH₂OCH₂CH=CH | 2-Pyrrolyl |
| 607 | CH₂OCH₂CH=CH | 2,5-Dihydro-2-pyrrolyl |
| 608 | CH₂OCH₂CH=CH | 3-Pyrrol |
| 609 | CH₂OCH₂CH=CH | 2,5-Dihydro-3-pyrrolyl |
| 610 | CH₂OCH₂CH=CH | 3-Isoxazolyl |
| 611 | CH₂OCH₂CH=CH | 4-Methyl-3-isoxazolyl |
| 612 | CH₂OCH₂CH=CH | 5-Methyl-3-isoxazolyl |
| 613 | CH₂OCH₂CH=CH | 4,5-Dimethyl-3-isoxazolyl |
| 614 | CH₂OCH₂CH=CH | 4,5-Dihydro-3-isoxazolyl |
| 615 | CH₂OCH₂CH=CH | 4-Methyl-4,5-dihydro-3-isoxazolyl |
| 616 | CH₂OCH₂CH=CH | 5-Methyl-4,5-dihydro-3-isoxazolyl |
| 617 | CH₂OCH₂CH=CH | 4,5-Dimethyl-4,5-dihydro-3-isoxazolyl |
| 618 | CH₂OCH₂CH=CH | 4-Isoxazolyl |
| 619 | CH₂OCH₂CH=CH | 3-Methyl-4-isoxazolyl |

## Patentansprüche

1. 4-Benzoyl-pyrazole der Formel I 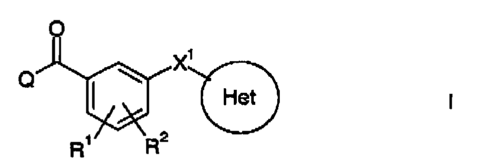 in der die Substituenten folgende Bedeutung haben:
R¹ Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR³ oder -S(O)ₙR³;
R² Wasserstoff oder ein wie voranstehend unter R¹ genannter Rest;
R³ Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl; wobei die genannten Alkylreste partiell oder vollständig halogeniert sein können und/oder eine bis drei der folgenden Gruppen tragen können:
Hydroxy, Mercapto, Amino, Cyano, R³, -OR³, -SR³, -N(R³)₂, =NOR³, -OCOR³, -SCOR³, -NR³COR³, -CO₂R³, -COSR³, -CON(R³)₂, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy,
wobei
Heterocyclyl drei- bis siebengliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, und
Hetaryl, aromatische mono- oder polycyclische Reste, welche neben Kohlenstoffringgliedem zusätzlich ein bis vier Stickstoffatome oder ein bis drei Stickstoffatome und ein Sauerstoff- oder ein Schwefelatom oder ein Sauerstoff- oder ein Schwefelatom enthalten, bedeutet und
wobei die acht letztgenannten Reste ihrerseits substituiert sein können durch ein bis drei Halogenatome und/oder einen oder zwei Reste aus folgender Gruppe: Nitro, Cyano, Methyl, Trifluormethyl, Methoxy, Trifluormethoxy oder Methoxycarbonyl;
n 0, 1 oder 2;
Q ein in 4-Stellung verknüpftes Pyrazol der Formel II, 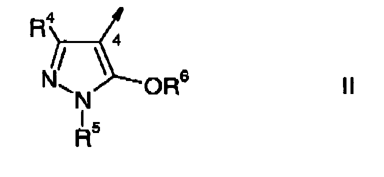 wobei
R⁴ für Wasserstoff, C₁-C₆-Alkyl oder C₁-C₆-Halogenalkyl;
R⁵ für C₁-C₈-Alkyl, C₁-C₆-Halogenalkyl, Phenyl oder Phenyl, das partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy, C₁-C₄-Halogenalkoxy;
R⁶ für Wasserstoff, C₁-C₆-Alkyl, C₁-C₆-Halogenalkyl, C₁-C₆-Alkylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, Phenylcarbonyl, Phenylcarbonylmethyl, Phenoxycarbonyl oder Phenylsulfonyl,
wobei die vier letztgenannten Substituenten unsubstituiert sind oder der Phenylring jeweils partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy, C₁-C₄-Halogenalkoxy;
stehen;
X¹ eine geradkettige oder verzweigte C₁-C₆-Alkylen-, eine C₂-C₆-Alkenylen- oder eine C₂-C₆-Alkinylenkette, die durch ein Heteroatom ausgewählt aus der Gruppe:
Sauerstoff oder Schwefel
unterbrochen ist und wobei die genannten Alkylen-, Alkenylen- oder Alkinylenreste partiell halogeniert sein können und/oder eine bis drei der folgenden Gruppen tragen können:
-OR⁷, -OCOR⁷, -OCONHR⁷ oder -OSO₂R⁷;
R⁷ Wasserstoff, C₁-C₆-Alkyl, C₂-C₆-Alkenyl, C₂-C₆-Alkinyl, Phenyl, Phenyl-C₁-C₆-alkyl, wobei die genannten Alkyl-, Alkenyl- oder Alkinylreste partiell oder vollständig halogeniert sein können und/oder durch einen oder mehrere der folgenden Reste substituiert sein können:
Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy;
Het eine drei- bis sechsgliedrige, mono- oder polycyclische, teilweise oder vollständig gesättigte, heterocyclische Gruppe, die ein bis drei Heteroatome, ausgewählt aus einer Gruppe bestehend aus Sauerstoff, Stickstoff und Schwefel, enthält, oder eine drei- bis sechsgliedrige heteroaromatische Gruppe mit bis zu drei Heteroatomen ausgewählt aus folgenden drei Gruppen:
Stickstoff in Kombination mit mindestens einem Stickstoff oder
Schwefel in Kombination mit mindestens einem Stickstoff;
wobei die genannte heterocyclische oder heteroaromatische Gruppe partiell oder vollständig halogeniert sein kann und/oder durch R⁸ substituiert sein kann;
R⁸ Wasserstoff, Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy, wobei die Alkylreste in allen Fällen jeweils durch einen oder mehrere der folgenden Reste substituiert sein können:
Cyano, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C4-Halogenalkyl, C₁-C₄-Alkylthio, C₁-C₄-Halogenalkylthio, C₁-C₄-Alkoxy, C₁-C₄-Halogenalkoxy;
sowie deren landwirtschaftlich brauchbaren Salze.

2. 4-Benzoyl-pyrazole der Formel la nach Anspruch 1 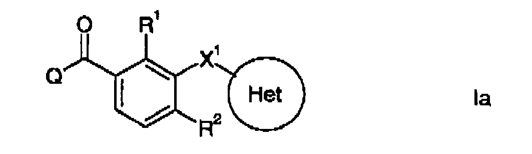 in der die Substituenten R¹, R², Q, X¹ und Het die unter Anspruch 1 genannte Bedeutung haben.

3. 4-Benzoyl-pyrazole der Formel la nach Anspruch 2, in der X¹ für eine durch ein Sauerstoff unterbrochene C₁-C₃-Alkylen-C₂-C₃-Alkenylen- oder C₂-C₃-Alkinylenkette steht.

4. 4-Benzoyl-pyrazole der Formel la nach Anspruch 1 und 2, in der Het eine fünf- oder sechsgliedrige, teilweise oder vollständig gesättigte heterocyclische oder eine fünf- oder sechsgliedrige heteroaromatische Gruppe mit bis zu drei Heteroatomen ausgewählt aus folgenden drei Gruppen:
Stickstoff,
Sauerstoff in Kombination mit mindestens einem Stickstoff oder
Schwefel in Kombination mit mindestens einem Stickstoff;
steht.

5. 4-Benzoyl-pyrazole gemäß einem der Ansprüche 1 bis 4 mit der Formel:

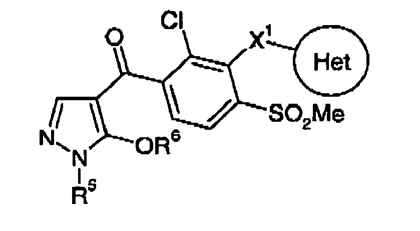

6. Verfahren zur Herstellung von 4-Benzoyl-pyrazolen der Formel I gemäß den Ansprüchen 1 bis 4, **dadurch gekennzeichnet, dass** man ein Pyrazol der Formel lla, in der die Substituenten R⁴ und R⁶ die unter Anspruch 1 genannte Bedeutung haben, 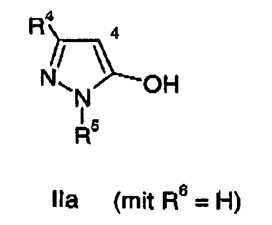 mit einer aktivierten Carbonsäure IIIa oder mit einer Carbonsäure IIIb, 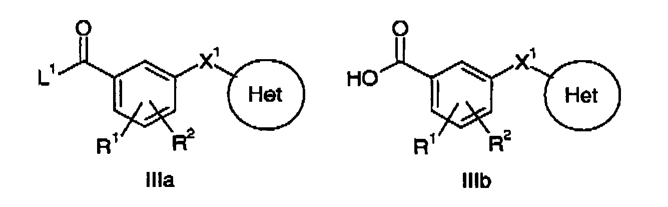 wobei die Substituenten R¹, R², X¹ und Het die in Anspruch 1 genannte Bedeutung haben und L¹ für eine nucleophil austauschbare Abgangsgruppe steht, acyliert und das Acylierungsprodukt gegebenenfalls in Gegenwart eines Katalysators zu den Verbindungen I umlagert und falls gewünscht zur Herstellung von 4-Benzoyl-pyrazolen der allgemeinen Formel I mit R⁶ ≠ H mit einer Verbindung der Formel IV,
L²-R⁶
IV (mit R⁶ ≠ H)
in der R⁶ die unter Anspruch 1 genannte Bedeutung hat mit Ausnahme von Wasserstoff und L² für eine nucleophil austauschbare Abgangsgruppe steht, umsetzt.

7. Mittel, enthaltend eine herbizid wirksame Menge mindestens eines 4-Benzoyl-pyrazols der Formel I oder eines landwirtschaftlich brauchbaren Salzes von I gemäß den Ansprüchen 1 bis 5 und für die Formulierung von Pflanzenschutzmitteln übliche Hilfsmittel.

8. Verfahren zur Herstellung von herbizid wirksamen Mitteln gemäß Anspruch 7, **dadurch gekennzeichnet, dass** man eine herbizid wirksame Menge mindestens eines 4-Benzoyl-pyrazols der Formel I oder eines landwirtschaftlich brauchbaren Salzes von I gemäß den Ansprüchen 1 bis 5 und für die Formulierung von Pflanzenschutzmitteln übliche Hilfsmittel mischt.

9. Verfahren zur Bekämpfung von unerwünschtem Pflanzenwuchs, **dadurch gekennzeichnet, dass** man eine herbizid wirksame Menge mindestens eines 4-Benzoyl-pyrazols der Formel I oder eines landwirtschaftlich brauchbaren Salzes von I gemäß den Ansprüchen 1 bis 5 auf Pflanzen, deren Lebensraum und/oder auf Samen einwirken lässt.

10. Verwendung der 4-Benzoyl-pyrazole der Formel I und deren landwirtschaftlich brauchbaren Salze gemäß den Ansprüchen 1 bis 5 als Herbizide.

## Claims

1. A 4-benzoylpyrazole of the formula I 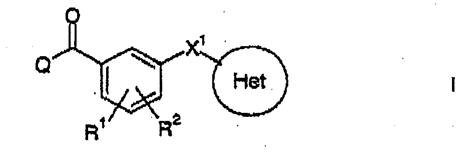 where:
R¹ is nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR³ or -S(O)ₙR³;
R² is hydrogen or a radical as named above under R¹;
R³ is hydrogen, C₁-C₆-alkyl, C₁-C₆-haloalkyl, C₂-C₆-alkenyl, C₂-C₆-alkynyl, phenyl or phenyl-C₁-C₆-alkyl; where the abovementioned alkyl radicals may be partially or fully halogenated and/or may carry one to three of the following groups:
hydroxyl, mercapto, amino, cyano, R³, -OR³, -SR³, -N(R³)₂, =NOR³, -OCOR³, -SCOR³, -NR³COR³, -CO₂R³, -COSR³, -CON(R³)₂, C₁-C₄-alkyliminooxy, C₁-C₄-alkoxyamino, C₁-C₄-alkylcarbonyl, C₁-C₄-alkoxy-C₂-C₆-alkoxycarbonyl, C₁-C₄-alkylsulfonyl, heterocyclyl, heterocyclyloxy, phenyl, benzyl, hetaryl, phenoxy, benzyloxy and hetaryloxy,
where
heterocyclyl is a three- to seven-membered, saturated or partially unsaturated, mono- or polycyclic heterocycle, which contains one to three heteroatoms selected from the group consisting of oxygen, nitrogen and sulfur, and
hetaryl is an aromatic mono- or polycyclic radical which, in addition to carbon ring members, additionally contains one to four nitrogen atoms or one to three nitrogen atoms and one oxygen or one sulfur atom or one oxygen or one sulfur atom, and
where the eight last-mentioned radicals may in turn be substituted by one to three halogen atoms and/or one or two radicals from the following group: nitro, cyano, methyl, trifluoromethyl, methoxy, trifluoromethoxy and methoxycarbonyl;
n is 0, 1 or 2;
Q is a pyrazole of the formula II 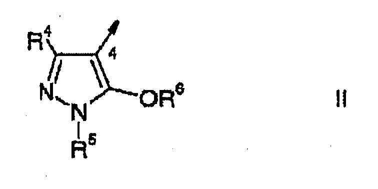 which is attached in position 4 and where
R⁴ is hydrogen, C₁-C₆-alkyl or C₁-C₆-haloalkyl;
R⁵ is C₁-C₆-alkyl, C₁-C₆-haloalkyl, phenyl or phenyl which may be partially or fully halogenated and/or may carry one to three of the following radicals:
nitro, cyano, C₁-C₄-alkyl, C₁-C₄-haloalkyl, C₁-C₄-alkoxy, C₁-C₄-haloalkoxy;
R⁶ is hydrogen, C₁-C₆-alkyl, C₁-C₆-haloalkyl, C₁-C₆-alkylcarbonyl, C₁-C₆-haloalkylcarbonyl, C₁-C₆-alkoxycarbonyl, C₁-C₆-alkylsulfonyl, C₁-C₆-haloalkylsulfonyl, phenylcarbonyl, phenylcarbonylmethyl, phenoxycarbonyl or phenylsulfonyl,
where the four last-mentioned substituents are unsubstituted, or the phenyl ring in question may be partially or fully halogenated and/or may carry one to three of the following radicals:
nitro, cyano, C₁-C₄-alkyl, C₁-C₄-haloalkyl, C₁-C₄-alkoxy, C₁-C₄-haloalkoxy;
X¹ is a straight-chain or branched C₁-C₆-alkylene, a C₂-C₆-alkenylene or a C₂-C₆-alkynylene chain which is interrupted by a hetero atom selected from the group consisting of:
oxygen and sulfur,
where the abovementioned alkylene, alkenylene or alkynylene radicals may be partially halogenated and/or may carry one to three of the following groups:
-OR⁷, -OCOR⁷, -OCONHR⁷ or -OSO₂R⁷;
R⁷ is hydrogen, C₁-C₆-alkyl, C₂-C₆-alkenyl, C₂-C₆-alkynyl, phenyl, phenyl-C₁-C₆-alkyl, where the abovementioned alkyl, alkenyl or alkynyl radicals may be partially or fully halogenated and/or may be substituted by one or more of the following radicals:
hydroxyl, mercapto, amino, cyano, nitro, formyl, C₁-C₄-alkylamino, C₁-C₄-dialkylamino, C₁-C₄-alkoxycarbonyl, C₁-C₄-alkylcarbonyl, C₁-C₄-alkylcarbonyloxy, C₁-C₄-alkyl, C₁-C₄-haloalkyl, C₁-C₄-alkylthio, C₁-C₄-haloalkylthio, C₁-C₄-alkoxy, C₁-C₄-haloalkoxy;
Het is a three- to six-membered, mono- or polycyclic, partially or fully saturated heterocyclic group, which contains one to three heteroatoms selected from the group consisting of oxygen, nitrogen and sulfur, or a three- to six-membered heteroaromatic group having up to three heteroatoms selected from the following two groups:
nitrogen in combination with at least one nitrogen or
sulfur in combination with at least one nitrogen;
where the abovementioned heterocyclic or heteroaromatic group may be partially or fully halogenated and/or may be substituted by R⁸;
R⁸ is hydrogen, hydroxyl, mercapto, amino, cyano, nitro, formyl, C₁-C₄-alkylamino, C₁-C₄-dialkylamino, C₁-C₄-alkoxycarbonyl, C₁-C₄-alkylcarbonyl, C₁-C₄-alkylcarbonyloxy, C₁-C₄-alkyl, C₁-C₄-haloalkyl, C₁-C₄-alkylthio, C₁-C₄-haloalkylthio, C₁-C₄-alkoxy, C₁-C₄-haloalkoxy, where the alkyl radicals may in each case be substituted by in each case one or more of the following radicals:
cyano, formyl, C₁-C₄-alkylamino, C₁-C₄-dialkylamino, C₁-C₄-alkoxycarbonyl, C₁-C₄-alkylcarbonyl, C₁-C₄-alkylcarbonyloxy, C₁-C₄-alkyl, C₁-C₄-haloalkyl, C₁-C₄-alkylthio, C₁-C₄-haloalkylthio, C₁-C₄-alkoxy, C₁-C₄-haloalkoxy;
and agriculturally useful salts thereof.

2. The 4-benzoylpyrazole of the formula Ia according to claim 1, 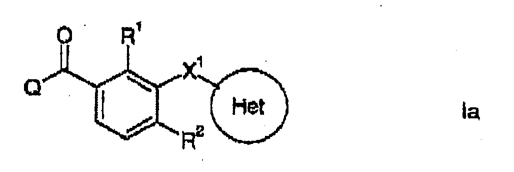 where the substituents R¹, R², Q, X¹ and Het are each as defined under claim 1.

3. The 4-benzoylpyrazole of the formula Ia according to claim 2, where X¹ is a C₁-C₃-alkylene, C₂-C₃-alkenylene or C₂-C₃-alkynylene chain which is interrupted by an oxygen.

4. The 4-benzoylpyrazole of the formula Ia according to claim 1 or 2, where Het is a five- or six-membered partially or fully saturated heterocyclic or a five- or six-membered heteroaromatic group having up to three hetero atoms selected from the following three groups:
nitrogen,
oxygen in combination with at least one nitrogen or
sulfur in combination with at least one nitrogen.

5. The 4-benzoylpyrazole of the formula I according to one of claims 1 to 4 having the formula:

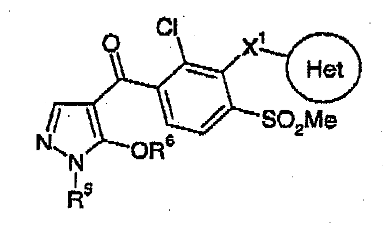

6. A process for preparing 4-benzoylpyrazoles of the formula I according to any of claims 1 to 4, which comprises acylating a pyrazole of the formula IIa, where the substituents R⁴ and R⁶ are each as defined under claim 1, 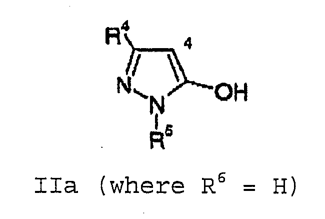 with an activated carboxylic acid IIIa or with a carboxylic acid IIIb, 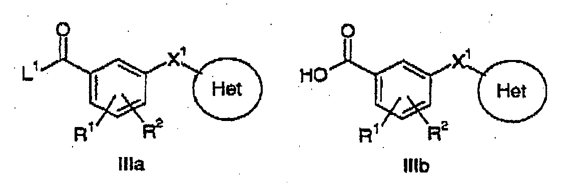 where the substituents R¹, R², X¹ and Het are each as defined in claim 1 and L¹ is a nucleophilically replaceable leaving group, and rearranging the acylation product, if appropriate in the presence of a catalyst, to give the compounds I and, if desired, reacting these with a compound of the formula IV
**L²-R⁶**
IV (where R⁶ ≠ H)
where R⁶ is as defined under claim 1 except for hydrogen and L² is a nucleophilically replaceable leaving group, to prepare 4-benzoylpyrazoles of the formula I where R⁶ ≠ H.

7. A composition comprising a herbicidally effective amount of at least one 4-benzoylpyrazole of the formula I or an agriculturally useful salt of I according to any of claims 1 to 5 and auxiliaries which are customarily used for formulating crop protection agents.

8. A process for preparing herbicidally active compositions according to claim 7, which comprises mixing a herbicidally effective amount of at least one 4-benzoylpyrazole of the formula I or an agriculturally useful salt of I as claimed in any of claims 1 to 5 and auxiliaries which are customarily used for formulating crop protection agents.

9. A method for controlling undesirable vegetation, which comprises allowing a herbicidally effective amount of at least one 4-benzoylpyrazole of the formula I or an agriculturally useful salt of I according to any of claims 1 to 5 to act on plants, their habitat and/or on seeds.

10. The use of the 4-benzoylpyrazoles of the formula I and agriculturally useful salts thereof according to any of claims 1 to 5 as herbicides.

## Revendications

1. 4-benzoyl-pyrazoles de formule I 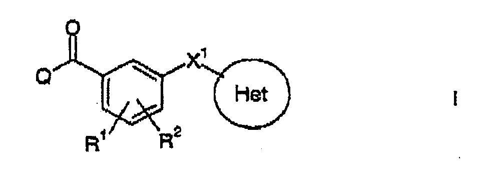 dans laquelle les substituants ont les significations suivantes :
R¹ représente un atome d'halogène ou un groupe nitro, cyano, sulfocyano, alkyle en C₁-C₆, halogénoalkyle en C₁-C₆, alcoxy(C₁-C₆)-alkyle(C₁-C₆), alcényle en C₂-C₆, alcynyle en C₂-C₆, -OR³ ou -S(O)ₙR³ ;
R² représente un atome d'hydrogène ou un radical tel que mentionné précédemment sous R¹ ;
R³ représente un atome d'hydrogène, un groupe alkyle en C₁-C₆, halogénoalkyle en C₁-C₆, alcényle en C₂-C₆, alcynyle en C₂-C₆, phényle ou phényl-alkyle(C₁-C₆) ; les radicaux alkyle cités pouvant être partiellement ou totalement halogénés et/ou pouvant porter un à trois des groupes suivants :
hydroxy, mercapto, amino, cyano, R³, -OR³, -SR³, -N(R³)₂, =NOR³, -OCOR³, -SCOR³, -NR³COR³, -CO₂R³, -COSR³, -CON(R³)₂, alkyl (C₁-C₄) imino-oxy, alcoxy (C₁-C₄) amino, alkyl (C₁-C₄)-carbonyle, alcoxy(C₁-C₄)-alcoxy(C₂-C₆)-carbonyle, alkyl(C₁-C₄)sulfonyle, hétérocyclyle, hétérocyclyloxy, phényle, benzyle, hétéroaryle, phénoxy, benzyloxy et hétéroaryloxy,
hétérocyclyle signifiant des hétérocycles mono- ou polycycliques à 3-7 chaînons, saturés ou partiellement insaturés, qui contiennent un à trois hétéroatomes choisis dans un ensemble constitué par les atomes d'oxygène, d'azote et de soufre, et
hétéroaryle signifiant des radicaux aromatiques mono- ou polycycliques, qui en plus d'atomes de carbone formant le cycle contiennent un à quatre atomes d'azote ou un à trois atomes d'azote et un atome d'oxygène ou un atome de soufre ou un atome d'oxygène ou un atome de soufre, et
les huit radicaux mentionnés en dernier pouvant pour leur part être substitués par un à trois atomes d'halogène et/ou un ou deux radicaux choisis dans l'ensemble suivant : nitro, cyano, méthyle, trifluorométhyle, méthoxy, trifluorométhoxy ou méthoxy-carbonyle ;
n est 0, 1 ou 2 ;
Q représente un groupe pyrazole lié en position 4, de formule II, 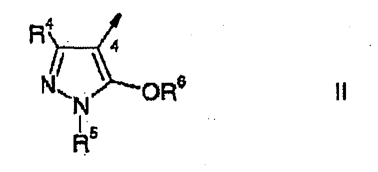 dans laquelle
R⁴ représente un atome d'hydrogène, un groupe alkyle en C₁-C₆ ou halogénoalkyle en C₁-C₆ ;
R⁵ représente un groupe alkyle en C₁-C₆, halogénoalkyle en C₁-C₆, phényle ou phényle qui peut être partiellement ou totalement halogéné et/ou peut porter un à trois des radicaux suivants :
nitro, cyano, alkyle en C₁-C₄, halogénoalkyle en C₁-C₄, alcoxy en C₁-C₄; halogénoalcoxy en C₁-C₄ ;
R⁶ représente un atome d'hydrogène, un groupe alkyle en C₁-C₆, halogénoalkyle en C₁-C₆, alkyl (C₁-C₆) carbonyle, halogénoalkyl (C₁-C₆)-carbonyle, alcoxy(C₁-C₆) carbonyle, alkyl(C₁-C₆)sulfonyle, halogénoalkyl(C₁-C₆)-sulfonyle, phénylcarbonyle, phénylcarbonyl-méthyle, phénoxycarbonyle ou phénylsulfonyle,
les quatre substituants mentionnés en dernier étant non substitués ou le cycle phényle pouvant chaque fois être partiellement ou totalement halogéné et/ou pouvant porter un à trois des radicaux suivants :
nitro, cyano, alkyle en C₁-C₄, halogénoalkyle en C₁-C₄, alcoxy en C₁-C₄, halogénoalcoxy en C₁-C₄ ;
X¹ représente une chaîne alkylène en C₁-C₆ droite ou ramifiée, une chaîne alcénylène en C₂-C₆ ou une chaîne alcynylène en C₂-C₆, qui est interrompue par un hétéroatome choisi dans l'ensemble constitué par :
les atomes d'oxygène et de soufre
et les radicaux alkylène, alcénylène ou alcynylène cités pouvant être partiellement halogénés et/ou pouvant porter un à trois des groupes suivants :
-OR⁷, -OCOR⁷, -OCONHR⁷ ou -OSO₂R⁷ ;
R⁷ représente un atome d'hydrogène, un groupe alkyle en C₁-C₆, alcényle en C₂-C₆, alcynyle en C₂-C₆, phényle, phényl-alkyle(C₁-C₆), les radicaux alkyle, alcényle ou alcynyle cités pouvant être partiellement ou totalement halogénés et/ou pouvant être substitués par un ou plusieurs des radicaux suivants :
hydroxy, mercapto, amino, cyano, nitro, formyle, alkyl(C₁-C₄)amino, dialkyl(C₁-C₄)-amino, alcoxy(C₁-C₄)carbonyle, alkyl(C₁-C₄)-carbonyle, alkyl(C₁-C₄)carbonyloxy, alkyle en C₁-C₄, halogénoalkyle en C₁-C₄, alkyl(C₁-C₄)thio, halogénoalkyl(C₁-C₄)thio, alcoxy en C₁-C₄, halogénoalcoxy en C₁-C₄ ;
Het représente un groupe hétérocyclique mono- ou polycyclique à 3-6 chaînons, partiellement ou totalement saturé, qui contient un à trois hétéroatomes choisis dans l'ensemble constitué par les atomes d'oxygène, d'azote et de soufre, ou un groupe hétéroaromatique à 3-6 chaînons comportant jusqu'à trois hétéroatomes choisis dans les deux ensembles suivants :
un atome d'azote en association avec au moins un atome d'azote ou
un atome de soufre en association avec au moins un atome d'azote ;
ledit groupe hétérocyclique ou hétéroaromatique pouvant être partiellement ou totalement halogéné et/ou pouvant être substitué par R⁸ ;
R⁸ représente un atome d'hydrogène, un groupe hydroxy, mercapto, amino, cyano, nitro, formyle, alkyl(C₁-C₄) amino, dialkyl(C₁-C₄)-amino, alcoxy(C₁-C₄)carbonyle, alkyl(C₁-C₄)-carbonyle, alkyl(C₁-C₄)carbonyloxy, alkyle en C₁-C₄, halogénoalkyle en C₁-C₄, alkyl(C₁-C₄)-thio, halogénoalkyl(C₁-C₄)thio, alcoxy en C₁-C₄, halogénoalcoxy en C₁-C₄,
les radicaux alkyle pouvant dans tous les cas être substitués chacun par un ou plusieurs des radicaux suivants :
cyano, formyle, alkyl(C₁-C₄)amino, dialkyl(C₁-C₄)amino, alcoxy(C₁-C₄)carbonyle, alkyl(C₁-C₄)carbonyle, alkyl(C₁-C₄)-carbonyloxy, alkyle en C₁-C₄, halogénoalkyle en C₁-C₄, alkyl(C₁-C₄)thio, halogénoalkyl(C₁-C₄)thio, alcoxy en C₁-C₄, halogénoalcoxy en C₁-C₄ ;
ainsi que leurs sels utilisables en agriculture.

2. 4-benzoyl-pyrazoles de formule Ia selon la revendication 1 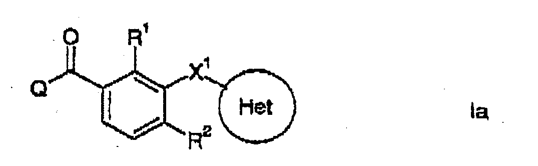 dans lesquels les substituants R¹, R², Q, X¹ et Het ont les significations données dans la revendication 1.

3. 4-benzoyl-pyrazoles de formule Ia selon la revendication 2, dans lesquels X¹ représente une chaîne alkylène en C₁-C₃, alcénylène en C₂-C₃ ou alcynylène en C₂-C₃, interrompue par un atome d'oxygène.

4. 4-benzoyl-pyrazoles de formule Ia selon les revendications 1 et 2, dans lesquels Het représente un groupe hétérocyclique à 5 ou 6 chaînons, partiellement ou totalement saturé ou un groupe hétéroaromatique à 5 ou 6 chaînons, comportant jusqu'à trois hétéroatomes choisis dans les trois ensembles suivants :
un atome d'azote,
un atome d'oxygène en association avec au moins un atome d'azote ou
un atome de soufre en association avec au moins un atome d'azote.

5. 4-benzoyl-pyrazoles selon l'une quelconque des revendications 1 à 4, correspondant à la formule

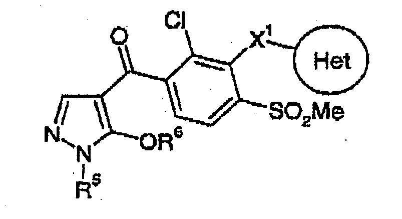

6. Procédé pour la préparation de 4-benzoyl-pyrazoles de formule I selon les revendications 1 à 4, **caractérisé en ce qu'**on soumet à une acylation un pyrazole de formule IIa, dans laquelle les substituants R⁴ et R⁵ ont les significations données dans la revendication 1, 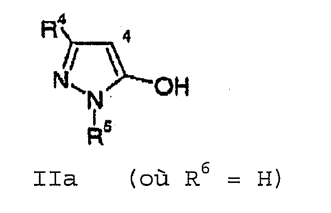 avec un acide carboxylique activé IIIa ou avec un acide carboxylique IIIb, 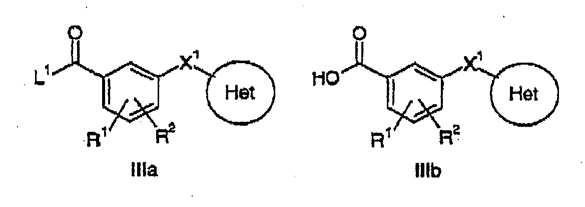 les substituants R¹, R², X¹ et Het ayant les significations données dans la revendication 1 et L¹ représentant un groupe partant pouvant être remplacé par substitution nucléophile, et le produit d'acylation est éventuellement transposé, en présence d'un catalyseur, en les composés I et, si on le désire, pour la préparation de 4-benzoyl-pyrazoles de formule générale I où R⁶ ≠ H, mis en réaction avec un composé de formule IV,
L²-R⁶
IV (où R⁶ ≠ H)
dans laquelle R6 a la signification mentionnée dans la revendication 1 à l'exception de celle d'un atome d'hydrogène et L² représente un groupe partant pouvant être remplacé par substitution nucléophile.

7. Composition contenant une quantité, efficace en tant qu'herbicide, d'au moins un 4-benzoyl-pyrazole de formule I ou d'un sel de I utilisable en agriculture, selon les revendications 1 à 5, et des adjuvants usuels pour la formulation de produits phytosanitaires.

8. Procédé pour la préparation de compositions efficaces en tant qu'herbicides selon la revendication 7, **caractérisé en ce qu'**on mélange une quantité, efficace en tant qu'herbicide, d'au moins un 4-benzoyl-pyrazole de formule I ou d'un sel de I utilisable en agriculture, selon les revendications 1 à 5, et des adjuvants usuels pour la formulation de produits phytosanitaires.

9. Procédé pour la lutte contre la croissance de plantes indésirables, **caractérisé en ce qu'**on laisse agir sue des plantes, leur habitat et/ou sur des semences une quantité, efficace en tant qu'herbicide, d'au moins un 4-benzoyl-pyrazole de formule I ou d'un sel de I utilisable en agriculture, selon les revendications 1 à 5.

10. Utilisation des 4-benzoyl-pyrazoles de formule I et de leurs sels utilisables en agriculture, selon les revendications 1 à 5, en tant qu'herbicide.
